(19) Europäisches Patentamt / European Patent Office / Office européen des brevets

(11) **EP 3 873 525 B1**

(12) **EUROPEAN PATENT SPECIFICATION**

(45) Date of publication and mention of the grant of the patent:
**25.12.2024  Bulletin 2024/52**

(21) Application number: **19878411.8**

(22) Date of filing: **01.11.2019**

(51) International Patent Classification (IPC):
*C07K 16/18* (2006.01)  *A61K 39/395* (2006.01)
*A61K 31/7105* (2006.01)  *G01N 33/53* (2006.01)
*G01N 33/532* (2006.01)  *A61P 25/00* (2006.01)
*A61K 39/00* (2006.01)  *G01N 33/68* (2006.01)

(52) Cooperative Patent Classification (CPC):
**A61P 25/00; C07K 16/18; G01N 33/6893;**
A61K 2039/505; G01N 2800/2871; G01N 2800/50

(86) International application number:
**PCT/US2019/059446**

(87) International publication number:
**WO 2020/092937 (07.05.2020 Gazette 2020/19)**

(54) **COMPOSITIONS AND METHODS FOR TREATING BRAIN INJURY**

ZUSAMMENSETZUNGEN UND VERFAHREN ZUR BEHANDLUNG VON HIRNVERLETZUNGEN

COMPOSITIONS ET PROCÉDÉS DE TRAITEMENT D'UNE LÉSION CÉRÉBRALE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **02.11.2018  US 201862754750 P**

(43) Date of publication of application:
**08.09.2021  Bulletin 2021/36**

(73) Proprietors:
• **Annexon, Inc.**
  **Brisbane, CA 94005 (US)**
• **The Regents of the University of California**
  **Oakland, CA 94607 (US)**

(72) Inventors:
• **ROSI, Susanna**
  **San Francisco, CA 94114 (US)**
• **KRUKOWSKI, Karen**
  **San Francisco, CA 94158 (US)**
• **YEDNOCK, Ted**
  **Forest Knolls, CA 94933 (US)**
• **SANKARANARAYANAN, Sethu**
  **Fremont, CA 94555 (US)**

(74) Representative: **Vossius & Partner**
**Patentanwälte Rechtsanwälte mbB**
**Siebertstraße 3**
**81675 München (DE)**

(56) References cited:
**WO-A1-2010/022149    WO-A1-2010/022149**
**WO-A1-2015/006504    WO-A1-2015/006504**
**WO-A1-2016/073685    WO-A1-2018/017711**
**WO-A2-2014/066744    WO-A2-2014/186599**
**WO-A2-2014/186599**

• **GARRETT M C ET AL:** "Synergistic neuroprotective effects of C3a and C5a receptor blockade following intracerebral hemorrhage", BRAIN RESEARCH, ELSEVIER, AMSTERDAM, NL, vol. 1298, 28 October 2009 (2009-10-28), pages 171 - 177, XP026677161, ISSN: 0006-8993, [retrieved on 20090503], DOI: 10.1016/J.BRAINRES.2009.04.047
• **JANICE A. LANSITA ET AL:** "Nonclinical Development of ANX005: A Humanized Anti-C1q Antibody for Treatment of Autoimmune and Neurodegenerative Diseases", INTERNATIONAL JOURNAL OF TOXICOLOGY, vol. 36, no. 6, 1 November 2017 (2017-11-01), US, pages 449 - 462, XP055702067, ISSN: 1091-5818, DOI: 10.1177/1091581817740873

- **HAMMAD ADNAN ET AL: "The role of the complement system in traumatic brain injury: a review", JOURNAL OF NEUROINFLAMMATION, vol. 15, no. 1, 22 January 2018 (2018-01-22), XP055925386, DOI: 10.1186/s12974-018-1066-z**
- **L. LONGHI ,C. PEREGO , E. R. ZANIER , F. ORTOLANO , P. BIANCHI ,N. STOCCHETTI ,M. G. DE SIMONI: "Neuroprotective effect of CI-inhibitor following traumatic brain injury in mice", ACTA NEUROCHIRURGICA SUPPLEMENTS, vol. 102, 1 January 2008 (2008-01-01), Vienna , AT, pages 381 - 384, XP009527579, ISSN: 0065-1419, DOI: 10.1007/ 978-3-211-85578-2_73**

Remarks:
    The complete document including Reference Table(s) and the Sequence Listing(s) can be downloaded from the EPO website

**Description**

**BACKGROUND**

In WO 2010/022149 methods for treating or ameliorating the damage resulting from intracerebral hemorrhage are disclosed. The methods involve administration of a complement inhibitor to inhibit C3a or C5a formation or activity in the affected tissue.

Traumatic brain injury (TBI) is the number one cause of mortality and disability in young adults in Western societies, and over 20,000 TBIs are sustained annually by US military personnel. TBI is of particular concern for the aging community since there is both increased incidence of TBI and decreased functional recovery in this population. In addition, TBI is the strongest environmental risk factor for development of Alzheimer's disease and other neurodegenerative disorders. A majority of patients who suffer moderate to severe TBIs have chronic and debilitating neuropsychological sequelae, including personality changes, cognitive impairment, and increased incidence of psychiatric illness. Unfortunately, the treatment options for TBIs are currently limited and mainly consist of medical support and rehabilitation. Clearly, better therapeutic options are needed for the treatment of TBI, including drugs that improve cognitive and behavioral outcomes in both the short- and long-term.

**SUMMARY**

The present invention is defined by the claims.

Accordingly, the present invention relates to an inhibitor of the classical complement pathway for use in treating a brain injury, wherein the inhibitor is an anti-C1q antibody comprising:

a light chain variable domain comprising an HVR-L1 having the amino acid sequence of SEQ ID NO: 5, an HVR-L2 having the amino acid of SEQ ID NO: 6, and an HVR-L3 having the amino acid of SEQ ID NO: 7; and
a heavy chain variable domain comprising an HVR-H1 having the amino acid sequence of SEQ ID NO: 9, an HVR-H2 having the amino acid of SEQ ID NO: 10, and an HVR-H3 having the amino acid of SEQ ID NO: 11.

In accordance with a preferred embodiment the inhibitor is to be administered during or within the first 4 weeks after a brain injury, the inhibitor is to be administered during or within the first week after brain injury, the inhibitor is to be administered during or within 24 hours after a brain injury, or the inhibitor is to be administered during or within 1, 2, 3, 4, 5, or 6 hours after a brain injury.

In accordance with a preferred embodiment the inhibitor inhibits synapse loss induced by the brain injury.

In accordance with another preferred embodiment the brain injury is a traumatic brain injury, hypoxic brain injury, brain infection, or stroke.

In accordance with a preferred embodiment the anti-C1q antibody specifically binds to and neutralizes a biological activity of C1q.

In accordance with a further preferred embodiment the anti-C1q antibody is a monoclonal antibody, a polyclonal antibody, a recombinant antibody, a humanized antibody, a chimeric antibody, a multispecific antibody, or an antigen-binding fragment thereof.

In accordance with a different preferred embodiment the antibody is an antigen-binding fragment and the antigen-binding fragment is a Fab fragment, a Fab' fragment, a F(ab')2 fragment, a Fv fragment, a diabody, or a single chain antibody molecule.

In accordance with a preferred embodiment the anti-C1q antibody comprises a light chain variable domain comprising an amino acid sequence with at least 95% homology to the amino acid sequence selected from SEQ ID NO: 4 and 35-38; and wherein the light chain variable domain comprises an HVR-L1 having the amino acid sequence of SEQ ID NO: 5, an HVR-L2 having the amino acid of SEQ ID NO: 6, and an HVR-L3 having the amino acid of SEQ ID NO: 7.

In accordance with a more preferred embodiment the light chain variable domain comprises an amino acid sequence selected from SEQ ID NO: 4 and 35-38.

In accordance with a preferred embodiment the anti-C1q antibody comprises a heavy chain variable domain comprising an amino acid sequence with at least 95% homology to the amino acid sequence selected from SEQ ID NO: 8 and 31-34 and wherein the heavy chain variable domain comprises an HVR-H1 having the amino acid sequence of SEQ ID NO: 9, an HVR-H2 having the amino acid of SEQ ID NO: 10, and an HVR-H3 having the amino acid of SEQ ID NO: 11.

In accordance with a more preferred embodiment the heavy chain variable domain comprises an amino acid sequence selected from SEQ ID NO: 8 and 31-34.

## DESCRIPTION OF THE FIGURES

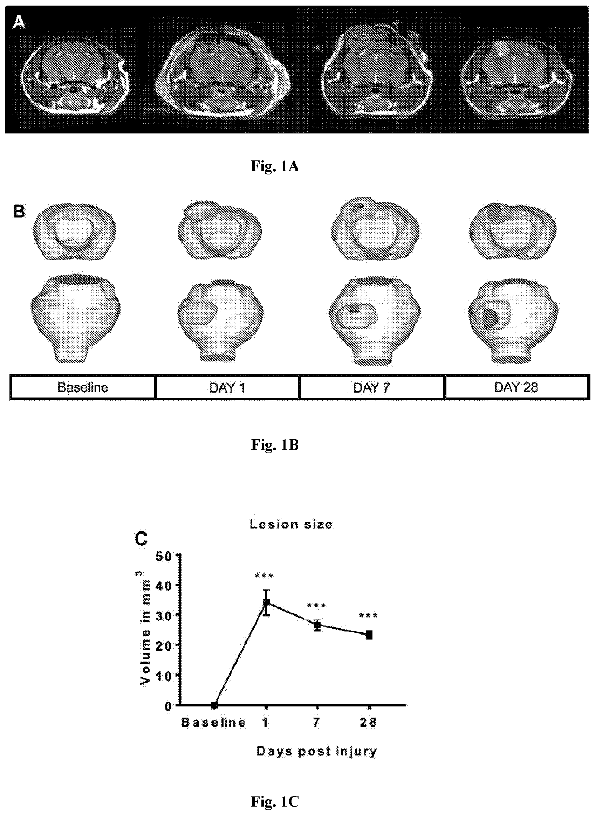

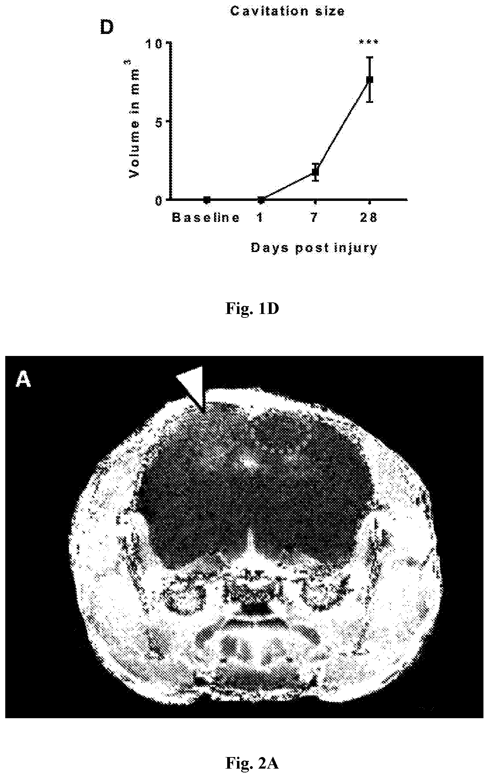

**Figs. 1A-1D** show lesion and cavitation size after contusion injury in aged animals. **Figs. 1A-1B** show MR images of injured animals brains. **Fig. 1C** shows lesion size peaking at one day post injury and plateauing by one month. **Fig. 1D** shows cavitation size increasing over time.

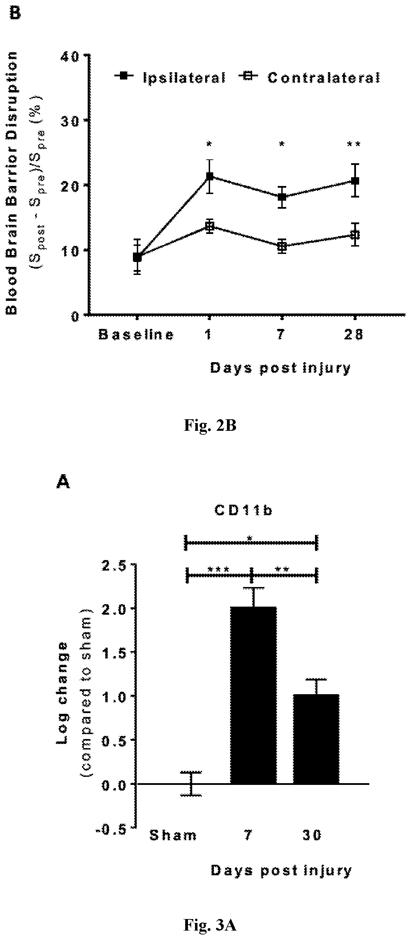

**Figs. 2A-2B** show BBB integrity was compromised after injury. **Fig. 2A** shows increased accumulation of contrast agent through vascular leakage. Fig. 2B shows disruption of BBB.

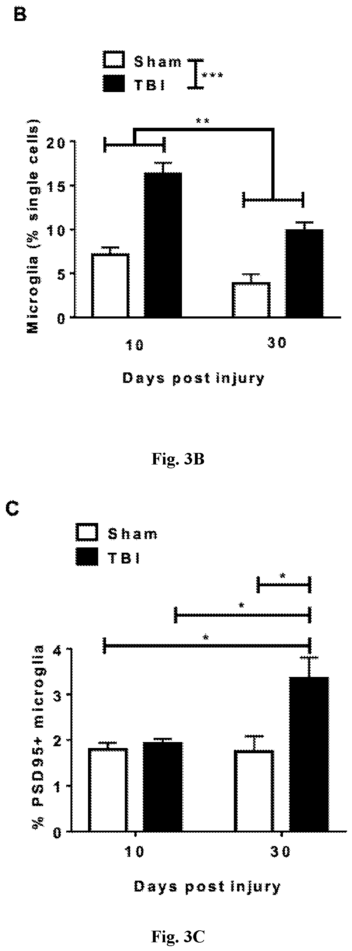

**Figs. 3A-3C** show increased microglia numbers and phagocytic activity chronically after contusion injury. **Fig. 3A** shows CD11b gene-expression changes in the hippocampus of aged animals were measured by qPCR analysis, comparing expression levels between sham, 7 and 30 days post injury. CD11b, a marker for microglia and macrophages significantly increases post injury. **Fig. 3B** shows that a significant increase in microglial numbers was evident in aged injured animals both at 10 and 30 dpi compared to sham animals. **Fig. 3C** shows a significant increase in the percentage of microglia that engulfed labeled synapse in aged injured animals at 30 dpi when compared with matched-sham controls. One-way ANOVA revealed a significant differences (F=21.12; p<0.0001). Tukey-post hoc revealed differences between groups. n=4-6/group.

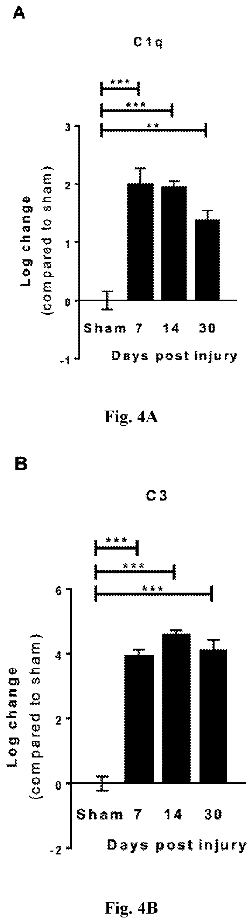

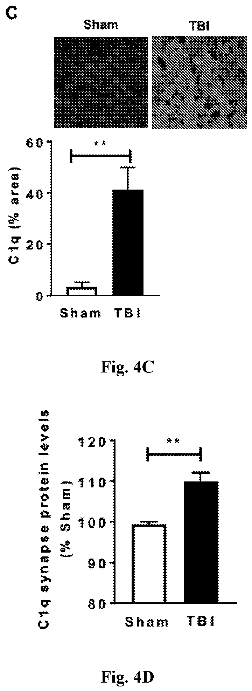

**Figs. 4A-4D** show that contusion injury induces robust complement initiation in the aged brain. **Fig. 4A** and **Fig. 4B** show C1q and C3 gene-expression changes, respectively, in the hippocampus of aged animals were measured by qPCR analysis, comparing expression levels between sham, 7, 14 and 30 days post injury. Both C1q and C3 expression levels were increased after injury. One-way ANOVA revealed a significant differences (C1q-F=15.89; p<0.0001; C3- F=50.58; p<0.0001). Tukey-post hoc revealed differences between groups. n=4-6/group. **Fig. 4C** shows that C1q protein expression was measured by immunohistochemical staining in the dorsal hippocampus. Representative images from Sham and TBI (30 dpi) animal, inset. C1q protein expression is increased chronically after injury. No reactivity was observed in secondary alone. 200x magnification. n=4-6/group. **Fig. 4D** shows that hippocampi were collected and synaptosomes were isolated by sucrose gradient followed by size calibration beads. C1q co-localization at synapses, measured by antibody staining TBI (30 dpi), was significantly increased when compared with sham animals. Student t-test. n=6/group. Bar depict group mean and SEM.

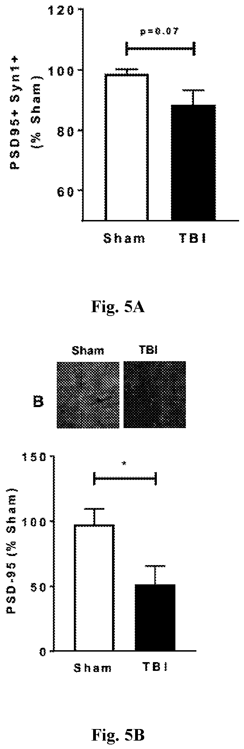

**Figs. 5A-5B** show that contusive injury results in chronic synapse loss in the aged brain. **Fig. 5A** shows that hippocampi were collected and synaptosomes were isolated by sucrose gradient followed by size calibration beads, then co-expression of pre and post synaptic markers. Pre-synaptic marker- Synapsin-1 and post synaptic markers- PSD-95 Significant decreases in total synaptosome numbers in the TBI (30 dpi) group when compared to Sham group. n=8-9/group. **Fig. 5B** shows that synaptic levels were measured by PSD-95 staining in the dorsal hippocampus. Representative images inset. Aged, TBI (30 dpi) animals had decreased PSD-95 expression. No reactivity was observed in secondary alone. 200x magnification. n=3/group. Student t-test. Bar depict group mean and SEM.

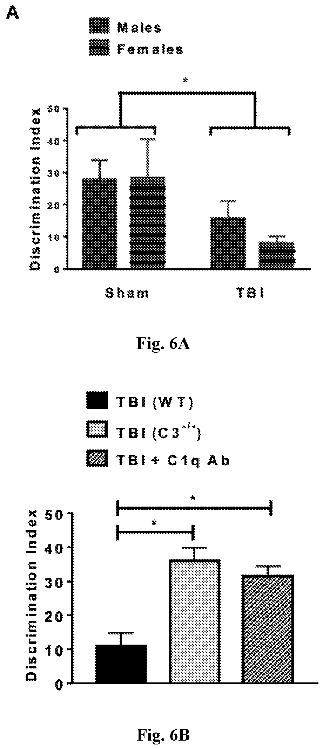

**Figs. 6A-6B** show that complement blockade prevents memory deficits in aged animals after contusion injury. Trauma-induced memory deficits were measured by novel object recognition. Animals were exposed to two identical objects, 24 hrs later the animals are exposed to one familiar object and one novel object. Memory deficits are calculated by a deficit in distinguishing the new (novel) object. **Fig. 6A** shows that both male and female animals displayed memory deficits following TBI (30 dpi). Two-way ANOVA reveals a significant injury effect (p<0.05). Males n=15-17/group; Females 6-7/group. **Fig. B** shos that genetic (C3$^{-/-}$) or pharmacological (C1q inhibiting Ab) blockade of complement initiation factors prevents trauma-induced deficits. TBI (WT) = 27 mice; TBI (C3$^{-/-}$)= 4 mice; TBI$^+$ C1q Ab = 7 mice. One-way ANOVA revealed significant differences (F=6.35; p<0.01). Tukey post-hoc analysis for differences between groups.

## DETAILED DESCRIPTION

General

While the present invention is solely defined by the claims, the present disclosure is also generally directed to methods of preventing, reducing risk of developing, or treating traumatic brain injury (TBI), comprising administering to a subject an inhibitor of the complement pathway, preferably an inhibitor of the classical complement pathway.

Traumatic brain injury (TBI), also known as intracranial injury, occurs when an external force injures the brain. TBI can be classified based on severity, mechanism (closed or penetrating head injury), or other features (e.g., occurring in a specific location or over a widespread area). Head injury is a broader category that may involve damage to other structures such as the scalp and skull. TBI can result in physical, cognitive, social, emotional, and behavioral symptoms, and outcome can range from complete recovery to permanent disability or death.

Causes of TBI include falls, vehicle collisions, and violence. Brain trauma occurs as a consequence of a sudden acceleration or deceleration within the cranium or by a complex combination of both movement and sudden impact. In addition to the damage caused at the moment of injury, a variety of events in the minutes to days following the injury may result in secondary injury. These processes include alterations in cerebral blood flow and the pressure within the skull. Some of the imaging techniques used for diagnosis include computed tomography and magnetic resonance imaging (MRIs).

It has been found herein that deleting complement genes or inhibiting complement proteins attenuates TBI pathology or behavioral deficits in rodent models. Accordingly, in certain implementations, inhibition of complement activation pathways by blocking C1q, the initiating factor in the classical complement cascade, prevents and/or reduces the risk of developing TBI, and/or treats TBI. Highly specific and potent inhibitory antibodies against C1q are disclosed herein, that are able to cross the BBB at sufficient levels to block C1q within the CNS. In rodents, free levels of C1q are completely eliminated in serum and CSF with anti-C1q antibody treatment. Similarly, in primates, levels of free C1q in serum and CSF are eliminated for 20 days following a single dose of anti-C1q antibody. This antibody is effective in several mouse models of neurodegeneration and demyelination, including models of Guillain-Barré syndrome, neuromyelitis optica, Alzheimer's disease, Huntington's disease, and spinal muscular atrophy. C1q expression is found elevated at acute time points after TBI and remains elevated during subacute time points when loss of synapses is also measured. In certain implementations, activation of the complement cascade could result in loss of neuronal / behavioral function. Without being bound by any particular theory, in TBI, the anti-C1q antibodies disclosed herein may act by blocking acute complement activation / neuronal damage and death; and/or by preventing the initiation of CMND over the days to weeks following TBI.

T2-weighted MRI was utilized to determine that there was an increase in lesion size and cavitation with time that persisted for up to a month post injury in the aged animals. Using T1-weighted MRI with post injection of Gadolinium-based contrast agent, the BBB integrity was identified to be compromised, as shown by increased accumulation of contrast agent through vascular leakage. There were long-lasting alterations in lesion size, cavitation and BBB breakdown at one-month post injury that were previously shown not to be present in young injured animals. Persistent disruption of protective barrier function may create a permissive environment for exacerbated entry of peripheral immune cells into the brain parenchyma and the prolonged inflammatory responses measured.

The inflammatory response was investigated herein at a chronic time point after injury in the aged brain. At one-month post injury, there is persistent and significant microglial cell activation, and that microglial cells develop enhanced phagocytic activity with time (30 dpi). While microglial cell activation occurs rapidly after injury, it is interesting that the heightened engulfment activity was not observed at an early end point (10 days post injury). These results indicate that changes in the myeloid compartment can progress for a prolonged period of time after injury, and may relate to the fact that aged animals develop worse cognitive deficits after injury. When considering therapeutic interventions, these results suggest that a longer or later treatment window might be of benefit when targeting myeloid cell activation.

Increased C1q, C3 and CR3 (CD11b) expression lasting for up to one-month post injury was detected. Coupled with C1q accumulation on the synapses, synapse loss and enhanced microglial cell phagocytic capacity were observed in aged injured animals. These results demonstrate that complement tagging of synapses and increased microglial cell engulfing activity are potential regulators of cognitive decline in the aged injured brain.

TBI induces complement and complement receptor expression for at least one month in aged animals, which is associated with a progressive increase in microglial cell phagocytic activity. Importantly, genetic or pharmacological interference with the complement pathway prevents memory decline in aged animals. Age and injury may have a compounding effect on complement expression and neuroinflammatory pathways suggesting broader therapeutic options and windows for these pathways. Blocking formation of the complement membrane attack complex (MAC) reduces acute deficits following severe TBI, whereas inhibition of C3 is required to prevent chronic inflammation and ongoing neuronal loss (at 30 dpi). Specialized degenerative conditions in the CNS of aged animals was investigated. Specific modulation of the classical complement cascade (via inhibition of C1q) is protective against the loss of cognitive function. C1q is the initiating molecule of the classical complement pathway, and has been shown to accumulate on synapses with age, reaching levels up to 300-fold higher than in younger animals. Such accumulation of C1q potentially puts the aging brain at risk for neurodegenerative insults, including TBI. C1q activation leads to tagging of synapses by activated components of C3, which are recognized by the receptor CR3 on microglia, leading to synapse elimination and neuroinflammation. While inhibition of C1q blocks the downstream role of C3 in this pathway, it allows C3 to retain its normal immune function in the lectin and alternative complement pathways, which may be important for neuronal health.

TBI-induced long term memory deficits in aged animals involve increased expression of early complement cascade components (C1q, C3 and CR3) in the brain; increased complement expression and inflammation are associated with a progressive increase in synaptic engulfment activity by microglial cells; and inhibition of the classical complement cascade, either through inhibition of C1q or deletion of C3 provides protection against cognitive decline. Importantly, complement-based interference may provide therapeutic options for treatment of TBI-induced cognitive deficits in the aging brain.

The anti-C1q antibodies disclosed herein are potent inhibitors of C1q and can be dosed for continuous inhibition in both the periphery and CNS over any period, and then optionally withdrawn to allow for return of normal C1q function at times when its activity may be important for CNS repair. Results obtained with anti-C1q antibodies disclosed herein in animal studies can be readily carried forward into the clinic with a humanized version of the same antibody (disclosed antibodies herein cross react with mouse and human C1q).

C1q is a large multimeric protein of 460 kDa consisting of 18 polypeptide chains (6 C1q A chains, 6 C1q B chains, and 6 C1q C chains). C1r and C1s complement proteins bind to the C1q tail region to form the C1 complex (C1qr$_2$s$_2$).

Neutralizing the activity of complement factors such as C1q, C1r, or C1s inhibits classical complement activity, and slow or prevent a brain injury, preferably a traumatic brain injury, hypoxic brain injury, brain infection, or stroke. Methods related to neutralizing complement factors such as C1q, C1r, or C1s in a brain injury, preferably a traumatic brain injury, hypoxic brain injury, brain infection, or stroke are disclosed herein.

In certain aspects, disclosed herein is a method of preventing, reducing risk of developing, or treating a brain injury, preferably a traumatic brain injury, hypoxic brain injury, brain infection, or stroke, comprising administering to a subject an inhibitor of the complement pathway.

Disclosed herein is a method of inhibiting a brain injury, preferably a traumatic brain injury, hypoxic brain injury, brain infection, or stroke, comprising administering to a patient an antibody, such as an anti-Clq antibody, an anti-Clr antibody, or an anti-Cls antibody. In certain preferred implementations, the antibody binds to C1q, C1r, or C1s and inhibits complement activation.

Full-length antibodies may be prepared by the use of recombinant DNA engineering techniques. Such engineered versions include those created, for example, from natural antibody variable regions by insertions, deletions or changes in or to the amino acid sequences of the natural antibodies. Particular examples of this type include those engineered variable region domains containing at least one CDR and optionally one or more framework amino acids from one antibody and the remainder of the variable region domain from a second antibody. The DNA encoding the antibody may be prepared by deleting all but the desired portion of the DNA that encodes the full length antibody. DNA encoding chimerized antibodies may be prepared by recombining DNA substantially or exclusively encoding human constant regions and DNA encoding variable regions derived substantially or exclusively from the sequence of the variable region of a mammal other than a human. DNA encoding humanized antibodies may be prepared by recombining DNA encoding constant regions and variable regions other than the complementarity determining regions (CDRs) derived substantially or exclusively from the corresponding human antibody regions and DNA encoding CDRs derived substantially or exclusively from a mammal other than a human.

Suitable sources of DNA molecules that encode antibodies include cells, such as hybridomas, that express the full length antibody. For example, the antibody may be isolated from a host cell that expresses an expression vector that encodes the heavy and/or light chain of the antibody.

Antibody fragments may also be prepared by the use of recombinant DNA engineering techniques involving the manipulation and re-expression of DNA encoding antibody variable and constant regions. Standard molecular biology techniques may be used to modify, add or delete further amino acids or domains as desired. Any alterations to the variable or constant regions are still encompassed by the terms 'variable' and 'constant' regions as used herein. In some instances, PCR is used to generate an antibody fragment by introducing a stop codon immediately following the codon encoding the interchain cysteine of C$_H$1, such that translation of the C$_H$1 domain stops at the interchain cysteine. Methods for designing suitable PCR primers are well known in the art and the sequences of antibody C$_H$1 domains are readily available. In some implementations, stop codons may be introduced using site-directed mutagenesis techniques.

An antibody of the present disclosure may be derived from any antibody isotype ("class") including for example IgG, IgM, IgA, IgD and IgE and subclasses thereof, including for example IgG1, IgG2, IgG3 and IgG4. In certain preferred implementations, the heavy and light chains of the antibody are from murine IgGl.

In some implementations, the inhibitor is an antibody, such as an anti-Clq antibody, an anti-Clr antibody, or an anti-Cls antibody. The anti-C1q antibody may inhibit the interaction between C1q and an autoantibody, or between C1q and C1r, or between C1q and C1s. The anti-C1r antibody may inhibit the interaction between C1r and C1q, or between C1r and C1s. The anti-C1r antibody may inhibit the catalytic activity of C1r, or the anti-C1r antibody may inhibit the processing of pro-C1r to an active protease. The anti-C1s antibody may inhibit the interaction between C1s and C1q, or between C1s and C1r, or between C1s and C2 or C4, or the anti-C1s antibody may inhibit the catalytic activity of C1s, or it may inhibit the processing of pro-C1s to an active protease. In some instances, the anti-C1q, antiC1r, or anti-C1s antibody causes clearance of C1q, C1r or C1s from the circulation or a tissue.

The antibody disclosed herein may be a monoclonal antibody, e.g., that binds mammalian C1q, C1r, or C1s, preferably human C1q, C1r, or C1s. The antibody may be a mouse antibody, a human antibody, a humanized antibody, a chimeric antibody, or an antibody fragment. The antibodies disclosed herein may also cross the blood brain barrier (BBB). The antibody may activate a BBB receptor-mediated transport system, such as a system that utilizes the insulin receptor, transferrin receptor, leptin receptor, LDL receptor, or IGF receptor. The antibody can be a chimeric antibody with sufficient human sequence that is suitable for administration to a human. The antibody can be glycosylated or nonglycosylated; in some implementations, the antibody is glycosylated, *e.g.*, in a glycosylation pattern produced by post-translational modification in a CHO cell.

The antibody may be a bispecific antibody, recognizing a first and a second antigen, *e.g.,* the first antigen is selected from C1q, C1r, and C1s and/or the second antigen is an antigen that allows the antibody to cross the blood-brain-barrier, such as an antigen selected from transferrin receptor (TR), insulin receptor (HIR), Insulin-like growth factor receptor (IGFR), low-density lipoprotein receptor related proteins 1 and 2 (LPR-1 and 2), diphtheria toxin receptor, CRM197, a llama single domain antibody, TMEM 30(A), a protein transduction domain, TAT, Syn-B, penetratin, a poly-arginine peptide, an angiopep peptide, or ANG1005.

An antibody of the present disclosure may bind to and inhibit a biological activity of C1q, C1r, or C1. For example, (1) C1q binding to an autoantibody, (2) C1q binding to C1r, (3) C1q binding to C1s, (4) C1q binding to phosphatidylserine, (5) C1q binding to pentraxin-3, (6) C1q binding to C-reactive protein (CRP), (7) C1q binding to globular C1q receptor (gC1qR), (8) C1q binding to complement receptor 1 (CR1), (9) C1q binding to B-amyloid, or (10) C1q binding to calreticulin. In other implementations, the biological activity of C1q is (1) activation of the classical complement activation pathway, (2) activation of antibody and complement dependent cytotoxicity, (3) CH50 hemolysis, (4) synapse loss, (5) B-cell antibody production, (6) dendritic cell maturation, (7) T-cell proliferation, (8) cytokine production (9) microglia activation, (10) Arthus reaction, (11) phagocytosis of synapses or nerve endings or (12) activation of complement receptor 3 (CR3/C3) expressing cells.

In some implementations, CH50 hemolysis comprises human, mouse, and/or rat CH50 hemolysis. In some implementations, the antibody is capable of neutralizing from at least about 50%, to at least about 95% of CH50 hemolysis. The antibody may also be capable of neutralizing at least 50% of CH50 hemolysis at a dose of less than 150 ng/ml, less than 100 ng/ml, less than 50 ng/ml, or less than 20 ng/ml.

Other *in vitro* assays to measure complement activity include ELISA assays for the measurement of split products of complement components or complexes that form during complement activation. Complement activation via the classical pathway can be measured by following the levels of C4d and C4 in the serum. Activation of the alternative pathway can be measured in an ELISA by assessing the levels of Bb or C3bBbP complexes in circulation. An *in vitro* antibody-mediated complement activation assay may also be used to evaluate inhibition of C3a production.

An antibody of the present disclosure may be a monoclonal antibody, a polyclonal antibody, a recombinant antibody, a humanized antibody, a chimeric antibody, a multispecific antibody, or an antibody fragment thereof.

The antibodies of the present disclosure may also be an antibody fragment, such as a Fab fragment, a Fab' fragment, a $F(ab')_2$ fragment, a Fv fragment, a diabody, or a single chain antibody molecule.

Disclosed herein are methods of administering to the subject a second agent, such as a second antibody or a second inhibitor. The antibody may be an anti-C1q antibody, an anti-C1r antibody, or an anti-C1s antibody. The inhibitor may be an inhibitor of antibody-dependent cellular cytotoxicity, alternative complement activation pathway; and/or an inhibitor of the interaction between the autoantibody and an autoantigen.

In some implementations, a method is provided of determining a subject's risk of developing a brain injury, preferably a traumatic brain injury, hypoxic brain injury, brain infection, or stroke, comprising: (a) administering an antibody to the subject (i.e. an anti-C1q, anti-C1r, or anti-C1s antibody), wherein the antibody is coupled to a detectable label; (b) detecting the detectable label to measure the amount or location of C1q, C1r, or C1s in the subject; and (c) comparing the amount or location of one or more of C1q, C1r, or C1s to a reference, wherein the risk of developing a brain injury, preferably a traumatic brain injury, hypoxic brain injury, brain infection, or stroke is characterized based on a the comparison of the amount or location of one or more of C1q, C1r, or C1s to the reference. The detectable label may comprise a nucleic acid, oligonucleotide, enzyme, radioactive isotope, biotin or a fluorescent label. In some instances, the antibody may be labeled with a coenzyme such as biotin using the process of biotinylation. When biotin is used as a label, the detection of the antibody is accomplished by addition of a protein such as avidin or its bacterial counterpart streptavidin, either of which can be bound to a detectable marker such as the aforementioned dye, a fluorescent marker such as fluorescein, a radioactive isotope or an enzyme such as peroxidase. In some implementations, the antibody is an antibody fragment (e.g., Fab, Fab'-SH, Fv, scFv, or $F(ab')_2$ fragments).

The antibodies disclosed herein may also be coupled to a labeling group, *e.g.,* a radioisotope, radionuclide, an enzymatic group, biotinyl group, a nucleic acid, oligonucleotide, enzyme, or a fluorescent label. A labeling group may be coupled to the antibody *via* a spacer arm of any suitable length to reduce potential steric hindrance. Various methods for labeling proteins are known in the art and can be used to prepare such labeled antibodies.

Various routes of administration are contemplated. Such methods of administration include but are not limited to, topical, parenteral, subcutaneous, intraperitoneal, intrapulmonary, intrathecal, intranasal, and intralesional administration. Parenteral infusions include intramuscular, intravenous, intraarterial, intraperitoneal, or subcutaneous administration. For treatment of central nervous system conditions, the antibody may be adapted to cross the blood-brain barrier following a non-invasive peripheral route of administration such as intravenous intramuscular, subcutaneous, intraperitoneal, or even oral administration.

Suitable antibodies include antibodies that bind to complement component C1q, C1r, or C1s. Such antibodies include monoclonal antibodies and homologues, analogs, and modified or derived forms thereof, including Fab, $F(ab')_2$,

Fv and single chain antibodies.

Preferred antibodies are monoclonal antibodies, which can be raised by immunizing rodents (e.g., mice, rats, hamsters and guinea pigs) with either (1) the native complement component (e.g., C1q, C1r, or C1s) derived from enzymatic digestion of a purified complement component from human plasma or serum, or (2) a recombinant complement component, or its derived fragment, expressed by either eukaryotic or prokaryotic systems. Other animals can be used for immunization, e.g., non-human primates, transgenic mice expressing human immunoglobulins, and severe combined immunodeficient (SCID) mice transplanted with human B-lymphocytes.

Polyclonal and monoclonal antibodies are naturally generated as immunoglobulin (Ig) molecules in the immune system's response to a pathogen. A dominating format with a concentration of 8 mg/ml in human serum, the ~150-kDa IgG1 molecule is composed of two identical ~50-kDa heavy chains and two identical ~25-kDa light chains.

Hybridomas can be generated by conventional procedures by fusing B-lymphocytes from the immunized animals with myeloma cells. In addition, anti -C1q, -C1r, or -C1s antibodies can be generated by screening recombinant single-chain Fv or Fab libraries from human B-lymphocytes in a phage-display system. The specificity of the MAbs to human C1q, C1r, or C1s can be tested by enzyme linked immunosorbent assay (ELISA), Western immunoblotting, or other immunochemical techniques.

The inhibitory activity on complement activation of antibodies identified in the screening process can be assessed by hemolytic assays using either unsensitized rabbit or guinea pig RBCs for the alternative complement pathway, or sensitized chicken or sheep RBCs for the classical complement pathway. Those hybridomas that exhibit an inhibitory activity specific for the classical complement pathway are cloned by limiting dilution. The antibodies are purified for characterization for specificity to human C1q, C1r, or C1s by the assays described above.

Based on the molecular structures of the variable regions of the anti -C1q, -C1r, or - C1s antibodies, molecular modeling and rational molecular design may be used to generate and screen small molecules that mimic the molecular structures of the binding region of the antibodies and inhibit the activities of C1q, C1r, or C1s. These small molecules can be peptides, peptidomimetics, oligonucleotides, or organic compounds. The mimicking molecules can be used as inhibitors of complement activation in inflammatory indications and autoimmune diseases. Alternatively, one can use large-scale screening procedures commonly used in the field to isolate suitable small molecules from libraries of combinatorial compounds.

A suitable dosage of an antibody as disclosed herein may be between 10 and 500 $\mu$g/ml of serum. The actual dosage can be determined in clinical trials following the conventional methodology for determining optimal dosages, i.e., administering various dosages and determining which doses provide suitable efficacy without undesirable side-effects.

Before the advent of recombinant DNA technology, proteolytic enzymes (proteases) that cleave polypeptide sequences were used to dissect the structure of antibody molecules and to determine which parts of the molecule are responsible for its various functions. Limited digestion with the protease papain cleaves antibody molecules into three fragments. Two fragments, known as Fab fragments, are identical and contain the antigen-binding activity. The Fab fragments correspond to the two identical arms of the antibody molecule, each of which consists of a complete light chain paired with the $V_H$ and $C_H1$ domains of a heavy chain. The other fragment contains no antigen binding activity but was originally observed to crystallize readily, and for this reason was named the Fc fragment (Fragment crystallizable).

A Fab molecule is an artificial ~50-kDa fragment of the Ig molecule with a heavy chain lacking constant domains $C_H2$ and $C_H3$. Two heterophilic ($V_L$-$V_H$ and $C_L$-$C_H1$) domain interactions underlie the two-chain structure of the Fab molecule, which is further stabilized by a disulfide bridge between $C_L$ and $C_H1$. Fab and IgG have identical antigen binding sites formed by six complementarity-determining regions (CDRs), three each from $V_L$ and $V_H$ (LCDR1, LCDR2, LCDR3 and HCDR1, HCDR2, HCDR3). The CDRs define the hypervariable antigen binding site of antibodies. The highest sequence variation is found in LCDR3 and HCDR3, which in natural immune systems are generated by the rearrangement of $V_L$ and $J_L$ genes or $V_H$, $D_H$ and $J_H$ genes, respectively. LCDR3 and HCDR3 typically form the core of the antigen binding site. The conserved regions that connect and display the six CDRs are referred to as framework regions. In the three-dimensional structure of the variable domain, the framework regions form a sandwich of two opposing antiparallel $\beta$-sheets that are linked by hypervariable CDR loops on the outside and by a conserved disulfide bridge on the inside.

The publications discussed herein are provided solely for their disclosure prior to the filing date of the present application. Nothing herein is to be construed as an admission that the present disclosure is not entitled to antedate such publication by virtue of prior disclosure. Further, the dates of publication provided can be different from the actual publication dates, which may need to be independently confirmed.

## Anti-Complement C1q Antibodies

The antibodies of this disclosure specifically recognize complement factor C1q and/or C1q in the C1 complex of the classical complement activation pathway. The recognized complement factor may be derived, without limitation, from any organism having a complement system, including any mammalian organism such as human, mouse, rat, rabbit, monkey, dog, cat, cow, horse, camel, sheep, goat, or pig.

As used herein "C1 complex" refers to a protein complex that may include, without limitation, one C1q protein, two C1r proteins, and two C1s proteins (e.g., $C1qr_2s_2$).

As used herein "complement factor C1q" refers to both wild type sequences and naturally occurring variant sequences.

A non-limiting example of a complement factor C1q recognized by antibodies of this disclosure is human C1q, including the three polypeptide chains A, B, and C:

C1q, chain A (homo sapiens), Accession No. Protein Data Base: NP_057075.1; GenBank No.: NM_015991:

>gi|7705753|ref|NP_057075.1|complement C1q subcomponent subunit A precursor [Homo sapiens]

(SEQ ID NO:1)

MEGPRGWLVLCVLAISLASMVTEDLCRAPDGKKGEAGRPGRRGRPGLKGEQGEPG
APGIRTGIQGLKGDQGEPGPSGNPGKVGYPGPSGPLGARGIPGIKGTKGSPGNIKDQP
RPAFSAIRRNPPMGGNVVIFDTVITNQEEPYQNHSGRFVCTVPGYYYFTFQVLSQWEI
CLSIVSSSRGQVRRSLGFCDTTNKGLFQVVSGGMVLQLQQGDQVWVEKDPKKGHIY
QGSEADSVFSGFLIFPSA.

C1q, chain B (homo sapiens), Accession No. Protein Data Base: NP_000482.3; GenBank No.: NM_000491.3:

>gi|7298828|ref|NP_000482.3|complement Clq subcomponent subunit B precursor [Homo sapiens]

(SEQ ID NO:2)

MMMKIPWGSIPVLMLLLLLGLIDISQAQLSCTGPPAIPGIPGIPGTPGPDGQPGTPGIKG
EKGLPGLAGDHGEFGEKGDPGIPGNPGKVGPKGPMGPKGGPGAPGAPGPKGESGDY
KATQKIAFSATRTINVPLRRDQTIRFDHVITNMNNNYEPRSGKFTCKVPGLYYFTYHA
SSRGNLCVNLMRGRERAQKVVTFCDYAYNTFQVTTGGMVLKLEQGENVFLQATDK
NSLLGMEGANSIFSGFLLFPDMEA.

C1q, chain C (homo sapiens), Accession No. Protein Data Base: NP_001107573.1; GenBank No.: NM_001114101.1:

>gi|166235903|ref|NP_001107573.1|complement C1q subcomponent subunit C precursor [Homo sapiens]

(SEQ ID NO:3)

MDVGPSSLPHLGLKLLLLLLLLPLRGQANTGCYGIPGMPGLPGAPGKDGYDGLPGPK
GEPGIPAIPGIRGPKGQKGEPGLPGHPGKNGPMGPPGMPGVPGPMGIPGEPGEEGRYK
QKFQSVFTVTRQTHQPPAPNSLIRFNAVLTNPQGDYDTSTGKFTCKVPGLYYFVYHA
SHTANLCVLLYRSGVKVVTFCGHTSKTNQVNSGGVLLRLQVGEEVWLAVNDYYD
MVGIQGSDSVFSGFLLFPD.

Accordingly, a humanized anti-C1q antibody of the present disclosure may bind to polypeptide chain A, polypeptide chain B, and/or polypeptide chain C of a C1q protein. In some implementations, a humanized anti-C1q antibody of the present disclosure binds to polypeptide chain A, polypeptide chain B, and/or polypeptide chain C of human C1q or a homolog thereof, such as mouse, rat, rabbit, monkey, dog, cat, cow, horse, camel, sheep, goat, or pig C1q.

Suitable inhibitors include an antibody that binds complement C1q protein (i.e., an anti-complement C1q antibody, also referred to herein as an anti-C1q antibody and a C1q antibody) and a nucleic acid molecule that encodes such an antibody for a method of preventing, reducing risk of developing, or treating a brain injury, preferably a traumatic brain injury, hypoxic brain injury, brain infection, or stroke.

Other anti-C1q antibodies suitable for binding to C1q protein are well-known in the art and include, for example, antibodies Cat #: AF2379, AF1696, MAB1696, and MAB23791 (R&D System), NBP1-87492, NB100-64420, H00000712-B01P, H00000712-D01P, and H00000712-D01 (Novus Biologicals), MA1-83963, MA1-40311, PA5-14208, PA5-29586, and PA1-36177 (ThermoFisher Scientific), ab71940, ab11861, ab4223, ab72355, ab182451, ab46191, ab227072, ab182940, ab216979, and ab235454 (abcam), etc. Moreover, multiple siRNA, shRNA, CRISPR constructs for reducing C1q expression can be found in the commercial product lists of the above-referenced companies, such as SiRNA product # sc-43651, sc-44962, sc-105153, sc-141842, ShRNA product # sc-43651-SH, sc-43651-V, sc-44962-SH, sc-44962-V, sc-105153-SH, sc-105153-V, sc-141842-SH, sc-141842-V, CRISPR product # sc-419385, sc-419385-HDR, sc-419385-NIC, sc-419385-NIC-2, sc-402156, sc-402156-KO-2, sc-404309, sc-404309-HDR, sc-404309-NIC, sc-404309-NIC-2, sc-419386, sc-419386-HDR, sc-419386-NIC, sc-419386-NIC-2 (Santa Cruz Biotechnology, etc).

Light Chain and Heavy Chain Variable Domain Sequences of Antibody M1

Using standard techniques, the nucleic acid and amino acid sequences encoding the light chain variable and the heavy chain variable domain of antibody M1 were determined. The amino acid sequence of the light chain variable domain of antibody M1 is:

DVQITQSPSYLAASPGETITINC**RASKSINKYLA**WYQEKPGKTNKLLIY**SGSTLQS**GIP
SRFSGSGSGTDFTLTISSLEPEDFAMYYC**QQHNEYPLT**FGAGTKLELK (SEQ ID
NO:4).

The hyper variable regions (HVRs) of the light chain variable domain are depicted in bolded and underlined text. In some implementations, the HVR-L1 of the M1 light chain variable domain has the sequence RASKSINKYLA (SEQ ID NO:5), the HVR-L2 of the M1 light chain variable domain has the sequence SGSTLQS (SEQ ID NO:6), and the HVR-L3 of the M1 light chain variable domain has the sequence QQHNEYPLT (SEQ ID NO:7).

The amino acid sequence of the heavy chain variable domain of antibody M1 is:

QVQLQQPGAELVKPGASVKLSCKSS**GYHFTSYWMH**WVKQRPGQGLEWIG**VIHPN**
**SGSINYNEKFES**KATLTVDKSSSTAYMQLSSLTSEDSAVYYCAG**ERDSTEVLPMDY**
WGQGTSVTVSS (SEQ ID NO:8).

The hyper variable regions (HVRs) of the heavy chain variable domain are depicted in bolded and underlined text. In some implementations, the HVR-H1 of the M1 heavy chain variable domain has the sequence GYHFTSYWMH (SEQ ID NO:9), the HVR-H2 of the M1 heavy chain variable domain has the sequence VIHPNSGSINYNEKFES (SEQ ID NO:10), and the HVR-H3 of the M1 heavy chain variable domain has the sequence ERDSTEVLPMDY (SEQ ID NO: 11).

The nucleic acid sequence encoding the light chain variable domain was determined to be:

GATGTCCAGATAACCCAGTCTCCATCTTATCTTGCTGCATCTCCTGGAGAAACCA
TTACTATTAATTGCAGGGCAAGTAAGAGCATTAACAAATATTTAGCCTGGTATCA
AGAGAAACCTGGGAAAACTAATAAGCTTCTTATCTACTCTGGATCCACTTTGCAA
TCTGGAATTCCATCAAGGTTCAGTGGCAGTGGATCTGGTACAGATTTCACTCTCA
CCATCAGTAGCCTGGAGCCTGAAGATTTTGCAATGTATTACTGTCAACAACATAA
TGAATACCCGCTCACGTTCGGTGCTGGGACCAAGCTGGAGCTGAAA (SEQ ID
NO:12).

The nucleic acid sequence encoding the heavy chain variable domain was determined to be:

CAGGTCCAACTGCAGCAGCCTGGGGCTGAGCTGGTAAAGCCTGGGGCTTCAGTG
AAGTTGTCCTGCAAGTCTTCTGGCTACCATTTCACCAGCTACTGGATGCACTGGG
TGAAGCAGAGGCCTGGACAAGGCCTTGAGTGGATTGGAGTGATTCATCCTAATA
GTGGTAGTATTAACTACAATGAGAAGTTCGAGAGCAAGGCCACACTGACTGTAG
ACAAATCCTCCAGCACAGCCTACATGCAACTCAGCAGCCTGACATCTGAGGACT
CGGCGGTCTATTATTGTGCAGGAGAGAGAGATTCTACGGAGGTTCTCCCTATGGA
CTACTGGGGTCAAGGAACCTCAGTCACCGTCTCCTCA (SEQ ID NO:13).

## Deposit of Material

The following materials have been deposited according to the Budapest Treaty in the American Type Culture Collection, ATCC Patent Depository, 10801 University Blvd., Manassas, Va. 20110-2209, USA (ATCC):

| Sample ID | Isotype | Deposit Date | ATCC Accession No. |
|---|---|---|---|
| Mouse hybridoma C1qM1 7788-1(M) 051613 producing anti-C1q antibody M1 | IgG1, kappa | Jun. 6, 2013 | PTA-120399 |

The hybridoma cell line producing the M1 antibody (mouse hybridoma C1qM1 7788-1(M) 051613) has been deposited with ATCC under conditions that assure that access to the culture will be available during pendency of the patent application and for a period of 30 years, or 5 years after the most recent request, or for the effective life of the patent, whichever is longer. A deposit will be replaced if the deposit becomes nonviable during that period. The deposit is available as required by foreign patent laws in countries wherein counterparts of the subject application, or its progeny are filed..

Disclosed herein are methods of administering an anti-C1q antibody comprising a light chain variable domain and a heavy chain variable domain. The antibody may bind to at least human C1q, mouse C1q, or rat C1q. The antibody may be a humanized antibody, a chimeric antibody, or a human antibody. The light chain variable domain comprises the HVR-L1, HVR-L2, and HVR-L3 of the monoclonal antibody M1 produced by a hybridoma cell line deposited with Accession Number PTA-120399. The heavy chain variable domain comprises the HVR-H1, HVR-H2, and HVR-H3 of the monoclonal antibody M1 produced by a hybridoma cell line deposited with ATCC Accession Number PTA-120399.

In some implementations, the amino acid sequence of the light chain variable domain and heavy chain variable domain comprise one or more of SEQ ID NO:5 of HVR-L1, SEQ ID NO:6 of HVR-L2, SEQ ID NO:7 of HVR-L3, SEQ ID NO:9 of HVR-H1, SEQ ID NO:10 of HVR-H2, and SEQ ID NO:11 of HVR-H3.

The antibody may comprise a light chain variable domain amino acid sequence that is at least 85%, 90%, or 95% identical to SEQ ID NO:4, preferably while retaining the HVR-L1 RASKSINKYLA (SEQ ID NO:5), the HVR-L2 SGSTLQS (SEQ ID NO:6), and the HVR-L3 QQHNEYPLT (SEQ ID NO:7). The antibody may comprise a heavy chain variable domain amino acid sequence that is at least 85%, 90%, or 95% identical to SEQ ID NO:8, preferably while retaining the HVR-H1 GYHFTSYWMH (SEQ ID NO:9), the HVR-H2 VIHPNSGSINYNEKFES (SEQ ID NO:10), and the HVR-H3 ERDSTEVLPMDY (SEQ ID NO:11).

Disclosed herein are methods of administering an anti-C1q antibody, which inhibits the interaction between C1q and an autoantibody. In preferred implementations, the anti-C1q antibody causes clearance of C1q from the circulation or tissue.

The anti-C1q antibody may bind to a C1q protein, and binds to one or more amino acids of the C1q protein within amino acid residues selected from (a) amino acid residues 196-226 of SEQ ID NO:1 (SEQ ID NO:16), or amino acid residues of a C1q protein chain A (C1qA) corresponding to amino acid residues 196-226 (GLFQVVSGGMVLQLQQGDQVWVEKDPKKGHI) of SEQ ID NO:1 (SEQ ID NO:16); (b) amino acid residues 196-221 of SEQ ID NO:1 (SEQ ID NO:17), or amino acid residues of a C1qA corresponding to amino acid residues 196-221 (GLFQVVSGGMVLQLQQGDQVWVEKDP) of SEQ ID. NO: 1 (SEQ ID NO: 17); (c) amino acid residues 202-221 of SEQ ID NO:1 (SEQ ID NO: 18), or amino acid residues of a C1qA corresponding to amino acid residues 202-221 (SGGMVLQLQQGDQVWVEKDP) of SEQ ID NO: 1 (SEQ ID NO: 18); (d) amino acid residues 202-219 of SEQ ID NO: 1 (SEQ ID NO: 19), or amino acid residues of a C1qA corresponding to amino acid residues 202-219 (SGGMVLQLQQGDQVWVEK) of SEQ ID NO:1 (SEQ ID NO: 19); and (e) amino acid residues Lys 219 and/or Ser 202 of SEQ ID NO: 1, or amino acid residues of a C1qA corresponding Lys 219 and/or Ser 202 of SEQ ID NO: 1.

In some implementations, the antibody further binds to one or more amino acids of the C1q protein within amino acid residues selected from: (a) amino acid residues 218-240 of SEQ ID NO:3 (SEQ ID NO:20) or amino acid residues of a C1q protein chain C (C1qC) corresponding to amino acid residues 218-240 (WLAVNDYYDMVGI QGSDSVFSGF) of SEQ ID NO:3 (SEQ ID NO:20); (b) amino acid residues 225-240 of SEQ ID NO:3 (SEQ ID NO:21) or amino acid residues of a C1qC corresponding to amino acid residues 225-240 (YDMVGI QGSDSVFSGF) of SEQ ID NO:3 (SEQ ID NO:21); (c) amino acid residues 225-232 of SEQ ID NO:3 (SEQ ID NO:22) or amino acid residues of a C1qC corresponding to amino acid residues 225-232 (YDMVGIQG) of SEQ ID NO:3 (SEQ ID NO:22); (d) amino acid residue Tyr 225 of SEQ ID NO:3 or an amino acid residue of a C1qC corresponding to amino acid residue Tyr 225 of SEQ ID NO:3; (e) amino acid residues 174-196 of SEQ ID NO:3 (SEQ ID NO:23) or amino acid residues of a C1qC corresponding to amino acid residues 174-196 (HTANLCVLLYRSGVKVVTFCGHT) of SEQ ID NO:3 (SEQ ID NO:23); (f) amino acid residues 184-192 of SEQ ID NO:3 (SEQ ID NO:24) or amino acid residues of a C1qC corresponding to amino acid residues 184-192 (RSGVKVVTF) of SEQ ID NO:3 (SEQ ID NO:24); (g) amino acid residues 185-187 of SEQ ID NO:3 or amino acid residues of a C1qC corresponding to amino acid residues 185-187 (SGV) of SEQ ID NO:3; (h) amino acid residue Ser 185 of SEQ ID NO:3 or an amino acid residue of a C1qC corresponding to amino acid residue Ser 185 of SEQ ID NO:3.

In certain implementations, the anti-C1q antibody binds to amino acid residue Lys 219 and Ser 202 of the human C1qA as shown in SEQ ID NO: 1 or amino acids of a human C1qA corresponding to Lys 219 and Ser 202 as shown in SEQ ID NO: 1, and amino acid residue Tyr 225 of the human C1qC as shown in SEQ ID NO:3 or an amino acid residue of a human C1qC corresponding to Tyr 225 as shown in SEQ ID NO:3. In certain implementations, the anti-C1q antibody binds to amino acid residue Lys 219 of the human C1qA as shown in SEQ ID NO: 1 or an amino acid residue of a human C1qA corresponding to Lys 219 as shown in SEQ ID NO:1, and amino acid residue Ser 185 of the human C1qC as shown in SEQ ID NO:3 or an amino acid residue of a human C1qC corresponding to Ser 185 as shown in SEQ ID NO:3.

In some implementations, the anti-C1q antibody binds to a C1q protein and binds to one or more amino acids of the C1q protein within amino acid residues selected from: (a) amino acid residues 218-240 of SEQ ID NO:3 (SEQ ID NO:20) or amino acid residues of a C1qC corresponding to amino acid residues 218-240 (WLAVNDYYDMVGI QGSDSVFSGF) of SEQ ID NO:3 (SEQ ID NO:20); (b) amino acid residues 225-240 of SEQ ID NO:3 (SEQ ID NO:21) or amino acid residues of a C1qC corresponding to amino acid residues 225-240 (YDMVGI QGSDSVFSGF) of SEQ ID NO:3 (SEQ ID NO:21); (c) amino acid residues 225-232 of SEQ ID NO:3 (SEQ ID NO:22) or amino acid residues of a C1qC corresponding to amino acid residues 225-232 (YDMVGIQG) of SEQ ID NO:3 (SEQ ID NO:22); (d) amino acid residue Tyr 225 of SEQ ID NO:3 or an amino acid residue of a C1qC corresponding to amino acid residue Tyr 225 of SEQ ID NO:3; (e) amino acid residues 174-196 of SEQ ID NO:3 (SEQ ID NO:23) or amino acid residues of a C1qC corresponding to amino acid residues 174-196 (HTANLCVLLYRSGVKVVTFCGHT) of SEQ ID NO:3 (SEQ ID NO:23); (f) amino acid residues 184-192 of SEQ ID NO:3 (SEQ ID NO:24) or amino acid residues of a C1qC corresponding to amino acid residues 184-192 (RSGVKVVTF) of SEQ ID NO:3 (SEQ ID NO:24); (g) amino acid residues 185-187 of SEQ ID NO:3 or amino acid residues of a C1qC corresponding to amino acid residues 185-187 (SGV) of SEQ ID NO:3; (h) amino acid residue Ser 185 of SEQ ID NO:3 or an amino acid residue of a C1qC corresponding to amino acid residue Ser 185 of SEQ ID NO:3.

In some implementations, the anti-C1q antibody of this disclosure inhibits the interaction between C1q and C1s. In some implementations, the anti-C1q antibody inhibits the interaction between C1q and C1r. In some implementations the anti-C1q antibody inhibits the interaction between C1q and C1s and between C1q and C1r. In some implementations, the anti-C1q antibody inhibits the interaction between C1q and another antibody, such as an autoantibody. In preferred implementations, the anti-C1q antibody causes clearance of C1q from the circulation or tissue. In some implementations, the antiC1q antibody inhibits the respective interactions, at a stoichiometry of less than 2.5:1; 2.0:1; 1.5:1; or 1.0:1. In some implementations, the C1q antibody inhibits an interaction, such as the C1q-C1s interaction, at approximately equimolar concentrations of C1q and the anti-C1q antibody. In other implementations, the anti-C1q antibody binds to C1q with a stoichiometry of less than 20:1; less than 19.5:1; less than 19:1; less than 18.5:1; less than 18:1; less than 17.5:1; less than 17:1; less than 16.5:1; less than 16:1; less than 15.5:1; less than 15:1; less than 14.5:1; less than 14:1; less than 13.5:1; less than 13:1; less than 12.5:1; less than 12:1; less than 11.5:1; less than 11:1; less than 10.5:1; less than 10:1; less than 9.5:1; less than 9:1; less than 8.5:1; less than 8:1; less than 7.5:1; less than 7:1; less than 6.5:1; less than 6:1; less than 5.5:1; less than 5:1; less than 4.5:1; less than 4:1; less than 3.5:1; less than 3:1; less than 2.5:1; less than 2.0:1; less than 1.5:1; or less than 1.0:1. In certain implementations, the antiC1q antibody binds C1q with a binding stoichiometry that ranges from 20:1 to 1.0:1 or less than1.0:1. In certain implementations, the anti-C1q antibody binds C1q with a binding stoichiometry that ranges from 6:1 to 1.0:1 or less than1.0:1. In certain implementations, the anti-C1q antibody binds C1q with a binding stoichiometry that ranges from 2.5:1 to 1.0:1 or less than1.0:1. In some implementations, the anti-C1q antibody inhibits the interaction between C1q and C1r, or between C1q and C1s, or between C1q and both C1r and C1s. In some implementations, the anti-C1q antibody inhibits the interaction between C1q and C1r, between C1q and C1s, and/or between C1q and both C1r and C1s. In some implementations, the anti-C1q antibody binds to the C1q A-chain. In other implementations, the anti-C1q antibody binds to the C1q B-chain. In other implementations, the anti-C1q antibody binds to the C1q C-chain. In some implementations, the anti-C1q antibody binds to the C1q A-chain, the C1q B-chain and/or the C1q C-chain. In some implementations, the anti-C1q antibody binds to the globular domain of the C1q A-chain, B-chain, and/or C-chain. In other implementations, the anti-C1q antibody binds to the collagen-like domain of the C1q A-chain, the C1q B-chain, and/or the C1q C-chain.

Where antibodies of this disclosure inhibit the interaction between two or more complement factors, such as the interaction of C1q and C1s, or the interaction between C1q and C1r, the interaction occurring in the presence of the antibody may be reduced by at least 10%, at least 20%, at least 30%, at least 40%, at least 50%, at least 60%, at least 70%, at least 80%, at least 90%, at least 95%, or at least 99% relative to a control wherein the antibodies of this disclosure are absent. In certain implementations, the interaction occurring in the presence of the antibody is reduced by an amount that ranges from at least 30% to at least 99% relative to a control wherein the antibodies of this disclosure are absent.

In some implementations, the antibodies of this disclosure inhibit C2 or C4-cleavage by at least 20%, at least 30%, at least 40%, at least 50%, at least 60%, at least 70%, at least 80%, at least 90%, at least 95%, or at least 99%, or by an amount that ranges from at least 30% to at least 99%, relative to a control wherein the antibodies of this disclosure are absent. Methods for measuring C2 or C4-cleavage are well known in the art. The $EC_{50}$ values for antibodies of this disclosure with respect C2 or C4-cleavage may be less than 3 $\mu$g/ml; 2.5 $\mu$g/ml; 2.0 $\mu$g/ml; 1.5 $\mu$g/ml; 1.0 $\mu$g/ml; 0.5 $\mu$g/ml; 0.25 $\mu$g/ml; 0.1 $\mu$g/ml; 0.05 $\mu$g/ml. In some implementations, the antibodies of this disclosure inhibit C2 or C4-cleavage at approximately equimolar concentrations of C1q and the respective anti-C1q antibody.

In some implementations, the antibodies of this disclosure inhibit autoantibody-dependent and complement-dependent cytotoxicity (CDC) by at least 20%, at least 30%, at least 40%, at least 50%, at least 60%, at least 70%, at least 80%, at least 90%, at least 95%, or at least 99%, or by an amount that ranges from at least 30% to at least 99%, relative to a control wherein the antibodies of this disclosure are absent. The $EC_{50}$ values for antibodies of this disclosure with respect to inhibition of autoantibody-dependent and complement-dependent cytotoxicity may be less than 3 $\mu$g/ml; 2.5 $\mu$g/ml; 2.0 $\mu$g/ml; 1.5 $\mu$g/ml; 1.0 $\mu$g/ml; 0.5 $\mu$g/ml; 0.25 $\mu$g/ml; 0.1 $\mu$g/ml; 0.05 $\mu$g/ml.

In some implementations, the antibodies of this disclosure inhibit complement-dependent cell-mediated cytotoxicity (CDCC) by at least 20%, at least 30%, at least 40%, at least 50%, at least 60%, at least 70%, at least 80%, at least 90%, at least 95%, or at least 99%, or by an amount that ranges from at least 30% to at least 99%, relative to a control wherein the antibodies of this disclosure are absent. Methods for measuring CDCC are well known in the art. The $EC_{50}$ values for antibodies of this disclosure with respect CDCC inhibition may be l less than 3 $\mu$g/ml; 2.5 $\mu$g/ml; 2.0 $\mu$g/ml; 1.5 $\mu$g/ml; 1.0 $\mu$g/ml; 0.5 $\mu$g/ml; 0.25 $\mu$g/ml; 0.1 $\mu$g/ml; 0.05 $\mu$g/ml. In some implementations, the antibodies of this disclosure inhibit CDCC but not antibody-dependent cellular cytotoxicity (ADCC).

*Humanized anti-complement C1q Antibodies*

Humanized antibodies of the present disclosure specifically bind to a complement factor C1q and/or C1q protein in the C1 complex of the classical complement pathway. The humanized anti-C1q antibody may specifically bind to human C1q, human and mouse C1q, to rat C1q, or human C1q, mouse C1q, and rat C1q.

In some implementations, the human heavy chain constant region is a human IgG4 heavy chain constant region comprising the amino acid sequence of SEQ ID NO:47, or with at least 70%, at least about 75%, at least about 80%, at least about 85%, at least about 90% homology to SEQ ID NO: 47. The human IgG4 heavy chain constant region may comprise an Fc region with one or more modifications and/or amino acid substitutions according to Kabat numbering. In such cases, the Fc region comprises a leucine to glutamate amino acid substitution at position 248, wherein such a substitution inhibits the Fc region from interacting with an Fc receptor. In some implementations, the Fc region comprises a serine to proline amino acid substitution at position 241, wherein such a substitution prevents arm switching in the antibody.

The amino acid sequence of human IgG4 (S241P L248E) heavy chain constant domain is:

ASTKGPSVFPLAPCSRSTSESTAALGCLVKDYFPEPVTVSWNSGALTSGVHTFPAVLQ SSGLYSLSSVVTVPSSSLGTKTYTCNVDHKPSNTKVDKRVESKYGPPCPPCPAPEFEG GPSVFLFPPKPKDTLMISRTPEVTCVVVDVSQEDPEVQFNWYVDGVEVHNAKTKPR EEQFNSTYRVVSVLTVLHQDWLNGKEYKCKVSNKGLPSSIEKTISKAKGQPREPQVY TLPPSQEEMTKNQVSLTCLVKGFYPSDIAVEWESNGQPENNYKTTPPVLDSDGSFFL YSRLTVDKSRWQEGNVFSCSVMHEALHNHYTQKSLSLSLGK (SEQ ID NO: 47).

The antibody may comprise a heavy chain variable domain and a light chain variable domain, wherein the heavy chain variable domain comprises an amino acid sequence selected from any one of SEQ ID NOs: 31-34, or an amino acid sequence with at least about 90% homology to the amino acid sequence selected from any one of SEQ ID NOs: 31-34. In certain such implementations, the light chain variable domain comprises an amino acid sequence selected from any one of SEQ ID NOs: 35-38, or an amino acid sequence with at least about 90% homology to the amino acid sequence selected from any one of SEQ ID NOs: 35-38.

The amino acid sequence of heavy chain variable domain variant 1 (VH1) is: QVQLVQSGAELKKPGASVKV SCKSS**GYHFTSYWMH**WVKQAPGQGLEWIG**VIHPN SGSINYNEKFES**KATITVDKSTSTAYMQLSSLTSEDSAVYYCA **GERDSTEVLPMDY** WGQGTSVTVSS (SEQ ID NO: 31). The hyper variable regions (HVRs) of VH1 are depicted in bolded and underlined text.

The amino acid sequence of heavy chain variable domain variant 2 (VH2) is: QVQLVQSGAELKKPGASVKV SCKSS**GYHFTSYWMH**WVKQAPGQGLEWIG**VIHPN SGSINYNEKFES**RATITVDKSTSTAYMELSSLRSEDTAVYYCA **GERDSTEVLPMDY** WGQGTTVTVSS (SEQ ID NO: 32). The hyper variable regions (HVRs) of VH2 are depicted in bolded and underlined text.

The amino acid sequence of heavy chain variable domain variant 3 (VH3) is: QVQLVQSGAELKKPGASVKV SCKSS**GYHFTSYWMH**WVKQAPGOGLEWIG**VIHPN SGSINYNEKFES**RVTITVDKSTSTAYMELSSLRSEDTAVYYCA **GERDSTEVLPMDY** WGQGTTVTVSS (SEQ ID NO: 33). The hyper variable regions (HVRs) of VH3 are depicted in bolded and underlined text.

The amino acid sequence of heavy chain variable domain variant 4 (VH4) is: QVQLVQSGAELKKPGASVKV SCKSS**GYHFTSYWMH**WVRQAPGQGLEWIG**VIHPN SGSINYNEKFES**RVTITVDKSTSTAYMELSSLRSEDTAVYYCA **GERDSTEVLPMDY** WGQGTTVTVSS (SEQ ID NO: 34). The hyper variable regions (HVRs) of VH4 are depicted in bolded and underlined text.

The amino acid sequence of kappa light chain variable domain variant 1 (Vκ1) is: DVQITQSPSYLAASLGER ATINC**RASKSINKYLA**WYQQKPGKTNKLLIY**SGSTLQS**G IPARFSGSGSGTDFTLTISSLEPEDFAMYYC**QQHNEYPL T**FGQGTKLEIK (SEQ ID NO: 35). The hyper variable regions (HVRs) of Vκ1 are depicted in bolded and underlined text.

The amino acid sequence of kappa light chain variable domain variant 2 (Vκ2) is: DVQITQ SP S SLSASLGERATINC**RASKSINKYLA**WYQQKPGKANKLLIY**SGSTLQS**GI          PARFSGSGSGTDFTLTISSLEPEDFAMYYC**QQHNEYPLT**FGQGTKLEIK (SEQ ID NO: 36). The hyper variable regions (HVRs) of Vκ2 are depicted in bolded and underlined text.

The amino acid sequence of kappa light chain variable domain variant 3 (Vκ3) is: DVQITQ SP S SLSASLGERATINC**RASKSINKYLA**WYQQKPGKAPKLLIY**SGSTLQ**SGI          PARFSGSGSGTDFTLTISSLEPEDFAMYYC**QQHNEYPLT**FGQGTKLEIK (SEQ ID NO: 37). The hyper variable regions (HVRs) of Vκ3 are depicted in bolded and underlined text.

The amino acid sequence of kappa light chain variable domain variant 4 (Vκ4) is: DIQLTQSPSSLSASLGER ATINC**RASKSINKYLA**WYQQKPGKAPKLLIY**SGSTLQS**GI          PARFSGSGSGTDFTLTISSLEPEDFAMYYC**QQHNEYPLT**FGQGTKLEIK (SEQ ID NO: 38). The hyper variable regions (HVRs) of Vκ4 are depicted in bolded and underlined text.

The antibody may comprise a light chain variable domain amino acid sequence that is at least 85%, 90%, or 95% identical to SEQ ID NO:35-38 while retaining the HVR-L1 RASKSINKYLA (SEQ ID NO:5), the HVR-L2 SGSTLQS (SEQ ID NO:6), and the HVR-L3 QQHNEYPLT (SEQ ID NO:7). The antibody may comprise a heavy chain variable domain amino acid sequence that is at least 85%, 90%, or 95% identical to SEQ ID NO:31-34 while retaining the HVR-H1 GYHFTSYWMH (SEQ ID NO:9), the HVR-H2 VIHPNSGSINYNEKFES (SEQ ID NO:10), and the HVR-H3 ERDSTEVLPMDY (SEQ ID NO:11).

In some implementations, humanized anti-C1q antibodies of the present disclosure include a heavy chain variable region that contains an Fab region and a heavy chain constant regions that contains an Fc region, where the Fab region specifically binds to a C1q protein of the present disclosure, but the Fc region is incapable of binding the C1q protein. In some implementations, the Fc region is from a human IgG1, IgG2, IgG3, or IgG4 isotype. In some implementations, the Fc region is incapable of inducing complement activity and/or incapable of inducing antibody-dependent cellular cytotoxicity (ADCC). In some implementations, the Fc region comprises one or more modifications, including, without limitation, amino acid substitutions. In certain implementations, the Fc region of humanized anti-C1q antibodies of the present disclosure comprise an amino acid substitution at position 248 according to Kabat numbering convention or a position corresponding to position 248 according to Kabat numbering convention, and/or at position 241 according to Kabat numbering convention or a position corresponding to position 241 according to Kabat numbering convention. In some implementations, the amino acid substitution at position 248 or a positions corresponding to position 248 inhibits the Fc region from interacting with an Fc receptor. In some implementations, the amino acid substitution at position 248 or a positions corresponding to position 248 is a leucine to glutamate amino acid substitution. In some implementations, the amino acid substitution at position 241 or a positions corresponding to position 241 prevents arm switching in the antibody. In some implementations, the amino acid substitution at position 241 or a positions corresponding to position 241 is a serine to proline amino acid substitution. In certain implementations, the Fc region of humanized anti-C1q antibodies of the present disclosure comprises the amino acid sequence of SEQ ID NO: 47, or an amino acid sequence with at least about 70%, at least about 75%, at least about 80% at least about 85% at least about 90%, or at least about 95% homology to the amino acid sequence of SEQ ID NO: 47.

In some implementations, humanized anti-C1q antibodies of the present disclosure may bind to a C1q protein and binds to one or more amino acids of the C1q protein within amino acid residues selected from (a) amino acid residues 196-226 of SEQ ID NO: 1 (SEQ ID NO: 16), or amino acid residues of a C1q protein chain A (C1qA) corresponding to amino acid residues 196-226 (GLFQVVSGGMVLQLQQGDQVWVEKDPKKGHI) of SEQ ID NO: 1 (SEQ ID NO: 16); (b) amino acid residues 196-221 of SEQ ID NO: 1 (SEQ ID NO:17), or amino acid residues of a C1qA corresponding to amino acid residues 196-221 (GLFQVVSGGMVLQLQQGDQVWVEKDP) of SEQ ID. NO: 1 (SEQ ID NO:17); (c) amino acid residues 202-221 of SEQ ID NO:1 (SEQ ID NO: 18), or amino acid residues of a C1qA corresponding to amino acid residues 202-221 (SGGMVLQLQQGDQVWVEKDP) of SEQ ID NO: 1 (SEQ ID NO: 18); (d) amino acid residues 202-219 of SEQ ID NO: 1 (SEQ ID NO: 19), or amino acid residues of a C1qA corresponding to amino acid residues 202-219 (SGGMVLQLQQGDQVWVEK) of SEQ ID NO: 1 (SEQ ID NO: 19); and (e) amino acid residues Lys 219 and/or Ser 202 of SEQ ID NO: 1, or amino acid residues of a C1qA corresponding Lys 219 and/or Ser 202 of SEQ ID NO: 1.

In some implementations, the humanized anti-C1q antibodies may further binds to one or more amino acids of the C1q protein within amino acid residues selected from: (a) amino acid residues 218-240 of SEQ ID NO: 3 (SEQ ID NO:20) or amino acid residues of a C1q protein chain C (C1qC) corresponding to amino acid residues 218-240 (WLAVNDYYDMVGI QGSDSVFSGF) of SEQ ID NO: 3 (SEQ ID NO:20); (b) amino acid residues 225-240 of SEQ ID NO: 3 (SEQ ID NO:21) or amino acid residues of a C1qC corresponding to amino acid residues 225-240 (YDMVGI QGSDSVFSGF) of SEQ ID NO: 3 (SEQ ID NO:21); (c) amino acid residues 225-232 of SEQ ID NO: 3 (SEQ ID NO:22) or amino acid residues of a C1qC corresponding to amino acid residues 225-232 (YDMVGIQG) of SEQ ID NO: 3 (SEQ ID NO:22); (d) amino acid residue Tyr 225 of SEQ ID NO: 3 or an amino acid residue of a C1qC corresponding to amino acid residue Tyr 225 of SEQ ID NO: 3; (e) amino acid residues 174-196 of SEQ ID NO: 3 (SEQ ID NO:23) or amino acid residues of a C1qC corresponding to amino acid residues 174-196 (HTANLCVLLYRSGVKVVTFCGHT) of SEQ ID NO: 3 (SEQ ID NO:23); (f) amino acid residues 184-192 of SEQ ID NO: 3 (SEQ ID NO:24) or amino acid residues of a C1qC corresponding to amino acid residues 184-192 (RSGVKVVTF) of SEQ ID NO: 3 (SEQ ID NO:24); (g) amino acid residues 185-187 of SEQ ID NO: 3 or amino acid residues of a C1qC corresponding to amino acid residues 185-187 (SGV) of SEQ ID NO: 3; (h) amino acid residue Ser 185 of SEQ ID NO: 3 or an amino acid residue of a C1qC corresponding to amino acid residue Ser 185 of SEQ ID NO: 3.

In certain implementations, humanized anti-C1q antibodies of the present disclosure may bind to amino acid residue Lys 219 and Ser 202 of the human C1qA as shown in SEQ ID NO: 1 or amino acids of a human C1qA corresponding to Lys 219 and Ser 202 as shown in SEQ ID NO: 1, and amino acid residue Tyr 225 of the human C1qC as shown in SEQ ID NO: 3 or an amino acid residue of a human C1qC corresponding to Tyr 225 as shown in SEQ ID NO: 3. In certain implementations, the anti-C1q antibody binds to amino acid residue Lys 219 of the human C1qA as shown in SEQ ID NO: 1 or an amino acid residue of a human C1qA corresponding to Lys 219 as shown in SEQ ID NO: 1, and amino acid residue Ser 185 of the human C1qC as shown in SEQ ID NO: 3 or an amino acid residue of a human C1qC corresponding to Ser 185 as shown in SEQ ID NO: 3.

In some implementations, humanized anti-C1q antibodies of the present disclosure may bind to a C1q protein and binds to one or more amino acids of the C1q protein within amino acid residues selected from: (a) amino acid residues 218-240 of SEQ ID NO: 3 (SEQ ID NO:20) or amino acid residues of a C1qC corresponding to amino acid residues 218-240 (WLAVNDYYDMVGI QGSDSVFSGF) of SEQ ID NO: 3 (SEQ ID NO:20); (b) amino acid residues 225-240 of SEQ ID NO: 3 (SEQ ID NO:21) or amino acid residues of a C1qC corresponding to amino acid residues 225-240 (YDMVGI QGSDSVFSGF) of SEQ ID NO: 3 (SEQ ID NO:21); (c) amino acid residues 225-232 of SEQ ID NO: 3 (SEQ ID NO:22) or amino acid residues of a C1qC corresponding to amino acid residues 225-232 (YDMVGIQG) of SEQ ID NO: 3 (SEQ ID NO:22); (d) amino acid residue Tyr 225 of SEQ ID NO: 3 or an amino acid residue of a C1qC corresponding to amino acid residue Tyr 225 of SEQ ID NO: 3; (e) amino acid residues 174-196 of SEQ ID NO: 3 (SEQ ID NO:23) or amino acid residues of a C1qC corresponding to amino acid residues 174-196 (HTANLCVLLYRSGVKVVTFCGHT) of SEQ ID NO: 3 (SEQ ID NO:23); (f) amino acid residues 184-192 of SEQ ID NO: 3 (SEQ ID NO:24) or amino acid residues of a C1qC corresponding to amino acid residues 184-192 (RSGVKVVTF) of SEQ ID NO: 3 (SEQ ID NO:24); (g) amino acid residues 185-187 of SEQ ID NO: 3 or amino acid residues of a C1qC corresponding to amino acid residues 185-187 (SGV) of SEQ ID NO: 3; (h) amino acid residue Ser 185 of SEQ ID NO: 3 or an amino acid residue of a C1qC corresponding to amino acid residue Ser 185 of SEQ ID NO: 3.

*Anti-Complement C1s Antibodies*

Suitable inhibitors include an antibody that binds complement C1s protein (i.e., an anti-complement C1s antibody, also referred to herein as an anti-C1s antibody and a C1s antibody) and a nucleic acid molecule that encodes such an antibody. Complement C1s is an attractive target as it is upstream in the complement cascade and has a narrow range of substrate specificity. Furthermore it is possible to obtain antibodies (for example, but not limited to, monoclonal antibodies) that specifically bind the activated form of C1s.

In certain aspects, disclosed herein are methods of administering an anti-C1s antibody. The antibody may be a murine, humanized, or chimeric antibody. In some implementations, the light chain variable domain comprises HVR-L1, HVR-L2, and HVR-L3, and the heavy chain comprises HVR-H1, HVR-H2, and HVR-H3 of a murine anti-human C1s monoclonal antibody 5A1 produced by a hybridoma cell line deposited with ATCC on 5/15/2013 or progeny thereof (ATCC Accession No. PTA-120351). In other implementations, the light chain variable domain comprises the HVR-L1, HVR-L2, and HVR-L3 and the heavy chain variable domain comprises the HVR-H1, HVR-H2, and HVR-H3 of a murine anti-human C1s monoclonal antibody 5C12 produced by a hybridoma cell line deposited with ATCC on 5/15/2013, or progeny thereof (ATCC Accession No. PTA-120352).

In some implementations, antibodies specifically bind to and inhibit a biological activity of C1s or the C1s proenzyme, such as C1s binding to C1q, C1s binding to C1r, or C1s binding to C2 or C4. The biological activity may be a proteolytic enzyme activity of C1s, the conversion of the C1s proenzyme to an active protease, or proteolytic cleavage of C2 or C4. In certain implementations, the biological activity is activation of the classical complement activation pathway, activation of antibody and complement dependent cytotoxicity, or C1F hemolysis.

Disclosed herein are methods of administering a humanized monoclonal antibody that specifically binds an epitope within a region encompassing domains IV and V of complement component C1s. In some cases, the antibody inhibits binding of C1s to complement component 4 (C4) and/or does not inhibit protease activity of C1s. In some implementations, the method comprises administering a humanized monoclonal antibody that binds complement component C1s in a C1 complex with high avidity.

Disclosed herein are methods of administering an anti-C1s antibody with one or more of the complementarity determining regions (CDRs) of an antibody light chain variable region comprising amino acid sequence SEQ ID NO:57 and/or one or more of the CDRs of an antibody heavy chain variable region comprising amino acid sequence SEQ ID NO:58. The anti-C1s antibody may bind a human or rat complement C1s protein. In some implementations, an anti-C1s antibody inhibits cleavage of at least one substrate cleaved by complement C1s protein.

In certain implementations, the antibody comprises: a) a complementarity determining region (CDR) having an amino acid sequence selected from SEQ ID NO:51, SEQ ID NO:52, SEQ ID NO:53, SEQ ID NO:54, SEQ ID NO:55, and SEQ ID NO:56; and/ or b) a CDR having an amino acid sequence selected from SEQ ID NO:62, SEQ ID NO:63, SEQ ID NO:53, SEQ ID NO:64, SEQ ID NO:65: and SEQ ID NO:66.

The antibody may comprise a CDR-L1 having amino acid sequence SEQ ID NO:51, a CDR-L2 having amino acid sequence SEQ ID NO:52, a CDR-L3 having amino acid sequence SEQ ID NO:53, a CDR-H1 having amino acid sequence SEQ ID NO:54, a CDR-H2 having amino acid sequence SEQ ID NO:55, and a CDR-H3 having amino acid sequence SEQ ID NO:56.

In other implementations, the antibody may comprise light chain CDRs of a variable region with an amino acid sequence of SEQ ID NO:67, and/or heavy chain CDRs of a variable region with an amino acid sequence of SEQ ID NO:68.

The antibody can be a humanized antibody that specifically binds complement component C1s, wherein the antibody competes for binding the epitope with an antibody that comprises one or more of the CDRs of an antibody light chain variable region comprising amino acid sequence SEQ ID NO:57 or SEQ ID NO:67, and/or one or more of the CDRs of an antibody heavy chain variable region comprising amino acid sequence SEQ ID NO:58 or SEQ ID NO:68.

In other instances, the antibody can be a humanized antibody that specifically binds complement C1s, wherein the antibody is selected from: a) a humanized antibody that specifically binds an epitope within the complement C1s protein, wherein the antibody competes for binding the epitope with an antibody that comprises a CDR having an amino acid sequence selected from SEQ ID NO:51, SEQ ID NO:52, SEQ ID NO:53, SEQ ID NO:54, SEQ ID NO:55, and SEQ ID NO:56; and b) a humanized antibody that specifically binds an epitope within the complement C1s protein, wherein the antibody competes for binding the epitope with an antibody that comprises a CDR having an amino acid sequence selected from SEQ ID NO:62, SEQ ID NO:63, SEQ ID NO:53, SEQ ID NO:64, SEQ ID NO:65, and SEQ ID NO:66. In some cases, the antibody competes for binding the epitope with an antibody that comprises heavy and light chain CDRs comprising: a) SEQ ID NO:51, SEQ ID NO:52, SEQ ID NO:53, SEQ ID NO:54, SEQ ID NO:55, and SEQ ID NO:56; or b) SEQ ID NO:62, SEQ ID NO:63, SEQ ID NO:53, SEQ ID NO:64, SEQ ID NO:65, and SEQ ID NO:66.

The antibody may comprise a light chain region and a heavy chain region that are present in separate polypeptides. The antibody may comprise an Fc region.

Disclosed herein is an anti-C1s antibody comprising a light chain variable region of an amino acid sequence that is 90% identical to amino acid sequence SEQ ID NO:57, and a heavy chain variable region comprising an amino acid sequence that is 90% identical to amino acid sequence SEQ ID NO:58.

The anti-C1s antibody may be selected from an antigen binding fragment, Ig monomer, a Fab fragment, a F(ab')$_2$ fragment, a Fd fragment, a scFv, a scAb, a dAb, a Fv, a single domain heavy chain antibody, a single domain light chain antibody, a mono-specific antibody, a bi-specific antibody, or a multi-specific antibody.

Disclosed herein are methods of administering an antibody that competes for binding the epitope bound by antibody IPN003 (also referred to herein as "IPN-M34" or "M34" or "TNT003"), *e.g.,* an antibody comprising a variable domain of antibody IPN003, such as antibody IPN003.

In some implementations, the method comprises administering an antibody that specifically binds an epitope within a complement C1s protein. In some implementations, the isolated anti-C1s antibody binds an activated C1s protein. In some implementations, the isolated anti-C1s antibody binds an inactive form of C1s. In other instances, the isolated antiC1s antibody binds both an activated C1s protein and an inactive form of C1s.

In some implementations, the method comprises administering a monoclonal antibody that inhibits cleavage of C4, where the isolated monoclonal antibody does not inhibit cleavage of C2. In some implementations, the method comprises administering a monoclonal antibody that inhibits cleavage of C2, where the isolated monoclonal antibody does not inhibit cleavage of C4. In some cases, the isolated monoclonal antibody is humanized. In some cases, the component of the classical complement pathway that is inhibited by the antibody is C1s. The present disclosure also provides methods of treating a complement-mediated disease or disorder, by administering to an individual in need thereof an isolated monoclonal antibody that inhibits cleavage of C4, or a pharmaceutical composition comprising the isolated monoclonal antibody, where the isolated monoclonal antibody does not inhibit cleavage of C2.

In some implementations, the method comprises administering a monoclonal antibody that inhibits cleavage of C2 or C4 by C1s, i.e., inhibits C1s-mediated proteolytic cleavage of C2 or C4. In some cases, the monoclonal antibody is humanized. In some cases, the antibody inhibits cleavage of C2 or C4 by C1s by inhibiting binding of C2 or C4 to C1s; for example, in some cases, the antibody inhibits C1s-mediated cleavage of C2 or C4 by inhibiting binding of C2 or C4 to a C2 or C4 binding site of C1s. Thus, in some cases, the antibody functions as a competitive inhibitor. The present disclosure also provides methods of treating a brain injury, preferably a traumatic brain injury, hypoxic brain injury, brain infection, or stroke, by administering to an individual in need thereof an isolated monoclonal antibody that inhibits cleavage of C2 or C4 by C1s, i.e., inhibits C1s-mediated proteolytic cleavage of C2 or C4.

In some implementations, the method comprises administering a monoclonal antibody that inhibits cleavage of C4 by C1s, where the antibody does not inhibit cleavage of complement component C2 by C1s; i.e., the antibody inhibits C1s-mediated cleavage of C4, but does not inhibit C1s-mediated cleavage of C2. In some cases, the monoclonal antibody is humanized. In some cases, the monoclonal antibody inhibits binding of C4 to C1s, but does not inhibit binding of C2 to C1s. In some implementations, the method comprises treating a complement-mediated disease or disorder, by administering to an individual in need thereof an isolated monoclonal antibody that inhibits cleavage of C4 by C1s, where the antibody does not inhibit cleavage of complement component C2 by C1s; i.e., the antibody inhibits C1s-mediated cleavage of C4, but does not inhibit C1s-mediated cleavage of C2. In some implementations of the method, the antibody is humanized.

In some implementations, the method comprises administering a humanized monoclonal antibody that specifically binds an epitope within a region encompassing domains IV and V of C1s. For example, the humanized monoclonal antibody specifically binds an epitope within amino acids 272-422 of the amino acid sequence depicted in FIG. 1 and set forth in SEQ ID NO:70. In some cases, the humanized monoclonal antibody specifically binds an epitope within amino acids 272-422 of the amino acid sequence depicted in FIG. 1 and set forth in SEQ ID NO:70, and inhibits binding of C4 to C1s. In some implementations, the method comprises treating a complement-mediated disease or disorder, by administering to an individual in need thereof a humanized monoclonal antibody that specifically binds an epitope within amino acids 272-422 of the amino acid sequence depicted in FIG. 1 and set forth in SEQ ID NO:70, and inhibits binding of C4 to C1s.

In some implementations, the method comprises administering a humanized monoclonal antibody that specifically binds a conformational epitope within a region encompassing domains IV and V of C1s. For example, the humanized monoclonal antibody that specifically binds a conformational epitope within amino acids 272-422 of the amino acid sequence depicted in FIG. 1 and set forth in SEQ ID NO:70. In some cases, the humanized monoclonal antibody specifically binds a conformational epitope within amino acids 272-422 of the amino acid sequence depicted in FIG. 1 and set forth in SEQ ID NO:70, and inhibits binding of C4 to C1s. In some implementations, the method comprises a brain injury, preferably a traumatic brain injury, hypoxic brain injury, brain infection, or stroke, the method comprising administering to an individual in need thereof a humanized monoclonal antibody that specifically binds a conformational epitope within amino acids 272-422 of the amino acid sequence depicted in FIG. 1 and set forth in SEQ ID NO:70, and inhibits binding of C4 to C1s.

In some implementations, the method comprises administering a monoclonal antibody that binds complement component C1s in a C1 complex. The C1 complex is composed of 6 molecules of C1q, 2 molecules of C1r, and 2 molecules of C1s. In some cases, the monoclonal antibody is humanized. Thus, in some cases, the humanized monoclonal antibody that binds complement component C1s in a C1 complex. In some cases, the antibody binds C1s present in a C1 complex with high avidity.

In some implementations, the anti-C1s antibody (*e.g.*, a subject antibody that specifically binds an epitope in a complement C1s protein) comprises: a) a light chain region comprising one, two, or three VL CDRs of an IPN003 antibody; and b) a heavy chain region comprising one, two, or three VH CDRs of an IPN003 antibody; where the VH and VL CDRs are as defined by Kabat (see, *e.g.*, Table 1; and Kabat 1991).

In other implementations, the anti-C1s antibody (*e.g.*, a subject antibody that specifically binds an epitope in a complement C1s protein) comprises: a) a light chain region comprising one, two, or three VL CDRs of an IPN003 antibody; and b) a heavy chain region comprising one, two, or three VH CDRs of an IPN003 antibody; where the VH and VL CDRs are as defined by Chothia (see, *e.g.*, Table 1, and Chothia 1987).

CDR amino acid sequences, and VL and VH amino acid sequences, of IPN003 antibody are provided in Table 2. Table 2 also provides the SEQ ID NOs assigned to each of the amino acid sequences.

In some implementations, the anti-C1s antibody (*e.g.*, a subject antibody that specifically binds an epitope in a complement C1s protein) comprises: a) a light chain region comprising one, two, or three CDRs selected from SEQ ID NO:51, SEQ ID NO:52, and SEQ ID NO:53; and b) a heavy chain region comprising one, two, or three CDRs selected from SEQ ID NO:54, SEQ ID NO:55, and SEQ ID NO:56. In some of these implementations, the anti-C1s antibody includes a humanized VH and/or VL framework region.

SEQ ID NO. 51:     SSVSSSYLHWYQ;
SEQ ID NO. 52:     STSNLASGVP;
SEQ ID NO. 53:     HQYYRLPPIT;
SEQ ID NO. 54:     GFTFSNYAMSWV;
SEQ ID NO. 55:     ISSGGSHTYY;
SEQ ID NO. 56:     ARLFTGYAMDY.

In some implementations, the anti-C1s antibody comprises a CDR having an amino acid sequence selected from SEQ ID NO:51, SEQ ID NO:52, SEQ ID NO:53, SEQ ID NO:54, SEQ ID NO:55, and SEQ ID NO:56.

In some implementations, the anti-C1s antibody comprises a light chain variable region comprising amino acid sequences SEQ ID NO:51, SEQ ID NO:52, and SEQ ID NO:53.

In some implementations, the anti-C1s antibody comprises a heavy chain variable region comprising amino acid sequences SEQ ID NO:54, SEQ ID NO:55, and SEQ ID NO:56.

In some implementations, the anti-C1s antibody comprises a CDR-L1 having amino acid sequence SEQ ID NO:51, a CDR-L2 having amino acid sequence SEQ ID NO:52, a CDR-L3 having amino acid sequence SEQ ID NO:53, a CDR-H1 having amino acid sequence SEQ ID NO:54, a CDR-H2 having amino acid sequence SEQ ID NO:55, and a CDR-H3 having amino acid sequence SEQ ID NO:56.

In some implementations, the anti-C1s antibody comprises a light chain variable region comprising an amino acid sequence that is 85%, 86%, 87%, 88%, 89%, 90%, 91%, 92%, 93%, 94%, 95%, 96%, 97%, 98% or 99% identical to the amino acid sequence set forth in SEQ ID NO:57.

SEQ ID NO. 57:

DIVMTQTTAIMSASLGERVTMTCTASSSVSSSYLHWYQQKPGSSPKLWIYSTSNLAS

GVPARFSGSGSGTFYSLTISSMEAEDDATYYCHQYYRLPPITFGAGTKLELK.

In some implementations, the anti-C1s antibody comprises a heavy chain variable region comprising an amino acid sequence that is 85%, 86%, 87%, 88%, 89%, 90%, 91%, 92%, 93%, 94%, 95%, 96%, 97%, 98% or 99% identical to the amino acid sequence set forth in SEQ ID NO. 58.

SEQ ID NO. 58:

QVKLEESGGALVKPGGSLKLSCAASGFTFSNYAMSWVRQIPEKRLEWVATISSGGSH
TYYLDSVKGRFTISRDNARDTLYLQMSSLRSEDTALYYCARLFTGYAMDYWGQGTS
VT.

In some implementations, the anti-C1s antibody comprises a light chain variable region comprising an amino acid sequence that is 90% identical to amino acid sequence SEQ ID NO:57.

In some implementations, the anti-C1s antibody comprises a heavy chain variable region comprising an amino acid sequence that is 90% identical to amino acid sequence SEQ ID NO:58.

In some implementations, the anti-C1s antibody comprises a light chain variable region comprising amino acid sequence SEQ ID NO:57.

In some implementations, the anti-C1s antibody comprises a heavy chain variable region comprising amino acid sequence SEQ ID NO:58.

In some implementations, the anti-C1s antibody comprises a light chain variable region comprising an amino acid sequence that is 90% identical to amino acid sequence SEQ ID NO:57 and a heavy chain variable region comprising an amino acid sequence that is 90% identical to amino acid sequence SEQ ID NO:58.

In some implementations, the anti-C1s antibody comprises a light chain variable region comprising amino acid sequence SEQ ID NO:57 and a heavy chain variable region comprising amino acid sequence SEQ ID NO:58.

In some implementations, the anti-C1s antibody specifically binds an epitope within the complement C1s protein, wherein the antibody competes for binding the epitope with an antibody that comprises light chain CDRs of an antibody light chain variable region comprising amino acid sequence SEQ ID NO:57 and heavy chain CDRs of an antibody heavy chain variable region comprising amino acid sequence SEQ ID NO:58.

In some implementations, the anti-C1s antibody comprises light chain CDRs of an antibody light chain variable region comprising amino acid sequence SEQ ID NO:57 and heavy chain CDRs of an antibody heavy chain variable region comprising amino acid sequence SEQ ID NO:58.

In some implementations, the anti-C1s antibody (e.g., a subject antibody that specifically binds an epitope in a complement C1s protein) comprises: a) a light chain region comprising one, two, or three CDRs selected from SEQ ID NO:62, SEQ ID NO:63, and SEQ ID NO:53; and b) a heavy chain region comprising one, two, or three CDRs selected from SEQ ID NO:64, SEQ ID NO:65, and SEQ ID NO:66.

SEQ ID NO.62: TASSSVSSSYLH;

SEQ ID NO. 63: STSNLAS;

SEQ ID NO.53: HQYYRLPPIT;

SEQ ID NO.64: NYAMS;

SEQ ID NO.65: TISSGGSHTYYLDSVKG;

SEQ ID NO 66: LFTGYAMDY

In some implementations, the anti-C1s antibody comprises a CDR having an amino acid sequence selected from SEQ ID NO:62, SEQ ID NO:63, SEQ ID NO:53, SEQ ID NO:64, SEQ ID NO:65, and SEQ ID NO:66.

In some implementations, the anti-C1s antibody comprises a light chain variable region comprising amino acid sequences SEQ ID NO:62, SEQ ID NO:63, and SEQ ID NO:53.

In some implementations, the anti-C1s antibody comprises a heavy chain variable region comprising amino acid sequences SEQ ID NO:64, SEQ ID NO:65, and SEQ ID NO:66.

In some implementations, the anti-C1s antibody comprises a CDR-L1 having amino acid sequence SEQ ID NO:62, a CDR-L2 having amino acid sequence SEQ ID NO:63, a CDR-L3 having amino acid sequence SEQ ID NO:53, a CDR-H1 having amino acid sequence SEQ ID NO:64, a CDR-H2 having amino acid sequence SEQ ID NO:65, and a CDR-H3 having amino acid sequence SEQ ID NO:66.

In some implementations, the anti-C1s antibody comprises a light chain variable region comprising an amino acid sequence that is 85%, 86%, 87%, 88%, 89%, 90%, 91%, 92%, 93%, 94%, 95%, 96%, 97%, 98% or 99% identical to the amino acid sequence set forth in SEQ ID NO:67.

SEQ ID NO. 67:

QIVLTQSPAIMSASLGERVTMTCTASSSVSSSYLHWYQQKPGSSPKLWIYSTSNLASG VPARFSGSGSGTFYSLTISSMEAEDDATYYCHQYYRLPPITFGAGTKLELK.

In some implementations, the anti-C1s antibody comprises a heavy chain variable region comprising an amino acid sequence that is 85%, 86%, 87%, 88%, 89%, 90%, 91%, 92%, 93%, 94%, 95%, 96%, 97%, 98% or 99% identical to the amino acid sequence set forth in SEQ ID NO:68.

SEQ ID NO. 68:

EVMLVESGGALVKPGGSLKLSCAASGFTFSNYAMSWVRQIPEKRLEWVATISSGGS HTYYLDSVKGRFTISRDNARDTLYLQMSSLRSEDTALYYCARLFTGYAMDYWGQG TSVTVSS.

In some implementations, the anti-C1s antibody comprises a light chain variable region comprising an amino acid sequence that is 90% identical to amino acid sequence SEQ ID NO:67.

In some implementations, the anti-C1s antibody comprises a heavy chain variable region comprising an amino acid sequence that is 90% identical to amino acid sequence SEQ ID NO:68.

In some implementations, the anti-C1s antibody comprises a light chain variable region comprising amino acid sequence SEQ ID NO:67.

In some implementations, the anti-C1s antibody comprises a heavy chain variable region comprising amino acid sequence SEQ ID NO:68.

In some implementations, the anti-C1s antibody comprises a light chain variable region comprising an amino acid sequence that is 90% identical to amino acid sequence SEQ ID NO:67 and a heavy chain variable region comprising an amino acid sequence that is 90% identical to amino acid sequence SEQ ID NO:68.

In some implementations, the anti-C1s antibody comprises a light chain variable region comprising an amino acid sequence that is 95% identical to amino acid sequence SEQ ID NO:67 and a heavy chain variable region comprising an amino acid sequence that is 95% identical to amino acid sequence SEQ ID NO:68.

In some implementations, the anti-C1s antibody comprises a light chain variable region comprising amino acid sequence SEQ ID NO:67 and a heavy chain variable region comprising amino acid sequence SEQ ID NO:68.

In some implementations, the anti-C1s antibody specifically binds an epitope within the complement C1s protein, wherein the antibody competes for binding the epitope with an antibody that comprises light chain CDRs of an antibody light chain variable region comprising amino acid sequence SEQ ID NO:67 and heavy chain CDRs of an antibody heavy chain variable region comprising amino acid sequence SEQ ID NO:68.

In some implementations, the anti-C1s antibody comprises light chain CDRs of an antibody light chain variable region comprising amino acid sequence SEQ ID NO:67 and heavy chain CDRs of an antibody heavy chain variable region comprising amino acid sequence SEQ ID NO:68.

In some implementations, the anti-C1s antibody comprises a light chain variable region comprising an amino acid sequence that is 85%, 86%, 87%, 88%, 89%, 90%, 91%, 92%, 93%, 94%, 95%, 96%, 97%, 98% or 99% identical to the amino acid sequence set forth in SEQ ID NO:67.

In some implementations, the anti-C1s antibody comprises a heavy chain variable region comprising an amino acid sequence that is 85%, 86%, 87%, 88%, 89%, 90%, 91%, 92%, 93%, 94%, 95%, 96%, 97%, 98% or 99% identical to the amino acid sequence set forth in SEQ ID NO:68.

An anti-C1s antibody can comprise a heavy chain variable region comprising an amino acid sequence that is 85%, 86%, 87%, 88%, 89%, 90%, 91%, 92%, 93%, 94%, 95%, 96%, 97%, 98%, 99%, or 100% identical to the amino acid sequence set forth in SEQ ID NO:79 and depicted in FIG. 2 (VH variant 1).

An anti-C1s antibody can comprise a heavy chain variable region comprising an amino acid sequence that is 85%, 86%, 87%, 88%, 89%, 90%, 91%, 92%, 93%, 94%, 95%, 96%, 97%, 98%, 99%, or 100% identical to the amino acid sequence set forth in SEQ ID NO:80 and depicted in FIG. 3 (VH variant 2).

An anti-C1s antibody can comprise a heavy chain variable region comprising an amino acid sequence that is 85%, 86%, 87%, 88%, 89%, 90%, 91%, 92%, 93%, 94%, 95%, 96%, 97%, 98%, 99%, or 100% identical to the amino acid sequence set forth in SEQ ID NO:81 and depicted in FIG. 4 (VH variant 3).

An anti-C1s antibody can comprise a heavy chain variable region comprising an amino acid sequence that is 85%, 86%, 87%, 88%, 89%, 90%, 91%, 92%, 93%, 94%, 95%, 96%, 97%, 98%, 99%, or 100% identical to the amino acid sequence set forth in SEQ ID NO:82 and depicted in FIG. 5 (VH variant 4).

An anti-C1s antibody can comprise a light chain variable region comprising an amino acid sequence that is 85%, 86%, 87%, 88%, 89%, 90%, 91%, 92%, 93%, 94%, 95%, 96%, 97%, 98%, 99%, or 100% identical to the amino acid sequence set forth in SEQ ID NO:83 and depicted in FIG. 6 (VK variant 1).

An anti-C1s antibody can comprise a light chain variable region comprising an amino acid sequence that is 85%, 86%, 87%, 88%, 89%, 90%, 91%, 92%, 93%, 94%, 95%, 96%, 97%, 98%, 99%, or 100% identical to the amino acid sequence set forth in SEQ ID NO:84 and depicted in FIG. 7 (VK variant 2).

An anti-C1s antibody can comprise a light chain variable region comprising an amino acid sequence that is 85%, 86%, 87%, 88%, 89%, 90%, 91%, 92%, 93%, 94%, 95%, 96%, 97%, 98%, 99%, or 100% identical to the amino acid sequence set forth in SEQ ID NO:85 and depicted in FIG. 8 (VK variant 3).

An anti-C1s antibody can comprise a heavy chain variable region comprising 1, 2, 3, 4, 5, 6, 7, 8, 9, 10, 11, or 12 of the framework (FR) amino acid substitutions, relative to the IPN003 parental antibody FR amino acid sequences, depicted in Table 3 (FIG. 9).

*Definitions*

As used herein the specification, "a" or "an" may mean one or more. As used herein in the claim(s), when used in conjunction with the word "comprising", the words "a" or "an" may mean one or more than one. For example, reference to an "antibody" is a reference from one to many antibodies. As used herein "another" may mean at least a second or more.

As used herein, administration *"conjointly"* with another compound or composition includes simultaneous administration and/or administration at different times. Administration in conjunction also encompasses administration as a co-formulation or administration as separate compositions, including at different dosing frequencies or intervals, and using the same route of administration or different routes of administration.

The term *"immunoglobulin"* (Ig) is used interchangeably with *"antibody"* herein. The term "antibody" herein is used in the broadest sense and specifically covers monoclonal antibodies, polyclonal antibodies, multispecific antibodies (*e.g.*, bispecific antibodies) formed from at least two intact antibodies, and antibody fragments so long as they exhibit the desired biological activity.

The basic 4-chain antibody unit is a heterotetrameric glycoprotein composed of two identical light (L) chains and two identical heavy (H) chains. The pairing of a $V_H$ and $V_L$ together forms a single antigen-binding site. For the structure and properties of the different classes of antibodies, *see, e.g.,* Basic and Clinical Immunology, 8th Ed., Daniel P. Stites, Abba I. Terr and Tristram G. Parslow (eds.), Appleton & Lange, Norwalk, CT, 1994, page 71 and Chapter 6.

The L chain from any vertebrate species can be assigned to one of two clearly distinct types, called kappa ("κ") and lambda ("λ"), based on the amino acid sequences of their constant domains. Depending on the amino acid sequence of the constant domain of their heavy chains (CH), immunoglobulins can be assigned to different classes or isotypes. There are five classes of immunoglobulins: IgA, IgD, IgE, IgG, and IgM, having heavy chains designated alpha ("α"), delta ("δ"), epsilon ("ε"), gamma ("γ") and mu ("μ"), respectively. The γ and α classes are further divided into subclasses (isotypes) on the basis of relatively minor differences in the CH sequence and function, *e.g.*, humans express the following subclasses: IgG1, IgG2, IgG3, IgG4, IgA1, and IgA2. The subunit structures and three dimensional configurations of different classes of immunoglobulins are well known and described generally in, for example, Abbas et al., Cellular and Molecular Immunology, 4th ed. (W.B. Saunders Co., 2000).

*"Native antibodies"* are usually heterotetrameric glycoproteins of about 150,000 daltons, composed of two identical light (L) chains and two identical heavy (H) chains. Each light chain is linked to a heavy chain by one covalent disulfide bond, while the number of disulfide linkages varies among the heavy chains of different immunoglobulin isotypes. Each heavy and light chain also has regularly spaced intrachain disulfide bridges. Each heavy chain has at one end a variable domain ($V_H$) followed by a number of constant domains. Each light chain has a variable domain at one end ($V_L$) and a constant domain at its other end; the constant domain of the light chain is aligned with the first constant domain of the heavy chain, and the light chain variable domain is aligned with the variable domain of the heavy chain. Particular amino acid residues are believed to form an interface between the light chain and heavy chain variable domains.

An *"isolated"* molecule or cell is a molecule or a cell that is identified and separated from at least one contaminant molecule or cell with which it is ordinarily associated in the environment in which it was produced. Preferably, the isolated molecule or cell is free of association with all components associated with the production environment. The isolated molecule or cell is in a form other than in the form or setting in which it is found in nature. Isolated molecules therefore are distinguished from molecules existing naturally in cells; isolated cells are distinguished from cells existing naturally in tissues, organs, or individuals. In some implementations, the isolated molecule is an anti-C1s, anti-C1q, or anti-C1r antibody of the present disclosure. In other implementations, the isolated cell is a host cell or hybridoma cell producing an anti-C1s, anti-C1q, or anti-C1r antibody of the present disclosure.

An *"isolated"* antibody is one that has been identified, separated and/or recovered from a component of its production environment (*e.g.*, naturally or recombinantly). Preferably, the isolated polypeptide is free of association with all other contaminant components from its production environment. Contaminant components from its production environment, such as those resulting from recombinant transfected cells, are materials that would typically interfere with research, diagnostic or therapeutic uses for the antibody, and may include enzymes, hormones, and other proteinaceous or non-proteinaceous solutes. In certain preferred implementations, the polypeptide will be purified: (1) to greater than 95% by weight of antibody as determined by, for example, the Lowry method, and in some implementations, to greater than 99% by weight; (2) to a degree sufficient to obtain at least 15 residues of N-terminal or internal amino acid sequence by use of a spinning cup sequenator, or (3) to homogeneity by SDS-PAGE under non-reducing or reducing conditions using Coomassie blue or, preferably, silver stain. An isolated antibody includes the antibody *in situ* within recombinant T-cells since at least one component of the antibody's natural environment will not be present. Ordinarily, however, an isolated polypeptide or antibody will be prepared by a process including at least one purification step.

The *"variable region"* or *"variable domain"* of an antibody refers to the amino-terminal domains of the heavy or light chain of the antibody. The variable domains of the heavy chain and light chain may be referred to as "$V_H$" and "$V_L$", respectively. These domains are generally the most variable parts of the antibody (relative to other antibodies of the same class) and contain the antigen binding sites.

The term *"variable"* refers to the fact that certain segments of the variable domains differ extensively in sequence among antibodies. The V domain mediates antigen binding and defines the specificity of a particular antibody for its particular antigen. However, the variability is not evenly distributed across the entire span of the variable domains. Instead, it is concentrated in three segments called hypervariable regions (HVRs) both in the light-chain and the heavy chain variable domains. The more highly conserved portions of variable domains are called the framework regions (FR). The variable domains of native heavy and light chains each comprise four FR regions, largely adopting a beta-sheet configuration, connected by three HVRs, which form loops connecting, and in some cases forming part of, the beta-sheet structure. The HVRs in each chain are held together in close proximity by the FR regions and, with the HVRs from the other chain, contribute to the formation of the antigen binding site of antibodies (*see* Kabat et al., Sequences of Immunological Interest, Fifth Edition, National Institute of Health, Bethesda, MD (1991)). The constant domains are not involved directly in the binding of antibody to an antigen, but exhibit various effector functions, such as participation of the antibody in antibody-dependent-cellular toxicity.

As used herein, the term "CDR" or "complementarity determining region" is intended to mean the non-contiguous antigen binding sites found within the variable region of both heavy and light chain polypeptides. CDRs have been described by Kabat et al., J. Biol. Chem. 252:6609-6616 (1977); Kabat et al., U.S. Dept. of Health and Human Services, "Sequences of proteins of immunological interest" (1991) (also referred to herein as Kabat 1991); by Chothia et al., J. Mol. Biol. 196:901-917 (1987) (also referred to herein as Chothia 1987); and MacCallum et al., J. Mol. Biol. 262:732-745 (1996), where the definitions include overlapping or subsets of amino acid residues when compared against each other. Nevertheless, application of either definition to refer to a CDR of an antibody or grafted antibodies or variants thereof is intended to be within the scope of the term as defined and used herein. The amino acid residues, which encompass the CDRs, as defined by each of the above cited references are set forth below in Table 1 as a comparison. The CDRs listed in Table 2 were defined in accordance with Kabat 1991.

**Table 1**

| CDR Definitions | | | |
|---|---|---|---|
| | Kabat[1] | Chothia[2] | MacCallum[3] |
| $V_H$ CDR-1 | 31-35 | 26-32 | 30-35 |
| $V_H$ CDR-2 | 50-65 | 53-55 | 47-58 |
| $V_H$ CDR-3 | 95-102 | 96-101 | 93-101 |
| $V_L$ CDR-1 | 24-34 | 26-32 | 30-36 |
| $V_L$ CDR-2 | 50-56 | 50-52 | 46-55 |
| $V_L$ CDR-3 | 89-97 | 91-96 | 89-96 |
| [1]Residue numbering follows the nomenclature of Kabat et al., supra<br>[2]Residue numbering follows the nomenclature of Chothia et al., supra<br>[3]Residue numbering follows the nomenclature of MacCallum et al., supra | | | |

Table 2

| Antibod y | CDR-1 | CDR-2 | CDR-3 | V region |
|---|---|---|---|---|
| IPN-M34 VL | SEQ ID NO. 51 SSVSSSYLHWYQ | SEQ ID NO. 52 STSNLASGVP | SEQ ID NO. 53 HQYYRLPPIT | SEQ ID NO. 57<br>DIVMTQTTAIMSASLGERVTMTCTASSSVSSS<br>YLHWYQQKPGSSPKLWIYSTSNLASGVPARFS<br>GSGSGTFYSLTISSMEAEDDATYYCHQYYRLP<br>PITFGAGTKLELK |
| IPN-M34 VH | SEQ ID NO. 54 GFTFSNYAMSW V | SEQ ID NO. 55 ISSGGSHTYY | SEQ ID NO. 56 ARLFTGYAM-DY | SEQ ID NO. 58<br>QVKLEESGGALVKPGGSLKLSCAASGFTFSNY<br>AMSWVRQIPEKRLEWVATISSGGSHTYYLDS<br>VKGRFTISRDNARDTLYLQMSSLRSEDTALYY<br>CARLFTGYAMDYWGQGTSVT |

As used herein, the terms "CDR-L1", "CDR-L2", and "CDR-L3" refer, respectively, to the first, second, and third CDRs in a light chain variable region. As used herein, the terms "CDR-H1", "CDR-H2", and "CDR-H3" refer, respectively, to the first, second, and third CDRs in a heavy chain variable region. As used herein, the terms "CDR-1", "CDR-2", and "CDR-3" refer, respectively, to the first, second and third CDRs of either chain's variable region.

The term *"monoclonal antibody"* as used herein refers to an antibody obtained from a population of substantially homogeneous antibodies, i.e., the individual antibodies of the population are identical except for possible naturally occurring mutations and/or post-translation modifications (*e.g.,* isomerizations, amidations) that may be present in minor amounts. Monoclonal antibodies are highly specific, being directed against a single antigenic site. In contrast to polyclonal antibody preparations which typically include different antibodies directed against different determinants (epitopes), each monoclonal antibody is directed against a single determinant on the antigen. In addition to their specificity, monoclonal antibodies are advantageous since they are typically synthesized by hybridoma culture, uncontaminated by other immunoglobulins. The modifier "monoclonal" indicates the character of the antibody as being obtained as a substantially homogeneous population of antibodies, and is not to be construed as requiring production of the antibody by any particular method. For example, the monoclonal antibodies to be used in accordance with the present disclosure may be made by a variety of techniques, including, for example, the hybridoma method *(e.g.,* Kohler and Milstein., Nature, 256:495-97 (1975); Hongo et al., Hybridoma, 14 (3):253-260 (1995), Harlow et al., Antibodies: A Laboratory Manual, (Cold Spring Harbor Laboratory Press, 2d ed. 1988); Hammerling et al., in: Monoclonal Antibodies and T-Cell Hybridomas 563-681 (Elsevier, N.Y., 1981)), recombinant DNA methods (see, *e.g.,* U.S. Patent No. 4,816,567), phage-display technologies (*see, e.g.,* Clackson et al., Nature, 352:624-628 (1991); Marks et al., J. Mol. Biol. 222:581-597 (1992); Sidhu et al., J. Mol. Biol. 338(2): 299-310 (2004); Lee et al., J. Mol. Biol. 340(5): 1073-1093 (2004); Fellouse, Proc. Nat'l Acad. Sci. USA 101(34):12467-472 (2004); and Lee et al., J. Immunol. Methods 284(1-2):119-132 (2004), and technologies for producing human or human-like antibodies in animals that have parts or all of the human immunoglobulin loci or genes encoding human immunoglobulin sequences (see, *e.g.,* WO 1998/24893; WO 1996/34096; WO 1996/33735; WO 1991/10741; Jakobovits et al., Proc. Nat'l Acad. Sci. USA 90:2551 (1993); Jakobovits et al., Nature 362:255-258 (1993); Bruggemann et al., Year in Immunol. 7:33 (1993); U.S. Patent Nos. 5,545,807; 5,545,806; 5,569,825; 5,625,126; 5,633,425; and 5,661,016; Marks et al., Bio/Technology 10:779-783 (1992); Lonberg et al., Nature 368:856-859 (1994); Morrison, Nature 368:812-813 (1994); Fishwild et al., Nature Biotechnol. 14:845-851 (1996); Neuberger, Nature Biotechnol. 14:826 (1996); and Lonberg and Huszar, Intern. Rev. Immunol. 13:65-93 (1995).

The terms *"full-length antibody,"* *"intact antibody"* and *"whole antibody"* are used interchangeably to refer to an antibody in its substantially intact form, as opposed to an antibody fragment. Specifically, whole antibodies include those with heavy and light chains including an Fc region. The constant domains may be native sequence constant domains (*e.g.*, human native sequence constant domains) or amino acid sequence variants thereof. In some cases, the intact antibody may have one or more effector functions.

An *"antibody fragment"* comprises a portion of an intact antibody, preferably the antigen binding and/or the variable region of the intact antibody. Examples of antibody fragments include Fab, Fab', F(ab')$_2$ and Fv fragments; diabodies; linear antibodies (*see* U.S. Patent 5,641,870, Example 2; Zapata et al., Protein Eng. 8(10): 1057-1062 (1995)); single-chain antibody molecules and multispecific antibodies formed from antibody fragments.

Papain digestion of antibodies produces two identical antigen-binding fragments, called *"Fab"* fragments, and a residual *"Fc"* fragment, a designation reflecting the ability to crystallize readily. The Fab fragment consists of an entire L chain along with the variable region domain of the H chain ($V_H$), and the first constant domain of one heavy chain ($C_H1$). Each Fab fragment is monovalent with respect to antigen binding, *i.e.,* it has a single antigen-binding site. Pepsin treatment of an antibody yields a single large F(ab')$_2$ fragment which roughly corresponds to two disulfide linked Fab fragments having different antigen-binding activity and is still capable of cross-linking antigen. Fab' fragments differ from Fab fragments by having a few additional residues at the carboxy terminus of the $C_H1$ domain including one or more cysteines from the antibody hinge region. Fab'-SH is the designation herein for Fab' in which the cysteine residue(s) of the constant domains bear a free thiol group. F(ab')$_2$ antibody fragments originally were produced as pairs of Fab' fragments with hinge cysteines between them. Other chemical couplings of antibody fragments are also known.

The Fc fragment comprises the carboxy-terminal portions of both H chains held together by disulfides. The effector functions of antibodies are determined by sequences in the Fc region, the region which is also recognized by Fc receptors (FcR) found on certain types of cells.

The term *"Fc region"* herein is used to define a C-terminal region of an immunoglobulin heavy chain, including native-sequence Fc regions and variant Fc regions. Although the boundaries of the Fc region of an immunoglobulin heavy chain might vary, the human IgG heavy-chain Fc region is usually defined to stretch from an amino acid residue at position Cys226, or from Pro230, to the carboxyl-terminus thereof. The C-terminal lysine (residue 447 according to the EU numbering system) of the Fc region may be removed, for example, during production or purification of the antibody, or by recombinantly engineering the nucleic acid encoding a heavy chain of the antibody. Accordingly, a composition of intact antibodies may comprise antibody populations with all K447 residues removed, antibody populations with no K447 residues removed, and antibody populations having a mixture of antibodies with and without the K447 residue. Suitable native-sequence Fc regions for use in the antibodies of the disclosure include human IgG1, IgG2, IgG3 and IgG4.

A *"native sequence Fc region"* comprises an amino acid sequence identical to the amino acid sequence of an Fc region found in nature. Native sequence human Fc regions include a native sequence human IgG1 Fc region (non-A and A allotypes); native sequence human IgG2 Fc region; native sequence human IgG3 Fc region; and native sequence human IgG4 Fc region as well as naturally occurring variants thereof.

A *"variant Fc region"* comprises an amino acid sequence which differs from that of a native sequence Fc region by virtue of at least one amino acid modification, preferably one or more amino acid substitution(s). Preferably, the variant Fc region has at least one amino acid substitution compared to a native sequence Fc region or to the Fc region of a parent polypeptide, *e.g.*, from about one to about ten amino acid substitutions, and preferably from about one to about five amino acid substitutions in a native sequence Fc region or in the Fc region of the parent polypeptide. The variant Fc region herein will preferably possess at least about 80% homology with a native sequence Fc region and/or with an Fc region of a parent polypeptide, and most preferably at least about 90% homology therewith, more preferably at least about 95% homology therewith.

*"Fc receptor"* or *"FcR"* describes a receptor that binds to the Fc region of an antibody. The preferred FcR is a native sequence human FcR. Moreover, a preferred FcR is one which binds an IgG antibody (a gamma receptor) and includes receptors of the FcγRI, FcγRII, and FcγRIII subclasses, including allelic variants and alternatively spliced forms of these receptors, FcγRII receptors include FcγRIIA (an "activating receptor") and FcγRIIB (an "inhibiting receptor"), which have similar amino acid sequences that differ primarily in the cytoplasmic domains thereof. Activating receptor FcγRIIA contains an immunoreceptor tyrosine-based activation motif ("ITAM") in its cytoplasmic domain. Inhibiting receptor FcγRIIB contains an immunoreceptor tyrosine-based inhibition motif ("ITIM") in its cytoplasmic domain. (*See, e.g.,* M. Daëron, Annu. Rev. Immunol. 15:203-234 (1997)). FcRs are reviewed in Ravetch and Kinet, Annu. Rev. Immunol. 9:457-92 (1991); Capel et al., Immunomethods 4:25-34 (1994); and de Haas et al., J. Lab. Clin. Med. 126: 330-41 (1995). Other FcRs, including those to be identified in the future, are encompassed by the term "FcR" herein. FcRs can also increase the serum half-life of antibodies.

Binding to FcRn *in vivo* and serum half-life of human FcRn high-affinity binding polypeptides can be assayed, *e.g.*, in transgenic mice or transfected human cell lines expressing human FcRn, or in primates to which the polypeptides having a variant Fc region are administered. WO 2004/42072 (Presta) describes antibody variants with improved or diminished binding to FcRs. *See also, e.g.,* Shields et al., J. Biol. Chem. 9(2):6591-6604 (2001).

*"Fv"* is the minimum antibody fragment, which contains a complete antigen-recognition and -binding site. This fragment consists of a dimer of one heavy- and one light-chain variable region domain in tight, non-covalent association. From the folding of these two domains emanate six hypervariable loops (3 loops each from the H and L chain) that contribute the amino acid residues for antigen binding and confer antigen binding specificity to the antibody. However, even a single variable domain (or half of an Fv comprising only three HVRs specific for an antigen) has the ability to recognize and bind antigen, although at a lower affinity than the entire binding site.

*"Single-chain Fv"* also abbreviated as *"sFv"* or *"scFv"* are antibody fragments that comprise the VH and VL antibody domains connected into a single polypeptide chain. Preferably, the sFv polypeptide further comprises a polypeptide linker between the $V_H$ and $V_L$ domains which enables the sFv to form the desired structure for antigen binding. For a review of the sFv, *see* Plückthun in The Pharmacology of Monoclonal Antibodies, vol. 113, Rosenburg and Moore eds., Springer-Verlag, New York, pp. 269-315 (1994).

*"Functional fragments"* of antibodies comprise a portion of an intact antibody, generally including the antigen binding or variable region of the intact antibody or the F region of an antibody which retains or has modified FcR binding capability. Examples of antibody fragments include linear antibody, single-chain antibody molecules and multispecific antibodies formed from antibody fragments.

The term *"diabodies"* refers to small antibody fragments prepared by constructing sFv fragments (see preceding paragraph) with short linkers (about 5-10) residues) between the $V_H$ and $V_L$ domains such that inter-chain but not intra-chain pairing of the V domains is achieved, thereby resulting in a bivalent fragment, *i.e.,* a fragment having two antigen-binding sites. Bispecific diabodies are heterodimers of two "crossover" sFv fragments in which the $V_H$ and $V_L$ domains of the two antibodies are present on different polypeptide chains. Diabodies are described in greater detail in, for example, EP 404,097; WO 1993/011161; WO/2009/121948; WO/2014/191493; Hollinger et al., Proc. Nat'l Acad. Sci. USA 90:6444-48 (1993).

As used herein, a "chimeric antibody" refers to an antibody (immunoglobulin) in which a portion of the heavy and/or light chain is identical with or homologous to corresponding sequences in antibodies derived from a particular species or belonging to a particular antibody class or subclass, while the remainder of the chain(s) is(are) identical with or homologous to corresponding sequences in antibodies derived from another species or belonging to another antibody class or subclass, as well as fragments of such antibodies, so long as they exhibit the desired biological activity (U.S. Patent No. 4,816,567; Morrison et al., Proc. Nat'l Acad. Sci. USA, 81:6851-55 (1984)). Chimeric antibodies of interest herein include PRIMATIZED® antibodies wherein the antigen-binding region of the antibody is derived from an antibody produced by, *e.g.*, immunizing macaque monkeys with an antigen of interest. As used herein, "humanized antibody" is a subset of "chimeric antibodies."

*"Humanized'* forms of non-human (*e.g.,* murine) antibodies are chimeric antibodies that contain minimal sequence derived from non-human immunoglobulin. In some implementations, a humanized antibody is a human immunoglobulin (recipient antibody) in which residues from an HVR of the recipient are replaced by residues from an HVR of a non-human species (donor antibody) such as mouse, rat, rabbit or non-human primate having the desired specificity, affinity, and/or capacity. In some instances, FR residues of the human immunoglobulin are replaced by corresponding non-human residues. Furthermore, humanized antibodies may comprise residues that are not found in the recipient antibody or in the donor antibody. These modifications may be made to further refine antibody performance, such as binding affinity. In general, a humanized antibody will comprise substantially all of at least one, and typically two, variable domains, in which all or substantially all of the hypervariable loops correspond to those of a non-human immunoglobulin sequence, and all or substantially all of the FR regions are those of a human immunoglobulin sequence, although the FR regions may include one or more individual FR residue substitutions that improve antibody performance, such as binding affinity, isomerization, immunogenicity, and the like. The number of these amino acid substitutions in the FR is typically no more than 6 in the H chain, and in the L chain, no more than 3. The humanized antibody optionally will also comprise at least a portion of an immunoglobulin constant region (Fc), typically that of a human immunoglobulin. For further details, *see, e.g.,* Jones et al., Nature 321:522-525 (1986); Riechmann et al., Nature 332:323-329 (1988); and Presta, Curr. Op. Struct. Biol. 2:593-596 (1992). *See also,* for example, Vaswani and Hamilton, Ann. Allergy, Asthma & Immunol. 1:105-115 (1998); Harris, Biochem. Soc. Transactions 23:1035-1038 (1995); Hurle and Gross, Curr. Op. Biotech. 5:428-433 (1994); and U.S. Patent Nos. 6,982,321 and 7,087,409.

A *"human antibody"* is one that possesses an amino-acid sequence corresponding to that of an antibody produced by a human and/or has been made using any of the techniques for making human antibodies as disclosed herein. This definition of a human antibody specifically excludes a humanized antibody comprising non-human antigen-binding residues. Human antibodies can be produced using various techniques known in the art, including phage-display libraries. Hoogenboom and Winter, J. Mol. Biol., 227:381 (1991); Marks et al., J. Mol. Biol., 222:581 (1991). Also available for the preparation of human monoclonal antibodies are methods described in Cole et al., Monoclonal Antibodies and Cancer Therapy, Alan R. Liss, p. 77 (1985); Boerner et al., J. Immunol., 147(1):86-95 (1991). *See also* van Dijk and van de Winkel, Curr. Opin. Pharmacol. 5:368-74 (2001). Human antibodies can be prepared by administering the antigen to a transgenic animal that has been modified to produce such antibodies in response to antigenic challenge, but whose endogenous loci have been disabled, *e.g.,* immunized xenomice (*see, e.g.,* U.S. Patent Nos. 6,075,181 and 6,150,584 regarding XENOMOUSE™ technology). *See also,* for example, Li et al., Proc. Nat'l Acad. Sci. USA, 103:3557-3562 (2006) regarding human antibodies generated via a human B-cell hybridoma technology.

The term *"hypervariable region," "HVR,"* or "HV," when used herein refers to the regions of an antibody-variable domain that are hypervariable in sequence and/or form structurally defined loops. Generally, antibodies comprise six HVRs; three in the VH (H1, H2, H3), and three in the VL (L1, L2, L3). In native antibodies, H3 and L3 display the most diversity of the six HVRs, and H3 in particular is believed to play a unique role in conferring fine specificity to antibodies. *See, e.g.,* Xu et al., Immunity 13:37-45 (2000); Johnson and Wu in Methods in Molecular Biology 248:1-25 (Lo, ed., Human Press, Totowa, NJ, 2003)). Indeed, naturally occurring camelid antibodies consisting of a heavy chain only are functional and stable in the absence of light chain. See, *e.g.,* Hamers-Casterman et al., Nature 363:446-448 (1993) and Sheriff et al., Nature Struct. Biol. 3:733-736 (1996).

A number of HVR delineations are in use and are encompassed herein. The HVRs that are Kabat complementarity-determining regions (CDRs) are based on sequence variability and are the most commonly used (Kabat et al., *supra*). Chothia refers instead to the location of the structural loops (Chothia and Lesk J. Mol. Biol. 196:901-917 (1987)). The AbM HVRs represent a compromise between the Kabat CDRs and Chothia structural loops, and are used by Oxford Molecular's AbM antibody-modeling software. The "contact" HVRs are based on an analysis of the available complex crystal structures. The residues from each of these HVRs are noted below.

| Loop | Kabat | AbM | Chothia | Contact |
|---|---|---|---|---|
| L1 | L24-L34 | L24-L34 | L26-L32 | L30-L36 |
| L2 | L50-L56 | L50-L56 | L50-L52 | L46-L55 |
| L3 | L89-L97 | L89-L97 | L91-L96 | L89-L96 |
| H1 | H31-H35B | H26-H35B | H26-H32 | H30-H35B (Kabat numbering) |
| H1 | H31-H35 | H26-H35 | H26-H32 | H30-H35 (Chothia numbering) |
| H2 | H50-H65 | H50-H58 | H53-H55 | H47-H58 |
| H3 | H95-H102 | H95-H102 | H96-H101 | H93-H101 |

HVRs may comprise "extended HVRs" as follows: 24-36 or 24-34 (L1), 46-56 or 50-56 (L2), and 89-97 or 89-96 (L3) in the VL, and 26-35 (H1), 50-65 or 49-65 (a preferred implementation) (H2), and 93-102, 94-102, or 95-102 (H3) in the VH. The variable-domain residues are numbered according to Kabat et al., *supra,* for each of these extended-HVR definitions.

*"Framework"* or "*FR*" residues are those variable-domain residues other than the HVR residues as herein defined.

The phrase *"variable-domain residue-numbering as in Kabat"* or *"amino-acid-position numbering as in Kabat,"* and variations thereof, refers to the numbering system used for heavy-chain variable domains or light-chain variable domains of the compilation of antibodies in Kabat et al., *supra.* Using this numbering system, the actual linear amino acid sequence may contain fewer or additional amino acids corresponding to a shortening of, or insertion into, a FR or HVR of the variable domain. For example, a heavy-chain variable domain may include a single amino acid insert (residue 52a according to Kabat) after residue 52 of H2 and inserted residues (*e.g.,* residues 82a, 82b, and 82c, *etc.* according to Kabat) after heavy-chain FR residue 82. The Kabat numbering of residues may be determined for a given antibody by alignment at regions of homology of the sequence of the antibody with a "standard" Kabat numbered sequence.

The Kabat numbering system is generally used when referring to a residue in the variable domain (approximately residues 1-107 of the light chain and residues 1-113 of the heavy chain) (*e.g.*, Kabat et al., Sequences of Immunological Interest. 5th Ed. Public Health Service, National Institutes of Health, Bethesda, Md. (1991)). The "EU numbering system" or "EU index" is generally used when referring to a residue in an immunoglobulin heavy chain constant region (*e.g.,* the EU index reported in Kabat *et al., supra*). The "EU index as in Kabat" refers to the residue numbering of the human IgG1 EU antibody. Unless stated otherwise herein, references to residue numbers in the variable domain of antibodies means residue numbering by the Kabat numbering system. Unless stated otherwise herein, references to residue numbers in the constant domain of antibodies means residue numbering by the EU numbering system (*e.g., see* United States Patent Publication No. 2010-280227).

An *"acceptor human framework"* as used herein is a framework comprising the amino acid sequence of a VL or VH framework derived from a human immunoglobulin framework or a human consensus framework. An acceptor human framework "derived from" a human immunoglobulin framework or a human consensus framework may comprise the same amino acid sequence thereof, or it may contain pre-existing amino acid sequence changes. In some implementations, the number of pre-existing amino acid changes are 10 or fewer, 9 or fewer, 8 or fewer, 7 or fewer, 6 or fewer, 5 or fewer, 4 or fewer, 3 or fewer, or 2 or fewer. Where pre-existing amino acid changes are present in a VH, preferable those changes occur at only three, two, or one of positions 71H, 73H and 78H; for instance, the amino acid residues at those positions may by 71A, 73T and/or 78A. In some implementations, the VL acceptor human framework is identical in sequence to the VL human immunoglobulin framework sequence or human consensus framework sequence.

A *"human consensus framework"* is a framework that represents the most commonly occurring amino acid residues in a selection of human immunoglobulin VL or VH framework sequences. Generally, the selection of human immunoglobulin VL or VH sequences is from a subgroup of variable domain sequences. Generally, the subgroup of sequences is a subgroup as in Kabat et al., Sequences of Proteins of Immunological Interest, 5th Ed. Public Health Service, National Institutes of Health, Bethesda, MD (1991). Examples include for the VL, the subgroup may be subgroup kappa I, kappa II, kappa III or kappa IV as in Kabat *et al., supra.* Additionally, for the VH, the subgroup may be subgroup I, subgroup II, or subgroup III as in Kabat *et al., supra.*

An *"amino-acid modification"* at a specified position refers to the substitution or deletion of the specified residue, or the insertion of at least one amino acid residue adjacent the specified residue. Insertion "adjacent" to a specified residue means insertion within one to two residues thereof. The insertion may be N-terminal or C-terminal to the specified residue. The preferred amino acid modification herein is a substitution.

An *"affinity-matured"* antibody is one with one or more alterations in one or more HVRs thereof that result in an improvement in the affinity of the antibody for antigen, compared to a parent antibody that does not possess those alteration(s). In some implementations, an affinity-matured antibody has nanomolar or even picomolar affinities for the target antigen. Affinity-matured antibodies are produced by procedures known in the art. For example, Marks et al., Bio/Technology 10:779-783 (1992) describes affinity maturation by VH- and VL-domain shuffling. Random mutagenesis of HVR and/or framework residues is described by, for example: Barbas et al. Proc Nat. Acad. Sci. USA 91:3809-3813 (1994); Schier et al. Gene 169:147-155 (1995); Yelton et al. J. Immunol. 155:1994-2004 (1995); Jackson et al., J. Immunol. 154(7):3310-9 (1995); and Hawkins et al, J. Mol. Biol. 226:889-896 (1992).

As use herein, the term *"specifically recognizes"* or *"specifically binds"* refers to measurable and reproducible interactions such as attraction or binding between a target and an antibody that is determinative of the presence of the target in the presence of a heterogeneous population of molecules including biological molecules. For example, an antibody that specifically or preferentially binds to a target or an epitope is an antibody that binds this target or epitope with greater affinity, avidity, more readily, and/or with greater duration than it binds to other targets or other epitopes of the target. It is also understood that, for example, an antibody (or a moiety) that specifically or preferentially binds to a first target may or may not specifically or preferentially bind to a second target. As such, *"specific binding"* or *"preferential binding"* does not necessarily require (although it can include) exclusive binding. An antibody that specifically binds to a target may have an association constant of at least about $10^3$ M$^{-1}$ or $10^4$ M$^{-1}$, sometimes about $10^5$ M$^{-1}$ or $10^6$ M$^{-1}$, in other instances about $10^6$ M$^{-1}$ or $10^7$ M$^{-1}$, about $10^8$ M$^{-1}$ to $10^9$M$^{-1}$, or about $10^{10}$ M$^{-1}$ to $10^{11}$ M$^{-1}$ or higher. A variety of immunoassay formats can be used to select antibodies specifically immunoreactive with a particular protein. For example, solid-phase ELISA immunoassays are routinely used to select monoclonal antibodies specifically immunoreactive with a protein. *See, e.g.,* Harlow and Lane (1988) Antibodies, A Laboratory Manual, Cold Spring Harbor Publications, New York, for a description of immunoassay formats and conditions that can be used to determine specific immunoreactivity.

*"Identity",* as used herein, indicates that at any particular position in the aligned sequences, the amino acid residue is identical between the sequences. "Similarity", as used herein, indicates that, at any particular position in the aligned sequences, the amino acid residue is of a similar type between the sequences. For example, leucine may be substituted for isoleucine or valine. Other amino acids which can often be substituted for one another include but are not limited to:

-   phenylalanine, tyrosine and tryptophan (amino acids having aromatic side chains);

-   lysine, arginine and histidine (amino acids having basic side chains);

-   aspartate and glutamate (amino acids having acidic side chains);

-   asparagine and glutamine (amino acids having amide side chains); and

-   cysteine and methionine (amino acids having sulphur-containing side chains).

Degrees of identity and similarity can be readily calculated. (*See e.g.,* Computational Molecular Biology, Lesk, A.M., ed., Oxford University Press, New York, 1988; Biocomputing. Informatics and Genome Projects, Smith, D.W., ed., Academic Press, New York, 1993; Computer Analysis of Sequence Data, Part 1, Griffin, A.M., and Griffin, H.G., eds., Humana Press, New Jersey, 1994; Sequence Analysis in Molecular Biology, von Heinje, G., Academic Press, 1987; and Sequence Analysis Primer, Gribskov, M. and Devereux, J., eds., M Stockton Press, New York, 1991)

As used herein, an "*interaction*" between a complement protein and a second protein encompasses, without limitation, protein-protein interaction, a physical interaction, a chemical interaction, binding, covalent binding, and ionic binding. As used herein, an antibody "inhibits interaction" between two proteins when the antibody disrupts, reduces, or completely eliminates an interaction between the two proteins. An antibody of the present disclosure, or fragment thereof, "inhibits interaction" between two proteins when the antibody or fragment thereof binds to one of the two proteins.

A *"blocking"* antibody, an *"antagonist"* antibody, an *"inhibitory"* antibody, or a *"neutralizing"* antibody is an antibody that inhibits or reduces one or more biological activities of the antigen it binds, such as interactions with one or more proteins. In some implementations, blocking antibodies, antagonist antibodies, inhibitory antibodies, or *"neutralizing"* antibodies substantially or completely inhibit one or more biological activities or interactions of the antigen.

Antibody *"effector functions"* refer to those biological activities attributable to the Fc region (a native sequence Fc region or amino acid sequence variant Fc region) of an antibody, and vary with the antibody isotype.

As used herein, the term "affinity" refers to the equilibrium constant for the reversible binding of two agents (e.g., an antibody and an antigen) and is expressed as a dissociation constant (KD). Affinity can be at least 1-fold greater, at least 2-fold greater, at least 3-fold greater, at least 4-fold greater, at least 5-fold greater, at least 6-fold greater, at least 7-fold greater, at least 8-fold greater, at least 9-fold greater, at least 10-fold greater, at least 20-fold greater, at least 30-fold greater, at least 40-fold greater, at least 50-fold greater, at least 60-fold greater, at least 70-fold greater, at least 80-fold greater, at least 90-fold greater, at least 100-fold greater, or at least 1,000-fold greater, or more, than the affinity of an antibody for unrelated amino acid sequences. Affinity of an antibody to a target protein can be, for example, from about 100 nanomolar (nM) to about 0.1 nM, from about 100 nM to about 1 picomolar (pM), or from about 100 nM to about 1 femtomolar (fM) or more. As used herein, the term "avidity" refers to the resistance of a complex of two or more agents to dissociation after dilution. The terms "immunoreactive" and "preferentially binds" are used interchangeably herein with respect to antibodies and/or antigen-binding fragments.

The term "binding" refers to a direct association between two molecules, due to, for example, covalent, electrostatic, hydrophobic, and ionic and/or hydrogen-bond interactions, including interactions such as salt bridges and water bridges. For example, a subject anti-C1s antibody binds specifically to an epitope within a complement C1s protein. "Specific binding" refers to binding with an affinity of at least about $10^{-7}$ M or greater, e.g., $5 \times 10^{-7}$ M, $10^{-8}$ M, $5 \times 10^{-8}$ M, and greater. "Non-specific binding" refers to binding with an affinity of less than about $10^{-7}$ M, e.g., binding with an affinity of $10^{-6}$ M, $10^{-5}$ M, $10^{-4}$ M, etc.

The term "$k_{on}$", as used herein, is intended to refer to the rate constant for association of an antibody to an antigen.

The term "$k_{off}$", as used herein, is intended to refer to the rate constant for dissociation of an antibody from the antibody/antigen complex.

The term "K$_D$", as used herein, is intended to refer to the equilibrium dissociation constant of an antibody-antigen interaction.

As used herein, *"percent (%) amino acid sequence identity"* and *"homology"* with respect to a peptide, polypeptide or antibody sequence refers to the percentage of amino acid residues in a candidate sequence that are identical with the amino acid residues in the specific peptide or polypeptide sequence, after aligning the sequences and introducing gaps, if necessary, to achieve the maximum percent sequence identity, and not considering any conservative substitutions as part of the sequence identity. Alignment for purposes of determining percent amino acid sequence identity can be achieved in various ways that are within the skill in the art, for instance, using publicly available computer software such as BLAST, BLAST-2, ALIGN or MEGALIGN™ (DNASTAR) software. Those skilled in the art can determine appropriate parameters for measuring alignment, including any algorithms known in the art needed to achieve maximal alignment over the full length of the sequences being compared.

A "biological sample" encompasses a variety of sample types obtained from an individual and can be used in a diagnostic or monitoring assay. The definition encompasses blood and other liquid samples of biological origin, solid tissue samples such as a biopsy specimen or tissue cultures or cells derived therefrom and the progeny thereof. The definition also includes samples that have been manipulated in any way after their procurement, such as by treatment with reagents, solubilization, or enrichment for certain components, such as polynucleotides. The term "biological sample" encompasses a clinical sample, and also includes cells in culture, cell supernatants, cell lysates, serum, plasma, biological fluid, and tissue samples. The term "biological sample" includes urine, saliva, cerebrospinal fluid, interstitial fluid, ocular fluid, synovial fluid, blood fractions such as plasma and serum, and the like. The term "biological sample" also includes solid tissue samples, tissue culture samples, and cellular samples.

An *"isolated"* nucleic acid molecule is a nucleic acid molecule that is identified and separated from at least one contaminant nucleic acid molecule with which it is ordinarily associated in the environment in which it was produced. Preferably, the isolated nucleic acid is free of association with all components associated with the production environment. The isolated nucleic acid molecules encoding the polypeptides and antibodies herein is in a form other than in the form or setting in which it is found in nature. Isolated nucleic acid molecules therefore are distinguished from nucleic acids encoding any polypeptides and antibodies herein that exist naturally in cells.

The term *"vector,"* as used herein, is intended to refer to a nucleic acid molecule capable of transporting another nucleic acid to which it has been linked. One type of vector is a "plasmid," which refers to a circular double stranded DNA into which additional DNA segments may be ligated. Another type of vector is a phage vector. Another type of vector is a viral vector, wherein additional DNA segments may be ligated into the viral genome. Certain vectors are capable of autonomous replication in a host cell into which they are introduced (e.g., bacterial vectors having a bacterial origin of replication and episomal mammalian vectors). Other vectors (e.g., non-episomal mammalian vectors) can be integrated into the genome of a host cell upon introduction into the host cell, and thereby are replicated along with the host genome. Moreover, certain vectors are capable of directing the expression of genes to which they are operatively linked. Such vectors are referred to herein as "recombinant expression vectors," or simply, "expression vectors." In general, expression vectors useful in recombinant DNA techniques are often in the form of plasmids. In the present specification, "plasmid" and "vector" may be used interchangeably as the plasmid is the most commonly used form of vector.

*"Polynucleotide,"* or *"nucleic acid,"* as used interchangeably herein, refer to polymers of nucleotides of any length, and include DNA and RNA. The nucleotides can be deoxyribonucleotides, ribonucleotides, modified nucleotides or bases, and/or their analogs, or any substrate that can be incorporated into a polymer by DNA or RNA polymerase or by a synthetic reaction. A polynucleotide may comprise modified nucleotides, such as methylated nucleotides and their analogs. If present, modification to the nucleotide structure may be imparted before or after assembly of the polymer. The sequence of nucleotides may be interrupted by non-nucleotide components. A polynucleotide may comprise modification(s) made after synthesis, such as conjugation to a label. Other types of modifications include, for example, "caps," substitution of one or more of the naturally occurring nucleotides with an analog, internucleotide modifications such as, for example, those with uncharged linkages (*e.g.,* methyl phosphonates, phosphotriesters, phosphoamidates, carbamates, *etc.*) and with charged linkages (*e.g.,* phosphorothioates, phosphorodithioates, *etc.*), those containing pendant moieties, such as, for example, proteins (*e.g.,* nucleases, toxins, antibodies, signal peptides, ply-L-lysine, *etc.*), those with intercalators (*e.g.,* acridine, psoralen, *etc.*), those containing chelators (*e.g.,* metals, radioactive metals, boron, oxidative metals, *etc.*), those containing alkylators, those with modified linkages (*e.g.,* alpha anomeric nucleic acids, *etc.*), as well as unmodified forms of the polynucleotides(s). Further, any of the hydroxyl groups ordinarily present in the sugars may be replaced, for example, by phosphonate groups, phosphate groups, protected by standard protecting groups, or activated to prepare additional linkages to additional nucleotides, or may be conjugated to solid or semi-solid supports. The 5' and 3' terminal OH can be phosphorylated or substituted with amines or organic capping group moieties of from 1 to 20 carbon atoms. Other hydroxyls may also be derivatized to standard protecting groups. Polynucleotides can also contain analogous forms of ribose or deoxyribose sugars that are generally known in the art, including, for example, 2'-O-methyl-, 2'-O-allyl-, 2'-fluoro- or 2'-azido-ribose, carbocyclic sugar analogs, α-anomeric sugars, epimeric sugars such as arabinose, xyloses or lyxoses, pyranose sugars, furanose sugars, sedoheptuloses, acyclic analogs, and basic nucleoside analogs such as methyl riboside. One or more phosphodiester linkages may be replaced by alternative linking groups. These alternative linking groups include, but are not limited to, implementations wherein phosphate is replaced by P(O)S ("thioate"), P(S)S ("dithioate"), (O)NR$_2$ ("amidate"), P(O)R, P(O)OR', CO, or CH$_2$ ("formacetal"), in which each R or R' is independently H or substituted or unsubstituted alkyl (1-20 C) optionally containing an ether (-O-) linkage, aryl, alkenyl, cycloalkyl, cycloalkenyl or aralkyl. Not all linkages in a polynucleotide need be identical. The preceding description applies to all polynucleotides referred to herein, including RNA and DNA.

A "gene editing agent" as used herein, is defined as an gene editing agent, representative examples of which include CRISPR-associated nucleases such as Cas9 and Cpfl gRNAs, Argonaute family of endonucleases, clustered regularly interspaced short palindromic repeat (CRISPR) nucleases, zinc-finger nucleases (ZFNs), transcription activator-like effector nucleases (TALENs), meganucleases, other endo- and/or exonucleases. See Schiffer, 2012, J Virol 88(17):8920-8936.

An "RNA interfering agent" as used herein, is defined as any agent which interferes with or inhibits expression of a target biomarker gene by RNA interference (RNAi). Such RNA interfering agents include, but are not limited to, nucleic acid molecules including RNA molecules which are homologous to the target biomarker gene of the present disclosure, or a fragment thereof, short interfering RNA (siRNA), and small molecules which interfere with or inhibit expression of a target biomarker nucleic acid by RNA interference (RNAi).

"RNA interference (RNAi)" is an evolutionarily conserved process whereby the expression or introduction of RNA of a sequence that is identical or highly similar to a target biomarker nucleic acid results in the sequence specific degradation or specific post-transcriptional gene silencing (PTGS) of messenger RNA (mRNA) transcribed from that targeted gene (see Coburn, G. and Cullen, B. (2002) J. of Virology 76(18):9225), thereby inhibiting expression of the target biomarker nucleic acid. In one implementation, the RNA is double stranded RNA (dsRNA). This process has been described in plants, invertebrates, and mammalian cells. In nature, RNAi is initiated by the dsRNA-specific endonuclease Dicer, which promotes processive cleavage of long dsRNA into double-stranded fragments termed siRNAs. siRNAs are incorporated into a protein complex that recognizes and cleaves target mRNAs. RNAi can also be initiated by introducing nucleic acid molecules, e.g., synthetic siRNAs, shRNAs, or other RNA interfering agents, to inhibit or silence the expression of target biomarker nucleic acids. As used herein, "inhibition of target biomarker nucleic acid expression" or "inhibition of marker gene expression" includes any decrease in expression or protein activity or level of the target biomarker nucleic acid or protein encoded by the target biomarker nucleic acid. The decrease may be of at least 30%, 40%, 50%, 60%, 70%, 80%, 90%, 95% or 99% or more as compared to the expression of a target biomarker nucleic acid or the activity or level of the protein encoded by a target biomarker nucleic acid which has not been targeted by an RNA interfering agent.

In addition to RNAi, genome editing can be used to modulate the copy number or genetic sequence of a biomarker of interest, such as constitutive or induced knockout or mutation of a biomarker of interest, such as a complement pathway component like C1q, C1r, and/or C1s. For example, the CRISPR-Cas system can be used for precise editing of genomic nucleic acids *(e.g.,* for creating non-functional or null mutations). In such implementations, the CRISPR guide RNA and/or the Cas enzyme may be expressed. For example, a vector containing only the guide RNA can be administered to an animal or cells transgenic for the Cas9 enzyme. Similar strategies may be used (e.g., designer zinc finger, transcription activator-like effectors (TALEs) or homing meganucleases). Such systems are well-known in the art (see, for example, U.S. Pat. No. 8,697,359; Sander and Joung (2014) Nat. Biotech. 32:347-355; Hale et al. (2009) Cell 139:945-956; Karginov and Hannon (2010) Mol. Cell 37:7; U.S. Pat. Publ. 2014/0087426 and 2012/0178169; Boch et al. (2011) Nat. Biotech. 29:135-136; Boch et al. (2009) Science 326:1509-1512; Moscou and Bogdanove (2009) Science 326:1501; Weber et al. (2011) PLoS One 6:e19722; Li et al. (2011) Nucl. Acids Res. 39:6315-6325; Zhang et al. (2011) Nat. Biotech. 29:149-153; Miller et al. (2011) Nat. Biotech. 29: 143-148; Lin et al. (2014) Nucl. Acids Res. 42:e47). Such genetic strategies can use constitutive expression systems or inducible expression systems according to well-known methods in the art.

"Piwi-interacting RNA (piRNA)" is the largest class of small non-coding RNA molecules. piRNAs form RNA-protein complexes through interactions with piwi proteins. These piRNA complexes have been linked to both epigenetic and post-transcriptional gene silencing of retrotransposons and other genetic elements in germ line cells, particularly those in spermatogenesis. They are distinct from microRNA (miRNA) in size (26-31 nt rather than 21-24 nt), lack of sequence conservation, and increased complexity. However, like other small RNAs, piRNAs are thought to be involved in gene silencing, specifically the silencing of transposons. The majority of piRNAs are antisense to transposon sequences, suggesting that transposons are the piRNA target. In mammals it appears that the activity of piRNAs in transposon silencing is most important during the development of the embryo, and in both C. elegans and humans, piRNAs are necessary for spermatogenesis. piRNA has a role in RNA silencing via the formation of an RNA-induced silencing complex (RISC).

"Aptamers" are oligonucleotide or peptide molecules that bind to a specific target molecule. "Nucleic acid aptamers" are nucleic acid species that have been engineered through repeated rounds of in vitro selection or equivalently, SELEX (systematic evolution of ligands by exponential enrichment) to bind to various molecular targets such as small molecules, proteins, nucleic acids, and even cells, tissues and organisms. "Peptide aptamers" are artificial proteins selected or engineered to bind specific target molecules. These proteins consist of one or more peptide loops of variable sequence displayed by a protein scaffold. They are typically isolated from combinatorial libraries and often subsequently improved by directed mutation or rounds of variable region mutagenesis and selection. The "Affimer protein", an evolution of peptide aptamers, is a small, highly stable protein engineered to display peptide loops which provides a high affinity binding surface for a specific target protein. It is a protein of low molecular weight, 12-14 kDa, derived from the cysteine protease inhibitor family of cystatins. Aptamers are useful in biotechnological and therapeutic applications as they offer molecular recognition properties that rival that of the commonly used biomolecule, antibodies. In addition to their discriminate recognition, aptamers offer advantages over antibodies as they can be engineered completely in a test tube, are readily produced by chemical synthesis, possess desirable storage properties, and elicit little or no immunogenicity in therapeutic applications.

"Short interfering RNA" (siRNA), also referred to herein as "small interfering RNA" is defined as an agent which functions to inhibit expression of a target biomarker nucleic acid, e.g., by RNAi. An siRNA may be chemically synthesized, may be produced by in vitro transcription, or may be produced within a host cell. In one implementation, siRNA is a double stranded RNA (dsRNA) molecule of about 15 to about 40 nucleotides in length, preferably about 15 to about 28 nucleotides, more preferably about 19 to about 25 nucleotides in length, and more preferably about 19, 20, 21, or 22 nucleotides in length, and may contain a 3' and/or 5' overhang on each strand having a length of about 0, 1, 2, 3, 4, or 5 nucleotides. The length of the overhang is independent between the two strands, i.e., the length of the overhang on one strand is not dependent on the length of the overhang on the second strand. Preferably, the siRNA is capable of promoting RNA interference through degradation or specific post-transcriptional gene silencing (PTGS) of the target messenger RNA (mRNA).

In another implementation, an siRNA is a small hairpin (also called stem loop) RNA (shRNA). In one implementation, these shRNAs are composed of a short (e.g., 19-25 nucleotide) antisense strand, followed by a 5-9 nucleotide loop, and the analogous sense strand. Alternatively, the sense strand may precede the nucleotide loop structure and the antisense strand may follow. These shRNAs may be contained in plasmids, retroviruses, and lentiviruses and expressed from, for example, the pol III U6 promoter, or another promoter (see, e.g., Stewart, et al. (2003) RNA Apr;9(4):493-501).

A *"host cell'* includes an individual cell or cell culture that can be or has been a recipient for vector(s) for incorporation of polynucleotide inserts. Host cells include progeny of a single host cell, and the progeny may not necessarily be completely identical (in morphology or in genomic DNA complement) to the original parent cell due to natural, accidental, or deliberate mutation. A host cell includes cells transfected *in vivo* with a polynucleotide(s) of this disclosure.

*"Carriers"* as used herein include pharmaceutically acceptable carriers, excipients, or stabilizers that are nontoxic to the cell or mammal being exposed thereto at the dosages and concentrations employed. Often the physiologically acceptable carrier is an aqueous pH buffered solution. Examples of physiologically acceptable carriers include buffers such as phosphate, citrate, and other organic acids; antioxidants including ascorbic acid; low molecular weight (less than about 10 residues) polypeptide; proteins, such as serum albumin, gelatin, or immunoglobulins; hydrophilic polymers such as polyvinylpyrrolidone; amino acids such as glycine, glutamine, asparagine, arginine or lysine; monosaccharides, disaccharides, and other carbohydrates including glucose, mannose, or dextrins; chelating agents such as EDTA; sugar alcohols such as mannitol or sorbitol; salt-forming counterions such as sodium; and/or nonionic surfactants such as TWEEN™, polyethylene glycol (PEG), and PLURONICS™.

The term *"preventing"* is art-recognized, and when used in relation to a condition, such as a brain injury, preferably a traumatic brain injury, hypoxic brain injury, brain infection, or stroke, is well understood in the art, and includes administration of a composition which reduces the frequency or severity, or delays the onset, of one or more symptoms of the medical condition in a subject relative to a subject who does not receive the composition. Thus, the prevention of a brain injury, preferably a traumatic brain injury, hypoxic brain injury, brain infection, or stroke includes, for example, increasing the platelet count in a population of patients receiving a therapy relative to a control population that did not receive the therapy, e.g., by a statistically and/or clinically significant amount. Similarly, the prevention of a brain injury, preferably a traumatic brain injury, hypoxic brain injury, brain infection, or stroke includes reducing the likelihood that a patient receiving a therapy will develop a brain injury, preferably a traumatic brain injury, hypoxic brain injury, brain infection, or stroke or related symptoms, relative to a patient who does not receive the therapy.

The term *"subject"* as used herein refers to a living mammal and may be interchangeably used with the term "patient". Examples of mammals include, but are not limited to, any member of the mammalian class: humans, non-human primates such as chimpanzees, and other apes and monkey species; farm animals such as cattle, horses, sheep, goats, swine; domestic animals such as rabbits, dogs, and cats; laboratory animals including rodents, such as rats, mice and guinea pigs, and the like. The term does not denote a particular age or gender.

As used herein, the term *"treating"* or *"treatment"* includes reducing, arresting, or reversing the symptoms, clinical signs, or underlying pathology of a condition to stabilize or improve a subject's condition or to reduce the likelihood that the subject's condition will worsen as much as if the subject did not receive the treatment.

The term *"therapeutically effective amount"* of a compound with respect to the subject method of treatment refers to an amount of the compound(s) in a preparation which, when administered as part of a desired dosage regimen (to a mammal, preferably a human) alleviates a symptom, ameliorates a condition, or slows the onset of disease conditions according to clinically acceptable standards for the disorder or condition to be treated or the cosmetic purpose, *e.g.,* at a reasonable benefit/risk ratio applicable to any medical treatment. A therapeutically effective amount herein may vary according to factors such as the disease state, age, sex, and weight of the patient, and the ability of the antibody to elicit a desired response in the individual.

As used herein, an individual *"at risk"* of developing a particular disease, disorder, or condition may or may not have detectable disease or symptoms of disease, and may or may not have displayed detectable disease or symptoms of disease prior to the treatment methods described herein. "At risk" denotes that an individual has one or more risk factors, which are measurable parameters that correlate with development of a particular disease, disorder, or condition, as known in the art. An individual having one or more of these risk factors has a higher probability of developing a particular disease, disorder, or condition than an individual without one or more of these risk factors.

*"Chronic"* administration refers to administration of the medicament(s) in a continuous as opposed to acute mode, so as to maintain the initial therapeutic effect (activity) for an extended period of time. *"Intermittent"* administration refers to treatment that is not administered consecutively without interruption, but rather is cyclic/periodic in nature.

As used herein, administration *"conjointly"* with another compound or composition includes simultaneous administration and/or administration at different times. Conjoint administration also encompasses administration as a co-formulation or administration as separate compositions, including at different dosing frequencies or intervals, and using the same route of administration or different routes of administration.

Unless defined otherwise, all technical and scientific terms used herein have the same meaning as commonly understood by one of ordinary skill in the art to which this disclosure belongs. References to disclose and describe the methods and/or materials in connection with which the publications are cited are, for example, the widely utilized methodologies described in Sambrook et al., Molecular Cloning: A Laboratory Manual 3d edition (2001) Cold Spring Harbor Laboratory Press, Cold Spring Harbor, N.Y.; Current Protocols in Molecular Biology (F.M. Ausubel, et al. eds., (2003)); the series Methods in Enzymology (Academic Press, Inc.): PCR 2: A Practical Approach (M.J. MacPherson, B.D. Hames and G.R. Taylor eds. (1995)), Harlow and Lane, eds. (1988) Antibodies, A Laboratory Manual, and Animal Cell Culture (R.I. Freshney, ed. (1987)); Oligonucleotide Synthesis (M.J. Gait, ed., 1984); Methods in Molecular Biology, Humana Press; Cell Biology: A Laboratory Notebook (J.E. Cellis, ed., 1998) Academic Press; Animal Cell Culture (R.I. Freshney), ed., 1987); Introduction to Cell and Tissue Culture (J.P. Mather and P.E. Roberts, 1998) Plenum Press; Cell and Tissue Culture: Laboratory Procedures (A. Doyle, J.B. Griffiths, and D.G. Newell, eds., 1993-8) J. Wiley and Sons; Handbook of Experimental Immunology (D.M. Weir and C.C. Blackwell, eds.); Gene Transfer Vectors for Mammalian Cells (J.M. Miller and M.P. Calos, eds., 1987); PCR: The Polymerase Chain Reaction, (Mullis et al., eds., 1994); Current Protocols in Immunology (J.E. Coligan et al., eds., 1991); Short Protocols in Molecular Biology (Wiley and Sons, 1999); Immunobiology (C.A. Janeway and P. Travers, 1997); Antibodies (P. Finch, 1997); Antibodies: A Practical Approach (D. Catty., ed., IRL Press, 1988-1989); Monoclonal Antibodies: A Practical Approach (P. Shepherd and C. Dean, eds., Oxford University Press, 2000); Using Antibodies: A Laboratory Manual (E. Harlow and D. Lane (Cold Spring Harbor Laboratory Press, 1999); The Antibodies (M. Zanetti and J. D. Capra, eds., Harwood Academic Publishers, 1995); and Cancer: Principles and Practice of Oncology (V.T. DeVita et al., eds., J.B. Lippincott Company, 1993).

*Nucleic acids, vectors and host cells*

Antibodies suitable for use in the methods of the present disclosure may be produced using recombinant methods and compositions, e.g., as described in U.S. Patent No. 4,816,567. In some implementations, isolated nucleic acids having a nucleotide sequence encoding any of the antibodies of the present disclosure are provided. Such nucleic acids may encode an amino acid sequence containing the $V_L/C_L$ and/or an amino acid sequence containing the $V_H/C_H1$ of the anti-C1q, anti-C1r or anti-C1s antibody. In some implementations, one or more vectors (*e.g.*, expression vectors) containing such nucleic acids are provided. A host cell containing such nucleic acid may also be provided. The host cell may contain (*e.g.*, has been transduced with): (1) a vector containing a nucleic acid that encodes an amino acid sequence containing the $V_L/C_L$ of the antibody and an amino acid sequence containing the $V_H/C_H1$ of the antibody, or (2) a first vector containing a nucleic acid that encodes an amino acid sequence containing the $V_L/C_L$ of the antibody and a second vector containing a nucleic acid that encodes an amino acid sequence containing the $V_H/C_H1$ of the antibody. In some implementations, the host cell is eukaryotic, *e.g.*, a Chinese Hamster Ovary (CHO) cell or lymphoid cell (*e.g.,* Y0, NS0, Sp20 cell).

Methods of making an anti-C1q, anti-C1r or anti-C1s antibody are disclosed herein. The method includes culturing a host cell of the present disclosure containing a nucleic acid encoding the anti-C1q, anti-C1r or anti-C1s antibody, under conditions suitable for expression of the antibody. In some implementations, the antibody is subsequently recovered from the host cell (or host cell culture medium).

For recombinant production of a humanized anti-C1q, anti-C1r or anti-C1s antibody of the present disclosure, a nucleic acid encoding the antibody is isolated and inserted into one or more vectors for further cloning and/or expression in a host cell. Such nucleic acid may be readily isolated and sequenced using conventional procedures (*e.g.*, by using oligonucleotide probes that are capable of binding specifically to genes encoding the heavy and light chains of the antibody).

Suitable vectors containing a nucleic acid sequence encoding any of the antibodies of the present disclosure, or fragments thereof polypeptides (including antibodies) described herein include, without limitation, cloning vectors and expression vectors. Suitable cloning vectors can be constructed according to standard techniques, or may be selected from a large number of cloning vectors available in the art. While the cloning vector selected may vary according to the host cell intended to be used, useful cloning vectors generally have the ability to self-replicate, may possess a single target for a particular restriction endonuclease, and/or may carry genes for a marker that can be used in selecting clones containing the vector. Suitable examples include plasmids and bacterial viruses, *e.g.*, pUC18, pUC19, Bluescript (*e.g.*, pBS SK+) and its derivatives, mpl8, mpl9, pBR322, pMB9, ColE1, pCR1, RP4, phage DNAs, and shuttle vectors such as pSA3 and pAT28. These and many other cloning vectors are available from commercial vendors such as BioRad, Stratagene, and Invitrogen.

The vectors containing the nucleic acids of interest can be introduced into the host cell by any of a number of appropriate means, including electroporation, transfection employing calcium chloride, rubidium chloride, calcium phosphate, DEAE-dextran, or other substances; microprojectile bombardment; lipofection; and infection (*e.g.*, where the vector is an infectious agent such as vaccinia virus). The choice of introducing vectors or polynucleotides will often depend on features of the host cell. In some implementations, the vector contains a nucleic acid containing one or more amino acid sequences encoding an anti-C1q, anti-C1r or anti-C1s antibody of the present disclosure.

Suitable host cells for cloning or expression of antibody-encoding vectors include prokaryotic or eukaryotic cells. For example, an anti-C1q, anti-C1r or anti-C1s antibody of the present disclosure may be produced in bacteria, in particular when glycosylation and Fc effector function are not needed. For expression of antibody fragments and polypeptides in bacteria (*e.g.,* U.S. Patent Nos. 5,648,237, 5,789,199, and 5,840,523; and Charlton, Methods in Molecular Biology, Vol. 248 (B.K.C. Lo, ed., Humana Press, Totowa, NJ, 2003), pp. 245-254, describing expression of antibody fragments in *E. coli*.). After expression, the antibody may be isolated from the bacterial cell paste in a soluble fraction and can be further purified.

*Antibody Screening*

Candidate antibodies can be screened for the ability to modulate complement activation. Such screening may be performed using an *in vitro* model, a genetically altered cell or animal, or purified protein. A wide variety of assays may be used for this purpose, such as an *in vitro* culture system.

Candidate antibodies may also be identified using computer-based modeling, by binding assays, and the like. Various in vitro models may be used to determine whether an antibody binds to, or otherwise affects complement activity. Such candidate antibodies may be tested by contacting plasma from a healthy donor and determine complement activation (e.g., by the antigen C3c capture ELISA). Such antibodies may be further tested in an *in vivo* model for an effect on a brain injury, preferably a traumatic brain injury, hypoxic brain injury, brain infection, or stroke.

Generally, a plurality of assay mixtures are run in parallel with different antibody concentrations to obtain a differential response to the various concentrations. Typically one of these concentrations serves as a negative control, i.e., at zero concentration or below the level of detection.

*Pharmaceutical Compositions and Administration*

A complement (e.g. an antibody) of the present disclosure may be administered in the form of pharmaceutical compositions.

Therapeutic formulations of an inhibitor of the disclosure may be prepared for storage by mixing the inhibitor having the desired degree of purity with optional pharmaceutically acceptable carriers, excipients or stabilizers (Remington's Pharmaceutical Sciences 16th edition, Osol, A. Ed. [1980]), in the form of lyophilized formulations or aqueous solutions. Acceptable carriers, excipients, or stabilizers are nontoxic to recipients at the dosages and concentrations employed, and include buffers such as phosphate, citrate, and other organic acids; antioxidants including ascorbic acid and methionine; preservatives (such as octadecyldimethylbenzyl ammonium chloride; hexamethonium chloride; benzalkonium chloride, benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (*e.g.*, Zn-protein complexes); and/or non-ionic surfactants such as TWEEN™, PLURONICS™ or polyethylene glycol (PEG).

Lipofections or liposomes may also be used to deliver an antibody or antibody fragment into a cell, wherein the epitope or smallest fragment which specifically binds to the binding domain of the target protein is preferred.

The inhibitor may also be entrapped in microcapsules prepared, for example, by coacervation techniques or by interfacial polymerization, for example, hydroxymethylcellulose or gelatin-microcapsules and poly-(methylmethacrylate) microcapsules, respectively, in colloidal drug delivery systems (for example, liposomes, albumin microspheres, microemulsions, nano-particles and nanocapsules) or in macroemulsions. Such techniques are disclosed in Remington's Pharmaceutical Sciences 16th edition, Osol, A. Ed. (1980).

The formulations to be used for parenteral administration must be sterile. This is readily accomplished by filtration through sterile filtration membranes.

Sustained-release preparations may be prepared. Suitable examples of sustained-release preparations include semipermeable matrices of solid hydrophobic polymers containing the inhibitor, which matrices are in the form of shaped articles, *e.g.*, films, or microcapsules. Examples of sustained-release matrices include polyesters, hydrogels (for example, poly(2-hydroxyethyl-methacrylate), or poly(vinylalcohol)), polylactides (U.S. Pat. No. 3,773,919), copolymers of L-glutamic acid and $\gamma$ ethyl-L-glutamate, non-degradable ethylene-vinyl acetate, degradable lactic acid-glycolic acid copolymers such as the LUPRON DEPOT™ (injectable microspheres composed of lactic acid-glycolic acid copolymer and leuprolide acetate), and poly-D-(-)-3-hydroxybutyric acid. While polymers such as ethylene-vinyl acetate and lactic acid-glycolic acid enable release of molecules for over 100 days, certain hydrogels release proteins for shorter time periods.

The antibodies and compositions of the present disclosure are typically administered by various routes, including, but not limited to, topical, parenteral, subcutaneous, intraperitoneal, intrapulmonary, intranasal, and intralesional administration. Parenteral routes of administration include intramuscular, intravenous, intra-arterial, intraperitoneal, intrathecal, or subcutaneous administration.

Pharmaceutical compositions may also include, depending on the formulation desired, pharmaceutically-acceptable, non-toxic carriers of diluents, which are defined as vehicles commonly used to formulate pharmaceutical compositions for animal or human administration. The diluent is selected so as not to affect the biological activity of the combination. Examples of such diluents are distilled water, buffered water, physiological saline, PBS, Ringer's solution, dextrose solution, and Hank's solution. In addition, the pharmaceutical composition or formulation may include other carriers, adjuvants, or nontoxic, nontherapeutic, non-immunogenic stabilizers, excipients and the like. The compositions may also include additional substances to approximate physiological conditions, such as pH adjusting and buffering agents, toxicity adjusting agents, wetting agents and detergents.

The composition may also include any of a variety of stabilizing agents, such as an antioxidant for example. When the pharmaceutical composition includes a polypeptide, the polypeptide may be complexed with various well-known compounds that enhance the *in vivo* stability of the polypeptide, or otherwise enhance its pharmacological properties (*e.g.*, increase the half-life of the polypeptide, reduce its toxicity, enhance other pharmacokinetic and/or pharmacodynamic characteristics, or enhance solubility or uptake). Examples of such modifications or complexing agents include sulfate, gluconate, citrate and phosphate. The polypeptides of a composition may also be complexed with molecules that enhance their in vivo attributes. Such molecules include, for example, carbohydrates, polyamines, amino acids, other peptides, ions (*e.g.*, sodium, potassium, calcium, magnesium, manganese), and lipids. Further guidance regarding formulations that are suitable for various types of administration may be found in Remington's Pharmaceutical Sciences, Mace Publishing Company, Philadelphia, Pa., 17th ed. (1985). For a brief review of methods for drug delivery, see, Langer, Science 249:1527-1533 (1990).

Toxicity and therapeutic efficacy of the active ingredient may be determined according to standard pharmaceutical procedures in cell cultures and/or experimental animals, including, for example, determining the LD50 (the dose lethal to 50% of the population) and the ED50 (the dose therapeutically effective in 50% of the population). The dose ratio between toxic and therapeutic effects is the therapeutic index and it may be expressed as the ratio LD50/ED50. Compounds that exhibit large therapeutic indices are preferred.

The data obtained from cell culture and/or animal studies may be used in formulating a range of dosages for humans. The dosage of the active ingredient typically lines within a range of circulating concentrations that include the ED50 with low toxicity. The dosage may vary within this range depending upon the dosage form employed and the route of administration utilized.

The pharmaceutical compositions described herein may be administered in a variety of different ways. Examples include administering a composition containing a pharmaceutically acceptable carrier via oral, intranasal, rectal, topical, intraperitoneal, intravenous, intramuscular, subcutaneous, subdermal, transdermal, intrathecal, and intracranial methods.

For oral administration, the active ingredient may be administered in solid dosage forms, such as capsules, tablets, and powders, or in liquid dosage forms, such as elixirs, syrups, and suspensions. The active component(s) may be encapsulated in gelatin capsules together with inactive ingredients and powdered carriers, such as glucose, lactose, sucrose, mannitol, starch, cellulose or cellulose derivatives, magnesium stearate, stearic acid, sodium saccharin, talcum, magnesium carbonate. Examples of additional inactive ingredients that may be added to provide desirable color, taste, stability, buffering capacity, dispersion or other known desirable features are red iron oxide, silica gel, sodium lauryl sulfate, titanium dioxide, and edible white ink. Similar diluents may be used to make compressed tablets. Both tablets and capsules may be manufactured as sustained release products to provide for continuous release of medication over a period of hours. Compressed tablets may be sugar coated or film coated to mask any unpleasant taste and protect the tablet from the atmosphere, or enteric-coated for selective disintegration in the gastrointestinal tract. Liquid dosage forms for oral administration may contain coloring and flavoring to increase patient acceptance.

Formulations suitable for parenteral administration include aqueous and non-aqueous, isotonic sterile injection solutions, which may contain antioxidants, buffers, bacteriostats, and solutes that render the formulation isotonic with the blood of the intended recipient, and aqueous and non-aqueous sterile suspensions that may include suspending agents, solubilizers, thickening agents, stabilizers, and preservatives.

The components used to formulate the pharmaceutical compositions are preferably of high purity and are substantially free of potentially harmful contaminants (*e.g.*, at least National Food (NF) grade, generally at least analytical grade, and more typically at least pharmaceutical grade). Moreover, compositions intended for parenteral use are usually sterile. To the extent that a given compound must be synthesized prior to use, the resulting product is typically substantially free of any potentially toxic agents, particularly any endotoxins, which may be present during the synthesis or purification process. Compositions for parental administration are also typically substantially isotonic and made under GMP conditions.

The compositions of the disclosure may be administered using any medically appropriate procedure, *e.g.*, intravascular (intravenous, intraarterial, intracapillary) administration, injection into the cerebrospinal fluid, intravitreal, topical, intracavity or direct injection in the brain. Intrathecal administration may be carried out through the use of an Ommaya reservoir, in accordance with known techniques. (F. Balis et al., Am J. Pediatr. Hematol. Oncol. 11, 74, 76 (1989).

Where the therapeutic agents are locally administered in the brain, one method for administration of the therapeutic compositions of the disclosure is by deposition into or near the site by any suitable technique, such as by direct injection (aided by stereotaxic positioning of an injection syringe, if necessary) or by placing the tip of an Ommaya reservoir into a cavity, or cyst, for administration. Alternatively, a convection-enhanced delivery catheter may be implanted directly into the site, into a natural or surgically created cyst, or into the normal brain mass. Such convection-enhanced pharmaceutical composition delivery devices greatly improve the diffusion of the composition throughout the brain mass. The implanted catheters of these delivery devices utilize high-flow microinfusion (with flow rates in the range of about 0.5 to 15.0 $\mu$l/minute), rather than diffusive flow, to deliver the therapeutic composition to the brain and/or tumor mass. Such devices are described in U.S. Pat. No. 5,720,720.

The effective amount of a therapeutic composition given to a particular patient may depend on a variety of factors, several of which may be different from patient to patient. A competent clinician will be able to determine an effective amount of a therapeutic agent to administer to a patient. Dosage of the agent will depend on the treatment, route of administration, the nature of the therapeutics, sensitivity of the patient to the therapeutics, etc. Utilizing LD50 animal data, and other information, a clinician may determine the maximum safe dose for an individual, depending on the route of administration. Utilizing ordinary skill, the competent clinician will be able to optimize the dosage of a particular therapeutic composition in the course of routine clinical trials. The compositions may be administered to the subject in a series of more than one administration. For therapeutic compositions, regular periodic administration will sometimes be required, or may be desirable. Therapeutic regimens will vary with the agent; for example, some agents may be taken for extended periods of time on a daily or semi-daily basis, while more selective agents may be administered for more defined time courses, *e.g.*, one, two three or more days, one or more weeks, one or more months, etc., taken daily, semi-daily, semi-weekly, weekly, etc.

Formulations may be optimized for retention and stabilization in the brain. When the agent is administered into the cranial compartment, it is desirable for the agent to be retained in the compartment, and not to diffuse or otherwise cross the blood brain barrier. Stabilization techniques include cross-linking, multimerizing, or linking to groups such as polyethylene glycol, polyacrylamide, neutral protein carriers, etc., in order to achieve an increase in molecular weight.

Other strategies for increasing retention include the entrapment of the agent in a biodegradable or bioerodible implant. The rate of release of the therapeutically active agent is controlled by the rate of transport through the polymeric matrix, and the biodegradation of the implant. The transport of drug through the polymer barrier will also be affected by compound solubility, polymer hydrophilicity, extent of polymer cross-linking, expansion of the polymer upon water absorption so as to make the polymer barrier more permeable to the drug, geometry of the implant, and the like. The implants are of dimensions commensurate with the size and shape of the region selected as the site of implantation. Implants may be particles, sheets, patches, plaques, fibers, microcapsules and the like and may be of any size or shape compatible with the selected site of insertion.

The implants may be monolithic, i.e., having the active agent homogenously distributed through the polymeric matrix, or encapsulated, where a reservoir of active agent is encapsulated by the polymeric matrix. The selection of the polymeric composition to be employed will vary with the site of administration, the desired period of treatment, patient tolerance, the nature of the disease to be treated and the like. Characteristics of the polymers will include biodegradability at the site of implantation, compatibility with the agent of interest, ease of encapsulation, a half-life in the physiological environment.

Biodegradable polymeric compositions which may be employed may be organic esters or ethers, which when degraded result in physiologically acceptable degradation products, including the monomers. Anhydrides, amides, orthoesters or the like, by themselves or in combination with other monomers, may find use. The polymers may be condensation polymers. The polymers may be cross-linked or non-cross-linked. Of particular interest are polymers of hydroxyaliphatic carboxylic acids, either homo- or copolymers, and polysaccharides. Included among the polyesters of interest are polymers of D-lactic acid, L-lactic acid, racemic lactic acid, glycolic acid, polycaprolactone, and combinations thereof. By employing the L-lactate or D-lactate, a slowly biodegrading polymer is achieved, while degradation is substantially enhanced with the racemate. Copolymers of glycolic and lactic acid are of particular interest, where the rate of biodegradation is controlled by the ratio of glycolic to lactic acid. The most rapidly degraded copolymer has roughly equal amounts of glycolic and lactic acid, where either homopolymer is more resistant to degradation. The ratio of glycolic acid to lactic acid will also affect the brittleness of in the implant, where a more flexible implant is desirable for larger geometries. Among the polysaccharides of interest are calcium alginate, and functionalized celluloses, particularly carboxymethylcellulose esters characterized by being water insoluble, a molecular weight of about 5 kD to 500 kD, etc. Biodegradable hydrogels may also be employed in the implants of the subject disclosure. Hydrogels are typically a copolymer material, characterized by the ability to imbibe a liquid. Exemplary biodegradable hydrogels which may be employed are described in Heller in: Hydrogels in Medicine and Pharmacy, N. A. Peppes ed., Vol. III, CRC Press, Boca Raton, Fla., 1987, pp 137-149.

*Kits*

The present disclosure also provides a pharmaceutical pack or kit comprising one or more containers filled with one or more of the ingredients of the pharmaceutical compositions. Associated with such container(s) may be a notice in the form prescribed by a governmental agency regulating the manufacture, use or sale of pharmaceuticals or biological products, which notice reflects approval by the agency of manufacture, use or sale for human administration.

Kits of the present disclosure may include one or more containers comprising a purified anti-C1q, anti-C1r or anti-C1s antibody and instructions for use in accordance with methods known in the art. Generally, these instructions comprise a description of administration of the inhibitor to treat or diagnose a disease, according to any methods known in the art. The kit may further comprise a description of selecting an individual suitable for treatment based on identifying whether that individual has a brain injury, preferably a traumatic brain injury, hypoxic brain injury, brain infection, or stroke.

The instructions generally include information as to dosage, dosing schedule, and route of administration for the intended treatment. The containers may be unit doses, bulk packages (*e.g.*, multi-dose packages) or sub-unit doses. Instructions supplied in the kits of the present disclosure are typically written instructions on a label or package insert (*e.g.*, a paper sheet included in the kit), but machine-readable instructions (*e.g.*, instructions carried on a magnetic or optical storage disk) are also acceptable.

The label or package insert indicates that the composition is used for treating a brain injury, preferably a traumatic brain injury, hypoxic brain injury, brain infection, or stroke. Instructions may be provided for practicing any of the methods described herein.

The kits of this disclosure are preferably disposed in suitable packaging. Suitable packaging includes, but is not limited to, vials, bottles, jars, flexible packaging (*e.g.*, sealed Mylar or plastic bags), and the like. Also contemplated are packages for use in combination with a specific device, such as an inhaler, nasal administration device (*e.g.*, an atomizer) or an infusion device such as a minipump. A kit may have a sterile access port (for example the container may be an intravenous solution bag or a vial having a stopper pierceable by a hypodermic injection needle). The container may also have a sterile access port (*e.g.*, the container may be an intravenous solution bag or a vial having a stopper pierceable by a hypodermic injection needle). At least one active agent in the composition is an inhibitor of classical complement pathway. The container may further comprise a second pharmaceutically active agent.

Kits may optionally provide additional components such as buffers and interpretive information. Normally, the kit comprises a container and a label or package insert(s) on or associated with the container.

*Inhibition of Complement*

A number of molecules are known that inhibit the activity of complement. In addition to known compounds, suitable inhibitors can be screened by methods described herein. As described above, normal cells can produce proteins that block complement activity, *e.g.*, CD59, C1 inhibitor, etc. In some implementations of the disclosure, complement is inhibited by upregulating expression of genes encoding such polypeptides.

Modifications of molecules that block complement activation are also known in the art. For example, such molecules include, without limitation, modified complement receptors, such as soluble CR1. The mature protein of the most common allotype of CR1 contains 1998 amino acid residues: an extracellular domain of 1930 residues, a transmembrane region of 25 residues, and a cytoplasmic domain of 43 residues. The entire extracellular domain is composed of 30 repeating units referred to as short consensus repeats (SCRs) or complement control protein repeats (CCPRs), each consisting of 60 to 70 amino acid residues. Recent data indicate that C1q binds specifically to human CR1. Thus, CR1 recognizes all three complement opsonins, namely C3b, C4b, and C1q. A soluble version of recombinant human CR1 (sCR1) lacking the transmembrane and cytoplasmic domains has been produced and shown to retain all the known functions of the native CR1. The cardioprotective role of sCR1 in animal models of ischemia/reperfusion injury has been confirmed. Several types of human C1q receptors (C1qR) have been described. These include the ubiquitously distributed 60- to 67-kDa receptor, referred to as cC1qR because it binds the collagen-like domain of C1q. This C1qR variant was shown to be calreticulin; a 126-kDa receptor that modulates monocyte phagocytosis. gC1qR is not a membrane-bound molecule, but rather a secreted soluble protein with affinity for the globular regions of C1q, and may act as a fluid-phase regulator of complement activation.

Decay accelerating factor (DAF) (CD55) is composed of four SCRs plus a serine/threonine-enriched domain that is capable of extensive O-linked glycosylation. DAF is attached to cell membranes by a glycosyl phosphatidyl inositol (GPI) anchor and, through its ability to bind C4b and C3b, it acts by dissociating the C3 and C5 convertases. Soluble versions of DAF (sDAF) have been shown to inhibit complement activation.

C1 inhibitor, a member of the "serpin" family of serine protease inhibitors, is a heavily glycosylated plasma protein that prevents fluid-phase C1 activation. C1 inhibitor regulates the classical pathway of complement activation by blocking the active site of C1r and C1s and dissociating them from C1q.

Peptide inhibitors of complement activation include C5a and other inhibitory molecules include Fucan.

## Methods of Treatment

By administering agents that inhibit complement activation, production of antigenspecific antibodies that mediate a brain injury, preferably a traumatic brain injury, hypoxic brain injury, brain infection, or stroke can be reduced. Such agents include an anti-C1q, antiC1r, or anti-C1s antibody inhibitor. Other agents may include inhibitors that upregulate expression of native complement, or agents that down-regulate C1q, C1r or C1s synthesis in cells, agents that block complement activation, agents that block the signal for complement activation, and the like.

The methods neutralize complement biological activity. The affected complement biological activity could be (1) C1q binding to an autoantibody, (2) C1q binding to C1r, (3) C1q binding to C1s, (4) C1q binding to IgM, (5) C1q binding to phosphatidylserine, (6) C1q binding to pentraxin-3, (7) C1q binding to C-reactive protein (CRP), (8) C1q binding to globular C1q receptor (gC1qR), (9) C1q binding to complement receptor 1 (CR1), (10) C1q binding to beta-amyloid, (11) C1q binding to calreticulin, (12) C1q binding to apoptotic cells, or (13) C1q binding to B cells. The affected complement biological activity could further be (1) activation of the classical complement activation pathway, (2) activation of antibody and complement dependent cytotoxicity, (3) CH50 hemolysis, (4) synapse loss, (5) B-cell antibody production, (6) dendritic cell maturation, (7) T-cell proliferation, (8) cytokine production (9) microglia activation, (10) Arthus reaction, (11) phagocytosis of synapses or nerve endings, or (12) activation of complement receptor 3 (CR3/C3) expressing cells.

It is contemplated that compositions may be obtained and used under the guidance of a physician for *in vivo* use. The dosage of the therapeutic formulation may vary widely, depending upon the nature of the disease, the frequency of administration, the manner of administration, the clearance of the agent from the host, and the like.

The effective amount of a therapeutic composition given to a particular patient may depend on a variety of factors, several of which may be different from patient to patient. Utilizing ordinary skill, the competent clinician will be able to tailor the dosage of a particular therapeutic or imaging composition in the course of routine clinical trials.

Therapeutic agents, e.g., inhibitors of complement, activators of gene expression, etc. can be incorporated into a variety of formulations for therapeutic administration by combination with appropriate pharmaceutically acceptable carriers or diluents, and may be formulated into preparations in solid, semi-solid, liquid or gaseous forms, such as tablets, capsules, powders, granules, ointments, solutions, suppositories, injections, inhalants, gels, microspheres, and aerosols. As such, administration of the compounds can be achieved in various ways, including oral, buccal, rectal, parenteral, intraperitoneal, intradermal, transdermal, intrathecal, nasal, intratracheal, etc., administration. The active agent may be systemic after administration or may be localized by the use of regional administration, intramural administration, or use of an implant that acts to retain the active dose at the site of implantation.

## Combination Treatments

The complement inhibitors of the present disclosure may be used, without limitation, conjointly with any additional treatment, such as an anticoagulant, for treating a brain injury, preferably a traumatic brain injury, hypoxic brain injury, brain infection, or stroke.

In some implementations, the antibodies of this disclosure may be administered in combination with an inhibitor of the alternative pathway of complement activation. Such inhibitors may include, without limitation, factor B blocking antibodies, factor D blocking antibodies, soluble, membrane-bound, tagged or fusion-protein forms of CD59, DAF, CR1, CR2, Crry or Compstatin-like peptides that block the cleavage of C3, non-peptide C3aR antagonists such as SB 290157, Cobra venom factor or non-specific complement inhibitors such as nafamostat mesilate (FUTHAN; FUT-175), aprotinin, K-76 monocarboxylic acid (MX-1) and heparin (*see, e.g.,* T.E. Mollnes & M. Kirschfink, Molecular Immunology 43 (2006) 107-121).

*Diagnostic Assays*

The present disclosure provides, in part, methods of determining a subject's risk of developing a brain injury, preferably a traumatic brain injury, hypoxic brain injury, brain infection, or stroke, comprising: administering an anti-C1q, anti-C1r, or anti-C1s antibody to the subject, wherein the anti-C1q, anti-C1r, or anti-C1s is coupled to a detectable label; detecting the detectable label to measure the amount or location of C1q, C1r, or C1s in the subject; and comparing the amount or location of one or more of C1q, C1r, or C1s to a reference, wherein the risk of developing a brain injury, preferably a traumatic brain injury, hypoxic brain injury, brain infection, or stroke is characterized based on the comparison of the amount or location of one or more of C1q, C1r, or C1s to the reference.

An exemplary method for detecting the level of C1q, C1r, or C1s, and thus useful for classifying whether a sample is associated with a brain injury, preferably a traumatic brain injury, hypoxic brain injury, brain infection, or stroke or a clinical subtype thereof involves obtaining a biological sample from a test subject and contacting the biological sample with an antibody capable of detecting C1q, C1r, or C1s such that the level of C1q, C1r, or C1s is detected in the biological sample. In certain instances, the statistical algorithm is a single learning statistical classifier system. For example, a single learning statistical classifier system can be used to classify a sample as a C1q, C1r, or C1s sample based upon a prediction or probability value and the presence or level of C1q, C1r, or C1s. The use of a single learning statistical classifier system typically classifies the sample as a C1q, C1r, or C1s sample with a sensitivity, specificity, positive predictive value, negative predictive value, and/or overall accuracy of at least about 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99%.

Other suitable statistical algorithms are well-known to those of skill in the art. For example, learning statistical classifier systems include a machine learning algorithmic technique capable of adapting to complex data sets (*e.g.*, panel of markers of interest) and making decisions based upon such data sets. In some implementations, a single learning statistical classifier system such as a classification tree (*e.g.*, random forest) is used. In other implementations, a combination of 2, 3, 4, 5, 6, 7, 8, 9, 10, or more learning statistical classifier systems are used, preferably in tandem. Examples of learning statistical classifier systems include, but are not limited to, those using inductive learning (*e.g.*, decision/classification trees such as random forests, classification and regression trees (C&RT), boosted trees, etc.), Probably Approximately Correct (PAC) learning, connectionist learning (*e.g.*, neural networks (NN), artificial neural networks (ANN), neuro fuzzy networks (NFN), network structures, perceptrons such as multi-layer perceptrons, multi-layer feedforward networks, applications of neural networks, Bayesian learning in belief networks, etc.), reinforcement learning (*e.g.*, passive learning in a known environment such as naive learning, adaptive dynamic learning, and temporal difference learning, passive learning in an unknown environment, active learning in an unknown environment, learning action-value functions, applications of reinforcement learning, etc.), and genetic algorithms and evolutionary programming. Other learning statistical classifier systems include support vector machines (*e.g.*, Kernel methods), multivariate adaptive regression splines (MARS), Levenberg-Marquardt algorithms, Gauss-Newton algorithms, mixtures of Gaussians, gradient descent algorithms, and learning vector quantization (LVQ).

In one implementation, the methods further involve obtaining a control biological sample (*e.g.*, biological sample from a subject who does not have a condition or disorder mediated by C1q, C1r, or C1s), a biological sample from the subject during remission or before developing a condition or disorder mediated by C1q, C1r, C1s, or a biological sample from the subject during treatment for developing a condition or disorder mediated by C1q, C1r, or C1s.

An exemplary method for detecting the presence or absence of C1q, C1r, or C1s is anti-C1q, anti-C1r, or anti-C1s antibody to the subject, wherein the anti-C1q, anti-C1r, or anti-C1s antibody is coupled to a detectable label. In some implementations, the detectable label comprises a nucleic acid, oligonucleotide, enzyme, radioactive isotope, biotin or a fluorescent label. In some implementations, the detectable label is detected using an imaging agent for x-ray, CT, MRI, ultrasound, PET and SPECT. In some implementations, the fluorescent label is selected from fluorescein, rhodamine, cyanine dyes or BODIPY.

**EXAMPLES**

**Example 1: Methods**

*Trauma Surgery*

Aged animals were anesthetized and maintained at 2-2.5% isoflurane and secured to a stereotaxic frame with non-traumatic ear bars. The hair on the scalp was removed by shaving with an electric razor. Eye lubricant was applied to their eyes, and betadine and 70% alcohol were applied to the scalp. A midline incision was made to expose the skull.

A unilateral TBI was induced via the controlled cortical impact (CCI) model in the parietal lobe. Mice received a craniectomy ~4 mm in diameter using an electric microdrill at the following coordinates: -2.00 mm anteroposterior and +2.00 mm mediolateral with respect to bregma. Any animal with excessive bleeding due to disruption of the dura was removed from the study. After the craniectomy, a contusion was delivered using a 3 mm convex tip attached to an electromagnetic impactor (Impact One, Leica). The impact parameters were set to a contusion depth of 0.95 mm (from dura) at a velocity of 4.0 m/s and sustained for 300 ms. After the CCI, the scalp was sutured. Sham animals underwent a similar procedure but with only slight drilling without removal of the skull.

Post-surgery, animals were placed in a cage on top of a heatpad until they were fully ambulatory and recovered. After recovery, animals were returned to their home cage and monitored for normal behavior and weight maintenance throughout the duration of the experiments.

*In vivo MR Acquisitions*

All in vivo MR experiments were conducted on a 14.1 tesla vertical MR system (Agilent Technologies, Palo-Alto, CA) equipped with 100 G/cm gradients and a 1H volume coil (ØI = 40 mm).

For each imaging session, mice were anesthetized using isoflurane (1-2% in O2) and a 27 G catheter was secured in the tail vein to allow for intravenous (iv) injection of Magnevist®. Animals were then positioned in a dedicated cradle maintaining constant anesthesia and placed in the MR bore; respiration and temperature were continuously monitored during all acquisitions to ensure animal well-being and data reproducibility.

After initial scout images, high resolution T2-weighted anatomical images were acquired using the following parameters: TE/TR = 12/2000 ms, slice thickness = 0.5 mm, number of averages = 8, matrix = 256 × 256, field of view (FOV) = 30 × 30 mm$^2$, acquisition time = 8 min. Next, BBB lesions were assessed using two identical high-resolution gradient echo sequences separated by 2 minutes 30 seconds delay during which 500 uL of Magnevist® (1 mmol/kg) was injected over 20 seconds. The corresponding parameters were as follows: TE/TR = 4.6/112 ms, slice thickness = 0.5 mm, number of averages = 30, flip angle = 10 matrix = 256 × 192, field of view (FOV) = 25 × 25 mm$^2$, acquisition time = 10 min 41 sec.

*MR data analysis*

Using T2-weighed images, three regions were manually delineated on each slice and for each animal to create masks for the brain, lesion and cavitation. Lesion ROI encompasses abnormal looking brain structure around the CCI focal point. Cavitation was defined as hyperintensity replacing tissue around the contusion. All segmentations were performed with ImageJ (University of Wisconsin, USA). From those masks, 3D reconstructions were obtained using AMIRA software (Mercury Computer systems, San Diego, USA).

Using T1-weighted images, the enhancement ratio maps were obtained using the following equation:

$$\frac{S_{post} - S_{pre}}{S_{pre}} \times 100 \frac{S_{post} - S_{pre}}{S_{pre}} \times 100 \qquad \text{Eq. [X]}$$

where $S_{pre}$ and $S_{post}$ represent the signal intensity before and after Magnevist® injection respectively at each voxel. Using ImageJ, manual ROI were drawn around the contusion point (ipsilateral) and reproduced on the opposite side (contralateral). The mean enhancement ratio was computed for each animal in both ROI.

*Tissue Collection*

All mice were lethally overdosed using a mixture of ketamine (10mg/ml) and xylaxine (1mg/ml). Once animals were completely anesthetized, the chest cavity was opened and animals were perfused with 1X phosphate buffer solution (PBS), pH 7.5 (Gibco, Carlsbad, CA, 70011-044).

For frozen tissue, following PBS, the whole brain was rapidly removed and the brain regions (hippocampus, cortex, injury site, etc.) were dissected, snap frozen on dry ice and stored at -80 °C.

*qPCR analysis*

Dissected ipsilateral hippocampi were used for qPCR analysis. RNA isolation and cDNA conversion were completed. RNA concentration and quality were determined using a NanoDrop (Thermo Scientific). Three hundred nanograms of RNA was reverse transcribed using High-Capacity cDNA Reverse Transcription Kit (Applied Biosystems). Individual samples were investigated in duplicate using SYBR Green Master Mix (Applied Biosystems) following manufacturer's suggested protocol. The relative expression of target genes was determined by the 2-ΔΔCt method and normalized against beta-actin gene expression using a Statagene Mx3005P Real-Time PCR system. The primers used were:

CD11b: Fw 5' CTGAGACTGGAGGCAACCAT-3' (SEQ ID NO: 69) Rev 5' GATATCTCCTTCGCGCAGAC-3' (SEQ ID NO: 70)
C1qa: 5': - 3'
C3: 5' - 3'
GAPDH: Fw 5' AAATGGTGAAGGTCGGTGTG-3' (SEQ ID NO: 71) Rev 5' TGAAGGGGTCGTTGATGG-3' (SEQ ID NO: 72)

*Immunohistochemistry analysis*

For immunohistochemistry analysis, following PBS, animals were perfused with 4% paraformaldehyde, pH 7.5 (PFA, Sigma Aldrich, St. Louis, MO, 441244) brains were fixed in ice-cold 4% paraformaldehyde, pH 7.5 (PFA, Sigma Aldrich, St. Louis, MO, 441244) for 2 hrs followed by sucrose (Fisher Science Education, Nazareth, PA, S25590A) protection (15% to 30%). Brains were embedded with 30% sucrose/ Optimal Cutting Temperature Compound (Tissue Tek, Radnor, PA, 4583) mixture on dry ice and stored at -80 °C. Brains were sectioned into 14 μm slices using a Leica cryostat (Leica Microsystems, Wetzlar, Germany) and mounted on slides (ThermoFisher Scientific, South San Francisco, CA). Slides were brought to room temperature (20 C) prior to use. Slides were washed in tris buffered saline tween solution for 10 min. and twice with tris buffered saline (TBS) for 10 min. each. All slides were blocked in Block Reagent (Perkin Elmer, Waltham, MA, FP1020) for 30 minutes in the dark. Slides were stained with primary antibodies specific for C1q or PSD-95 overnight, washed three times in TBS, and stained for the secondary antibody, goat anti-rabbit Alexa-568 (Invitrogen, Carlsbad, CA, A-11011). Tissues were fixed using ProLong Gold (Invitrogen, Carlsbad, CA, P36930) and a standard slide cover sealed with nail polish. 3-4 images separated by 50-100 μm in the dorsal hippocampus were averaged per animal. Nine μm z-stack images were acquired on a Nikon High Speed Widefield Confocal microscope (Ti inverted fluorescence; CSU-W1) at the UCSF Nikon Imaging Center.

*Flow synaptocytometry*

Hippocampi were homogenized in 0.32 M sucrose in HEPES (Sigma Aldrich, St. Louis, MO H0887) in a glass Dounce homogenizer. Gross tissue fragments were removed in a by centrifugation (1200 x g for 10 min), supernatant collected. Synaptosomes were isolated by further centrifugation (13000 x g for 20 minutes), supernatant was discarded and pellets containing synaptosomes were collected. Samples are kept cold (either on ice or in chilled centrifuges) for staining procedures. Synaptosome collection was standardized between samples by measuring total protein concentrations (Pierce BCA Protein Assay, Thermo Scientific, Rockford, IL, 23225). Synaptosomes (50 ug/ml) were divided into 1.5 ml microcentrifuge tubes for staining. For extracelluar staining, synaptosomes were stained with primary antibodies (C1q,) for 30 minutes at 4 °C and agitated after 15 minutes. Samples were washed 2x with 700 ul of antibody staining buffer (PBS + 5% BSA) at 13,000 x g for 5 min. at 4 °C. Secondary antibodies (Goat anti-rabbit 647, Invitrogen, Carlsbad, CA, A21244) were added for 30 minutes and agitated after 15 minutes at 4 °C in the dark. For intracellular staining, synaptosomes were first permeabilized by Cytofix/Cytoperm fixation solution (BD Biosciences, San Diego, CA, 554722) for 20 minutes on ice. Samples were washed 2x with 700 ul of perm/wash buffer (BD Biosciences, San Diego, CA, 554723) at 13,000 x g for 5 min. at 4 °C. Primary antibodies (Synapsin-1 and PSD-95) were added for 30 minutes and agitated after 15 minutes. Samples were washed 2x with 700 ul of perm/wash buffer at 13,000 x g for 5 min. at 4 °C. Secondary antibodies (Goat anti-rabbit 405, Invitrogen, Carlsbad, CA A31556; Goat anti-mouse 488 Invitrogen, Carlsbad, CA, A11001; Goat anti-mouse 647 Invitrogen, Carlsbad, CA, A21236) were added for 30 minutes and agitated after 15 minutes at 4 °C in the dark. All reagents were made fresh for each staining day. Synaptosomes were determined by size-calibrated beads and co-staining with antibodies specific for pre (synapsin-1) and post-synaptic (PSD-95, GluR1) markers. No positive staining was observed in samples containing: (1) no synaptosomes (2) secondary antibodies alone. A subset of samples from each group were selected for flow synaptocytometry analysis. A maximum of four samples were analyzed per day. Each day at least sham and TBI sample was analyzed standardized to the sham group. Samples were run in duplicate. Data were collected on an LSRII (BD) and analyzed with Flowjo™ software (v10, Tree Star Inc.). 30,000 events were collected for total synaptosomes. 50,000 events were collected for C1q expressing terminals.

### *C1q antibody administration*

Beginning immediately after TBI surgery aged, male animals received intraperitoneal injections of an inhibitory antibody against C1q (ANX-M1, M1) or an isotype-matched control antibody (mouse IgG1). Antibodies were provided by Annexon Biosciences (South San Francisco, CA) and were free of endotoxin and preservatives stored at 4°C. Animals received weekly injections (100 mg/kg) until experiment completion (animal termination).

### *Novel object recognition*

For all behavioral assays the experimenters were blinded both to surgery and treatment. One week prior to behavioral analysis, animals were handled for habituation to experimenters and room settings. Behaviors were performed in dark rooms during the animals wake cycle. All behaviors were recorded using an overhead camera connected to a video tracking and analysis system (Ethovision XT 12.0, Noldus Information Technology). When tracking was not optimal videos were manually scored by an investigator blinded to surgery and treatment

Recognition memory function was measured beginning at one-month post-surgery, by novel object recognition assay (NOR). The test environment consists of an open field arena ($30 \text{ cm}^2$) under red lighting. Mice were allowed to explore the arena for two 10-minute periods for two consecutive days (habituation phase). On day three (training phase), two identical objects (red Lego™ blocks) were secured to the floor in opposite corners of the arena using magnets and mice were allowed to explore the arena and objects for 5 minutes. 24 hours later, day four (testing phase), one of the objects was replaced with a novel object (orange Lego™ flower) of similar dimensions and texture. Mice were allowed to explore for 5 minutes. The objects and arena were cleaned with 70% ethanol between trials and animals. Trials were recorded, and exploratory behavior was defined as time the animals spent directing its nose towards an object. Data is expressed as percent of time mice spent exploring each object. Mice that had less than 5 seconds of exploration time during either training or testing were excluded from analysis.

### *Data analysis*

Results were analyzed using Prism software (v7.05, GraphPad™; La Jolla, CA) and expressed as mean $\pm$ standard error of the mean (SEM). Statistical analyses were performed as listed below with p values of <0.05 considered as significant.

### **Example 2: Lesion progression after contusion injury in aged animals.**

Mild to moderate focal contusion injury to 19 months old animals was examined with the highly reproducible controlled cortical injury (CCI) model. Using a combination of $T_2$-weighted and per/post contrast Ti-weighted MR imaging methods, lesion size, cavitation and blood brain barrier (BBB) breakdown were investigated over time (Fig. 1 and Fig. 2). On $T_2$-weighted MRI, lesions appear as a mix of hyper- and hypo-intense contrasts, delineating a measured total lesion size. On the same images, well-delineated hyperintense cavitation can be seen on the injured hemisphere. Increased lesion size and cavitation were measured by one day post injury (dpi) and persisted for up to one-month post injury. While lesion size peaked at one dpi and then plateau by 1 month, cavitation volume kept growing and by one month reached a volume of ~8 $\text{mm}^3$ (Fig. 1C and Fig. 1D). Next, BBB breakdown was measured following focal contusion injury using Ti-weighted MRI post injection of Gadolinium-based contrast agent, as commonly used in the clinical setting. In the aged brain, BBB integrity was compromised after injury, as shown by increased accumulation of contrast agent through vascular leakage. BBB breakdown was measured for up to one-month post injury (Fig. 2). Overall as expected, lesion size and cavitation are worse in aged compared to young animals after contusion injury.

### **Example 3: Increased microglia numbers chronically after contusion injury.**

In the injured brain of aging animals, there is an increase in microglial cells when compared to brains from young animals (3 months of age) for up to 7 days following TBI. Two independent methods were used, measuring mRNA with quantitative PCR (qPCR) and protein levels with flow cytometric staining, to determine whether the increase in microglia observed at 7 dpi persists at later time points when cognitive deficits are also observed. An increase in CD11b (complement receptor 3 or CR3) mRNA expression was measured in the injured hippocampus beginning at 7 dpi and lasting up to 30 days after injury when compared to aged-matched sham animals (Fig. 3A). These results were further confirmed using flow cytometric staining of hippocampus-derived microglia (CD45$^{lo}$, CD11b$^{+}$). A significant increase in microglial numbers was evident in aged injured animals both at 10 and 30 dpi compared to sham animals (Fig. 3B).

**Example 4: Contusion injury exacerbates microglia phagocytic activity in the aged brain.**

The phagocytic properties of microglia was investigated in the aged brain by a newly modified in vivo engulfment assay. In this assay, fluorescent antibody-labeled PSD-95 synapse particles are injected into the injured hippocampus of the aged sham or injured animals. Three days after injection, microglia are isolated and phagocytic activity is quantified by measuring the percentage of microglia that engulf labeled synapse (CD45$^{lo}$, CD11b$^{+}$ co-expressing with intracellular PSD-95-FITC). A significant increase in the percentage of microglia that engulfed labeled synapse in aged injured animals was found at 30 dpi when compared with matched-sham controls (Fig. 3C). Importantly, the increase in microglia engulfing activity is limited to chronic endpoints, as differences between sham and TBI animals at 10 dpi are not measured. These results suggest that the injury-induced changes in the aged brain take place progressively after injury and could be responsible for worse cognitive outcome.

**Example 5: Contusion injury induces robust complement expression in the aged brain.**

Due to reports linking microglia and complement expression/activation in different neurodegenerative conditions, the effect of injury on C1q expression was investigated over time. Using qPCR analysis, significant increases in C1q mRNA expression was measured within the injured hippocampus at 7, 14 and 30 days after injury (Fig. 4A). The downstream complement factor C3 was also upregulated following injury (Fig. 4B). The chronic induction of C1q was also confirmed by immunohistochemistry analysis of the hippocampus (CA1) (Fig. 4C). Notably, using flow synaptocytometry (Fig. 4D) C1q protein co-localized with individual synapses is significantly elevated at 30 dpi. As described above, expression of the complement C3 receptor (CR3 or CD11b) is also increased (Fig 3A), which is consistent with these results.

**Example 6: Contusive injury results in chronic synapse loss in the aged brain.**

C1q localization at the synapse has been shown to be directly associated with decreases in synapse numbers. Therefore, chronic C1q accumulation (30 dpi) was investigated if it is associated with hippocampal synapse loss after injury in aged animals. Synapse numbers in the hippocampus were assessed by flow cytometric staining after synapse isolation. Synapsin-1 was used as a standard presynaptic marker and PSD-95 as post-synaptic marker. Significant decreases in synapse numbers were measured in the aged injured animals when compared to the age-matched sham animals (Fig. 5A). The decreases, measured by cytometric analysis, were further confirmed by immunohistochemistry staining for the post synaptic marker PSD-95 (Fig. 5B).

**Example 7: Complement blockade prevents memory deficits in aged animals after contusion injury.**

To determine the potential role that increased complement expression plays in the development of injury-induced chronic memory deficits, two complementary approaches were used: 1) a genetic approach, with aged C3 knock-out (C3$^{-/-}$) mice and 2) pharmacological intervention with an anti-C1q antibody. To measure memory function 30 dpi, the novel object recognition test was used. This test has no aversive stimuli and it is based on the innate tendency to explore novelty. Animals are first exposed to two identical objects, twenty-four hours later they are exposed to one of the previously encountered object (familiar) and to a novel one. Recognition memory is measured by the time mice spend exploring the novel object compared to the familiar one. Aged, injured animals were significantly impaired in their ability to recognize the novel object compared to shammatched animals, denoting memory impairments (Fig. 6A). Importantly, memory deficits were observed in both male and female aged, injured animals and no sex-dependent differences (Fig. 6A). Notably, C3$^{-/-}$ aged injured animals and aged wild type injured animals that received the C1q blocking antibody (administered weekly) displayed significantly improved recognition memory capabilities when compared with the aged, injured wild type animals (Fig. 6B). These data demonstrate that activation of the classical complement pathway plays a significant role in the development of memory deficits in the injured aged animals. Most importantly targeting the complement initiation pathway can prevent chronic trauma-induced deficits in aged animals.

**Claims**

**1.**    An inhibitor of the classical complement pathway for use in treating a brain injury, wherein the inhibitor is an anti-C1q antibody comprising:

> a light chain variable domain comprising an HVR-L1 having the amino acid sequence of SEQ ID NO: 5, an HVR-L2 having the amino acid of SEQ ID NO: 6, and an HVR-L3 having the amino acid of SEQ ID NO: 7; and
> a heavy chain variable domain comprising an HVR-H1 having the amino acid sequence of SEQ ID NO: 9, an HVR-H2 having the amino acid of SEQ ID NO: 10, and an HVR-H3 having the amino acid of SEQ ID NO: 11.

2. The inhibitor for use of claim 1, wherein the inhibitor is to be administered during or within the first 4 weeks after a brain injury, the inhibitor is to be administered during or within the first week after brain injury, the inhibitor is to be administered during or within 24 hours after a brain injury, or the inhibitor is to be administered during or within 1, 2, 3, 4, 5, or 6 hours after a brain injury.

3. The inhibitor for use of claim 1 or 2, wherein the inhibitor inhibits synapse loss induced by the brain injury.

4. The inhibitor for use of any one of claims 1-3, wherein the brain injury is a traumatic brain injury, hypoxic brain injury, brain infection, or stroke.

5. The inhibitor for use of any one of claims 1-4, wherein the anti-C1q antibody specifically binds to and neutralizes a biological activity of C1q.

6. The inhibitor for use of any one of claims 1-5, wherein the anti-C1q antibody is a monoclonal antibody, a polyclonal antibody, a recombinant antibody, a humanized antibody, a chimeric antibody, a multispecific antibody, or an antigen-binding fragment thereof.

7. The inhibitor for use of any one of claims 1-5, wherein the antibody is an antigen-binding fragment and the antigen-binding fragment is a Fab fragment, a Fab' fragment, a F(ab')2 fragment, a Fv fragment, a diabody, or a single chain antibody molecule.

8. The inhibitor for use of any one of claims 1-7, wherein the anti-C1q antibody comprises a light chain variable domain comprising an amino acid sequence with at least 95% homology to the amino acid sequence selected from SEQ ID NO: 4 and 35-38; and wherein the light chain variable domain comprises an HVR-L1 having the amino acid sequence of SEQ ID NO: 5, an HVR-L2 having the amino acid of SEQ ID NO: 6, and an HVR-L3 having the amino acid of SEQ ID NO: 7.

9. The inhibitor for use of claim 8, wherein the light chain variable domain comprises an amino acid sequence selected from SEQ ID NO: 4 and 35-38.

10. The inhibitor for use of any one of claims 1-9, wherein the anti-C1q antibody comprises a heavy chain variable domain comprising an amino acid sequence with at least 95% homology to the amino acid sequence selected from SEQ ID NO: 8 and 31-34 and wherein the heavy chain variable domain comprises an HVR-H1 having the amino acid sequence of SEQ ID NO: 9, an HVR-H2 having the amino acid of SEQ ID NO: 10, and an HVR-H3 having the amino acid of SEQ ID NO: 11.

11. The inhibitor for use of claim 10, wherein the heavy chain variable domain comprises an amino acid sequence selected from SEQ ID NO: 8 and 31-34.

**Patentansprüche**

1. Inhibitor des klassischen Komplementwegs zur Verwendung bei der Behandlung einer Hirnverletzung, wobei der Inhibitor ein Anti-C1q-Antikörper ist, umfassend:

> eine variable Domäne der leichten Kette, umfassend eine HVR-L1 mit der Aminosäuresequenz von SEQ ID NO: 5, eine HVR-L2 mit der Aminosäuresequenz von SEQ ID NO: 6 und eine HVR-L3 mit der Aminosäuresequenz von SEQ ID NO: 7; und
> eine variable Domäne der schweren Kette, umfassend eine HVR-H1 mit der Aminosäuresequenz von SEQ ID NO: 9, eine HVR-H2 mit der Aminosäuresequenz von SEQ ID NO: 10 und eine HVR-H3 mit der Aminosäuresequenz von SEQ ID NO: 11.

2. Inhibitor zur Verwendung nach Anspruch 1, wobei der Inhibitor während oder innerhalb der ersten 4 Wochen nach einer Hirnverletzung verabreicht werden soll, der Inhibitor während oder innerhalb der ersten Woche nach einer Hirnverletzung verabreicht werden soll, der Inhibitor während oder innerhalb von 24 Stunden nach einer Hirnverletzung verabreicht werden soll oder der Inhibitor während oder innerhalb von 1, 2, 3, 4, 5 oder 6 Stunden nach einer Hirnverletzung verabreicht werden soll.

3. Inhibitor zur Verwendung nach Anspruch 1 oder 2, wobei der Inhibitor den durch Hirnverletzung induzierten Verlust von Synapsen hemmt.

4. Inhibitor zur Verwendung nach einem der Ansprüche 1-3, wobei die Hirnverletzung eine traumatische Hirnverletzung, eine hypoxische Hirnverletzung, eine Hirninfektion oder ein Schlaganfall ist.

5. Inhibitor zur Verwendung nach einem der Ansprüche 1-4, wobei der Anti-C1q-Antikörper spezifisch an eine biologische Aktivität von C1q bindet und diese neutralisiert.

6. Inhibitor zur Verwendung nach einem der Ansprüche 1-5, wobei der Anti-C1q-Antikörper ein monoklonaler Antikörper, ein polyklonaler Antikörper, ein rekombinanter Antikörper, ein humanisierter Antikörper, ein chimärer Antikörper, ein multispezifischer Antikörper oder ein Antigen-bindendes Fragment davon ist.

7. Inhibitor zur Verwendung nach einem der Ansprüche 1-5, wobei der Antikörper ein Antigen-bindendes Fragment ist und das Antigen-bindende Fragment ein Fab-Fragment, ein Fab'-Fragment, ein F(ab')2-Fragment, ein Fv-Fragment, ein Diabody oder ein einkettiges Antikörpermolekül ist.

8. Inhibitor zur Verwendung nach einem der Ansprüche 1-7, wobei der Anti-C1q-Antikörper eine variable Domäne der leichten Kette umfasst, die eine Aminosäuresequenz mit mindestens 95% Homologie zu der Aminosäuresequenz umfasst, die aus SEQ ID NO: 4 und 35-38 ausgewählt ist; und wobei die variable Domäne der leichten Kette eine HVR-L1 mit der Aminosäuresequenz von SEQ ID NO: 5, eine HVR-L2 mit der Aminosäuresequenz von SEQ ID NO: 6 und eine HVR-L3 mit der Aminosäuresequenz von SEQ ID NO: 7 umfasst.

9. Inhibitor zur Verwendung nach Anspruch 8, wobei die variable Domäne der leichten Kette eine Aminosäuresequenz umfasst, die aus SEQ ID NO: 4 und 35-38 ausgewählt ist.

10. Inhibitor zur Verwendung nach einem der Ansprüche 1-9, wobei der Anti-C1q-Antikörper eine variable Domäne der schweren Kette umfasst, die eine Aminosäuresequenz mit mindestens 95% Homologie zu der Aminosäuresequenz umfasst, die aus SEQ ID NO: 8 und 31-34 ausgewählt ist, und wobei die variable Domäne der schweren Kette eine HVR-H1 mit der Aminosäuresequenz von SEQ ID NO: 9, eine HVR-H2 mit der Aminosäuresequenz von SEQ ID NO: 10 und eine HVR-H3 mit der Aminosäuresequenz von SEQ ID NO: 11 umfasst.

11. Inhibitor zur Verwendung nach Anspruch 10, wobei die variable Domäne der schweren Kette eine Aminosäuresequenz umfasst, die aus SEQ ID NO: 8 und 31-34 ausgewählt ist.

**Revendications**

1. Inhibiteur de la voie classique du complément pour une utilisation dans le traitement d'une lésion cérébrale, dans lequel l'inhibiteur est un anticorps anti-C1q comprenant :

   un domaine variable de chaîne légère comprenant une HVR-L1 ayant la séquence d'acides aminés de SEQ ID NO: 5, une HVR-L2 ayant l'acide aminé de SEQ ID NO: 6, et une HVR-L3 ayant l'acide aminé de SEQ ID NO: 7 ; et un domaine variable de chaîne lourde comprenant une HVR-H1 ayant la séquence d'acides aminés de SEQ ID NO: 9, une HVR-H2 ayant l'acide aminé de SEQ ID NO: 10, et une HVR-H3 ayant l'acide aminé de SEQ ID NO: 11.

2. Inhibiteur pour une utilisation selon la revendication 1, dans lequel l'inhibiteur doit être administré pendant ou dans les 4 premières semaines suivant une lésion cérébrale, l'inhibiteur doit être administré pendant ou dans la première semaine suivant une lésion cérébrale, l'inhibiteur doit être administré pendant ou dans les 24 heures suivant une lésion cérébrale, ou l'inhibiteur doit être administré pendant ou dans les 1, 2, 3, 4, 5 ou 6 heures suivant une lésion cérébrale.

**3.** Inhibiteur pour une utilisation selon la revendication 1 ou 2, dans lequel l'inhibiteur inhibe la perte de synapses induite par la lésion cérébrale.

**4.** Inhibiteur pour une utilisation selon l'une quelconque des revendications 1 à 3, dans lequel la lésion cérébrale est une lésion cérébrale traumatique, une lésion cérébrale hypoxique, une infection cérébrale ou un AVC.

**5.** Inhibiteur pour une utilisation selon l'une quelconque des revendications 1 à 4, dans lequel l'anticorps anti-C1q lie spécifiquement et neutralise une activité biologique de C1q.

**6.** Inhibiteur pour une utilisation selon l'une quelconque des revendications 1 à 5, dans lequel l'anticorps anti-C1q est un anticorps monoclonal, un anticorps polyclonal, un anticorps recombinant, un anticorps humanisé, un anticorps chimérique, un anticorps multispécifique, ou un fragment de celui-ci se liant à l'antigène.

**7.** Inhibiteur pour une utilisation selon l'une quelconque des revendications 1 à 5, dans lequel l'anticorps est un fragment se liant à l'antigène et le fragment se liant à l'antigène est un fragment Fab, un fragment Fab', un fragment F(ab')2, un fragment Fv, un dianticorps, une molécule d'anticorps monocaténaire.

**8.** Inhibiteur pour une utilisation selon l'une quelconque des revendications 1 à 7, dans lequel l'anticorps anti-C1q comprend un domaine variable de chaîne légère comprenant une séquence d'acides aminés ayant au moins 95 % d'homologie avec la séquence d'acides aminés choisie parmi les SEQ ID NO : 4 et 35 à 38 ; et dans lequel le domaine variable de chaîne légère comprend une HVR-L1 ayant la séquence d'acides aminés de SEQ ID NO: 5, une HVR-L2 ayant l'acide aminé de SEQ ID NO: 6, et une HVR-L3 ayant l'acide aminé de SEQ ID NO: 7.

**9.** Inhibiteur pour une utilisation selon la revendication 8, dans lequel le domaine variable de chaîne légère comprend une séquence d'acides aminés choisie parmi les SEQ ID NO : 4 et 35 à 38.

**10.** Inhibiteur pour une utilisation selon l'une quelconque des revendications 1 à 9, dans lequel l'anticorps anti-C1q comprend un domaine variable de chaîne lourde comprenant une séquence d'acides aminés ayant au moins 95 % d'homologie avec la séquence d'acides aminés choisie parmi les SEQ ID NO : 8 et 31 à 34 ; et dans lequel le domaine variable de chaîne lourde comprend une HVR-H1 ayant la séquence d'acides aminés de SEQ ID NO: 9, une HVR-H2 ayant l'acide aminé de SEQ ID NO: 10, et une HVR-H3 ayant l'acide aminé de SEQ ID NO: 11.

**11.** Inhibiteur pour une utilisation selon la revendication 10, dans lequel le domaine variable de chaîne lourde comprend une séquence d'acides aminés choisie parmi les SEQ ID NO : 8 et 31 à 34.

**Fig. 1A**

| Baseline | DAY 1 | DAY 7 | DAY 28 |

**Fig. 1B**

**Fig. 1C**

Fig. 1D

Fig. 2A

B

Fig. 2B

A

Fig. 3A

**B**

Fig. 3B

**C**

Fig. 3C

**A**

C1q

Fig. 4A

**B**

C3

Fig. 4B

Fig. 4C

Fig. 4D

**Fig. 5A**

**Fig. 5B**

**Fig. 6A**

**Fig. 6B**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2010022149 A
- US 4816567 A
- WO 199824893 A
- WO 199634096 A
- WO 199633735 A
- WO 199110741 A
- US 5545807 A
- US 5545806 A
- US 5569825 A
- US 5625126 A
- US 5633425 A
- US 5661016 A
- US 5641870 A
- WO 200442072 A, Presta
- EP 404097 A
- WO 1993011161 A

- WO 2009121948 A
- WO 2014191493 A
- US 6982321 B
- US 7087409 B
- US 6075181 A
- US 6150584 A
- US 2010280227 A
- US 8697359 B
- US 20140087426 A
- US 20120178169 A
- US 5648237 A
- US 5789199 A
- US 5840523 A
- US 3773919 A
- US 5720720 A

**Non-patent literature cited in the description**

- Basic and Clinical Immunology. Appleton & Lange, 1994, 71
- **ABBAS et al.** Cellular and Molecular Immunology. W.B. Saunders Co., 2000
- **KABAT et al.** Sequences of Immunological Interest. National Institute of Health, 1991
- **KABAT et al.** *J. Biol. Chem.*, 1977, vol. 252, 6609-6616
- **KABAT et al.** Sequences of proteins of immunological interest. U.S. Dept. of Health and Human Services, 1991
- **CHOTHIA et al.** *J. Mol. Biol.*, 1987, vol. 196, 901-917
- **MACCALLUM et al.** *J. Mol. Biol.*, 1996, vol. 262, 732-745
- **KOHLER** ; **MILSTEIN**. *Nature*, 1975, vol. 256, 495-97
- **HONGO et al.** *Hybridoma*, 1995, vol. 14 (3), 253-260
- **HARLOW et al.** Antibodies: A Laboratory Manual. Cold Spring Harbor Laboratory Press, 1988
- **HAMMERLING et al.** Monoclonal Antibodies and T-Cell Hybridomas. Elsevier, 1981, 563-681
- **CLACKSON et al.** *Nature*, 1991, vol. 352, 624-628
- **MARKS et al.** *J. Mol. Biol.*, 1992, vol. 222, 581-597
- **SIDHU et al.** *J. Mol. Biol.*, 2004, vol. 338 (2), 299-310

- **LEE et al.** *J. Mol. Biol.*, 2004, vol. 340 (5), 1073-1093
- **FELLOUSE**. *Proc. Nat'l Acad. Sci. USA*, 2004, vol. 101 (34), 12467-472
- **LEE et al.** *J. Immunol. Methods*, 2004, vol. 284 (1-2), 119-132
- **JAKOBOVITS et al.** *Proc. Nat'l Acad. Sci. USA*, 1993, vol. 90, 2551
- **JAKOBOVITS et al.** *Nature*, 1993, vol. 362, 255-258
- **BRUGGEMANN et al.** *Year in Immunol.*, 1993, vol. 7, 33
- **MARKS et al.** *Bio/Technology*, 1992, vol. 10, 779-783
- **LONBERG et al.** *Nature*, 1994, vol. 368, 856-859
- **MORRISON**. *Nature*, 1994, vol. 368, 812-813
- **FISHWILD et al.** *Nature Biotechnol.*, 1996, vol. 14, 845-851
- **NEUBERGER**. *Nature Biotechnol.*, 1996, vol. 14, 826
- **LONBERG** ; **HUSZAR**. *Intern. Rev. Immunol.*, 1995, vol. 13, 65-93
- **ZAPATA et al.** *Protein Eng*, 1995, vol. 8 (10), 1057-1062
- **M. DAËRON**. *Annu. Rev. Immunol.*, 1997, vol. 15, 203-234
- **RAVETCH** ; **KINET**. *Annu. Rev. Immunol.*, 1991, vol. 9, 457-92

- **CAPEL et al.** *Immunomethods*, 1994, vol. 4, 25-34
- **DE HAAS et al.** *J. Lab. Clin. Med.*, 1995, vol. 126, 330-41
- **SHIELDS et al.** *J. Biol. Chem.*, 2001, vol. 9 (2), 6591-6604
- **PLÜCKTHUN**. The Pharmacology of Monoclonal Antibodies. Springer-Verlag, 1994, vol. 113, 269-315
- **HOLLINGER et al.** *Proc. Nat'l Acad. Sci. USA*, 1993, vol. 90, 6444-48
- **MORRISON et al.** *Proc. Nat'l Acad. Sci. USA*, 1984, vol. 81, 6851-55
- **JONES et al.** *Nature*, 1986, vol. 321, 522-525
- **RIECHMANN et al.** *Nature*, 1988, vol. 332, 323-329
- **PRESTA**. *Curr. Op. Struct. Biol.*, 1992, vol. 2, 593-596
- **VASWANI** ; **HAMILTON**. *Ann. Allergy, Asthma & Immunol.*, 1998, vol. 1, 105-115
- **HARRIS**. *Biochem. Soc. Transactions*, 1995, vol. 23, 1035-1038
- **HURLE** ; **GROSS**. *Curr. Op. Biotech.*, 1994, vol. 5, 428-433
- **HOOGENBOOM** ; **WINTER**. *J. Mol. Biol.*, 1991, vol. 227, 381
- **MARKS et al.** *J. Mol. Biol.*, 1991, vol. 222, 581
- **COLE et al.** Monoclonal Antibodies and Cancer Therapy. Alan R. Liss, 1985, 77
- **BOERNER et al.** *J. Immunol.*, 1991, vol. 147 (1), 86-95
- **VAN DIJK** ; **VAN DE WINKEL**. *Curr. Opin. Pharmacol.*, 2001, vol. 5, 368-74
- **LI et al.** *Proc. Nat'l Acad. Sci. USA*, 2006, vol. 103, 3557-3562
- **XU et al.** *Immunity*, 2000, vol. 13, 37-45
- **JOHNSON** ; **WU**. Methods in Molecular Biology. Human Press, 2003, vol. 248, 1-25
- **HAMERS-CASTERMAN et al.** *Nature*, 1993, vol. 363, 446-448
- **SHERIFF et al.** *Nature Struct. Biol.*, 1996, vol. 3, 733-736
- **CHOTHIA** ; **LESK**. *J. Mol. Biol.*, 1987, vol. 196, 901-917
- **KABAT et al.** Sequences of Immunological Interest. Public Health Service, National Institutes of Health, 1991
- **KABAT et al.** Sequences of Proteins of Immunological Interest. Public Health Service, National Institutes of Health, 1991
- **BARBAS et al.** *Proc Nat. Acad. Sci. USA*, 1994, vol. 91, 3809-3813
- **SCHIER et al.** *Gene*, 1995, vol. 169, 147-155
- **YELTON et al.** *J. Immunol.*, 1995, vol. 155, 1994-2004
- **JACKSON et al.** *J. Immunol.*, 1995, vol. 154 (7), 3310-9
- **HAWKINS et al.** *J. Mol. Biol.*, 1992, vol. 226, 889-896
- **HARLOW** ; **LANE**. Antibodies, A Laboratory Manual. Cold Spring Harbor Publications, 1988
- Computational Molecular Biology. Oxford University Press, 1988
- Biocomputing. Informatics and Genome Projects. Academic Press, 1993
- Computer Analysis of Sequence Data. Humana Press, 1994
- **VON HEINJE, G.** Sequence Analysis in Molecular Biology. Academic Press, 1987
- Sequence Analysis Primer. M Stockton Press, 1991
- **SCHIFFER**. *J Virol*, 2012, vol. 88 (17), 8920-8936
- **COBURN, G.** ; **CULLEN, B.** *J. of Virology*, 2002, vol. 76 (18), 9225
- **SANDER** ; **JOUNG**. *Nat. Biotech.*, 2014, vol. 32, 347-355
- **HALE et al.** *Cell*, 2009, vol. 139, 945-956
- **KARGINOV** ; **HANNON**. *Mol. Cell*, 2010, vol. 37, 7
- **BOCH et al.** *Nat. Biotech.*, 2011, vol. 29, 135-136
- **BOCH et al.** *Science*, 2009, vol. 326, 1509-1512
- **MOSCOU** ; **BOGDANOVE**. *Science*, 2009, vol. 326, 1501
- **WEBER et al.** *PLoS One*, 2011, vol. 6, e19722
- **LI et al.** *Nucl. Acids Res.*, 2011, vol. 39, 6315-6325
- **ZHANG et al.** *Nat. Biotech.*, 2011, vol. 29, 149-153
- **MILLER et al.** *Nat. Biotech.*, 2011, vol. 29, 143-148
- **LIN et al.** *Nucl. Acids Res.*, 2014, vol. 42, e47
- **STEWART et al.** *RNA*, April 2003, vol. 9 (4), 493-501
- **SAMBROOK et al.** Molecular Cloning: A Laboratory Manual. Cold Spring Harbor Laboratory Press, Cold Spring, 2001
- Current Protocols in Molecular Biology. 2003
- Methods in Enzymology. PCR 2: A Practical Approach. Academic Press, Inc., 1995
- Antibodies, A Laboratory Manual, and Animal Cell Culture. 1987
- Oligonucleotide Synthesis. 1984
- Methods in Molecular Biology. Humana Press
- Cell Biology: A Laboratory Notebook. Academic Press, 1998
- Animal Cell Culture. 1987
- **J.P. MATHER** ; **P.E. ROBERTS**. Introduction to Cell and Tissue Culture. Plenum Press, 1998
- Cell and Tissue Culture: Laboratory Procedures. J. Wiley and Sons, 1993
- Handbook of Experimental Immunology

- Gene Transfer Vectors for Mammalian Cells. 1987
- PCR: The Polymerase Chain Reaction. 1994
- Current Protocols in Immunology. 1991
- Short Protocols in Molecular Biology. Wiley and Sons, 1999
- **C.A. JANEWAY** ; **P. TRAVERS**. *Immunobiology*, 1997
- **P. FINCH**. *Antibodies*, 1997
- Antibodies: A Practical Approach. IRL Press, 1988
- Monoclonal Antibodies: A Practical Approach. Oxford University Press, 2000
- **E. HARLOW** ; **D. LANE**. Using Antibodies: A Laboratory Manual. Cold Spring Harbor Laboratory Press, 1999
- The Antibodies. Harwood Academic Publishers, 1995
- Cancer: Principles and Practice of Oncology. J.B. Lippincott Company, 1993
- **CHARLTON**. Methods in Molecular Biology. Humana Press, 2003, vol. 248, 245-254
- Remington's Pharmaceutical Sciences. 1980
- Remington's Pharmaceutical Sciences. Mace Publishing Company, 1985
- **LANGER**. *Science*, 1990, vol. 249, 1527-1533
- **F. BALIS et al.** *Am J. Pediatr. Hematol. Oncol.*, 1989, vol. 11 (74), 76
- **HELLER**. Hydrogels in Medicine and Pharmacy. CRC Press, 1987, vol. III, 137-149
- **T.E. MOLLNES** ; **M. KIRSCHFINK**. *Molecular Immunology*, 2006, vol. 43, 107-121